(19) **Europäisches Patentamt / European Patent Office / Office européen des brevets**

(11) **EP 4 751 574 A1**

(12) **EUROPEAN PATENT APPLICATION**

(43) Date of publication:
03.06.2026 Bulletin 2026/23

(21) Application number: 25224794.5

(22) Date of filing: 26.06.2019

(51) International Patent Classification (IPC):
$A23C\ 1/04^{(2006.01)}$ $\quad A23J\ 1/00^{(2006.01)}$
$A23C\ 1/00^{(2006.01)}$ $\quad A23L\ 33/19^{(2016.01)}$
$A23C\ 9/142^{(2006.01)}$ $\quad A23L\ 2/10^{(2006.01)}$
$A23L\ 2/39^{(2006.01)}$ $\quad A23J\ 1/20^{(2006.01)}$

(52) Cooperative Patent Classification (CPC):
(C-Sets available)
A23L 2/102; A23C 9/1427; A23J 1/205; A23L 2/39;
A23V 2002/00 (Cont.)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 27.06.2018 EP 18180224
27.06.2018 EP 18180212
27.06.2018 PCT/EP2018/067299
27.06.2018 PCT/EP2018/067316
27.06.2018 PCT/EP2018/067280

(62) Document number(s) of the earlier application(s) in
accordance with Art. 76 EPC:
19732383.5 / 3 813 537

(71) Applicant: Arla Foods amba
8260 Viby J (DK)

(72) Inventors:
• LAURIDSEN, Kasper Bøgelund
8260 Viby J (DK)
• NIELSEN, Søren Bang
8260 Viby J (DK)
• SØNDERGAARD, Kåre
8260 Viby J (DK)
• DE MOURA MACIEL, Guilherme
8260 Viby J (DK)
• BERTELSEN, Hans
8260 Viby J (DK)
• PARJIKOLAEI, Behnaz Razi
8260 Viby J (DK)
• JÆGER, Tanja Christine
8260 Viby J (DK)

(74) Representative: Guardian
IP Consulting I/S
Diplomvej, Building 381
2800 Kgs. Lyngby (DK)

Remarks:
This application was filed on 18-12-2025 as a
divisional application to the application mentioned
under INID code 62.

(54) **PROCESS FOR PRODUCING BETA-LACTOGLOBULIN ISOLATES AND RELATED METHODS AND USES**

(57) The present invention pertains to novel beta-lactoglobulin (BLG) isolates as well as to a method of producing such isolates and to uses of the powders, e.g. in beverage applications.

Figure 3

250 micron

(Cont. next page)

EP 4 751 574 A1

(52) Cooperative Patent Classification (CPC): (Cont.)

C-Sets
A23V 2002/00, A23V 2200/14, A23V 2200/216,
A23V 2200/238, A23V 2250/54244

**Description**

## FIELD OF THE INVENTION

The present invention pertains to novel beta-lactoglobulin (BLG) isolates as well as to a method of producing such isolates and to uses of the powders, e.g. in beverage applications.

## BACKGROUND

BLG is known as the gel-forming component of whey protein and is prone to undesirable aggregation and gel-formation even at pasteurisation temperature. BLG isolates are perceived as more heat-sensitive than traditional whey protein isolates which contain the more heat-resistant proteins alpha-lactalbumin (ALA) and caseinomacropeptide (CMP) in addition to BLG. Industrial food uses of BLG isolates have previously been limited due to a high price of BLG isolates and problematic handling of freeze dried BLG isolates.

Isolation of beta-lactoglobulin (BLG) from milk serum or whey is the subject of a number of publications and typically involves multiple separation steps and often chromatographic techniques to arrive at a purified beta-lactoglobulin product.

For example, de Jongh et al (Mild Isolation Procedure Discloses New Protein Structural Properties of β-Lactoglobulin, J Dairy Sci., vol. 84(3), 2001, pages 562-571) described purification of BLG from freshly milked milk by low temperature acid coagulation of casein and by subjecting the obtained acid whey to a combination of affinity chromatography (DEAE Sepharose) and gel permeation chromatography. The obtained BLG composition was stated to contain 0.985 g beta-lactoglobulin per 1 g protein. The BLG composition was dried by freeze drying.

Vyas et al (Scale-Up of Native β-Lactoglobulin Affinity Separation Process, J. Dairy Sci. 85:1639-1645, 2002) disclosed a scaled up method for producing native BLG based on affinity chromatography. The BLG composition was dried by freeze drying.

US2790790A1 described a process for isolating BLG from whey by addition of NaCl at pH 3.6-4.0 but did not describe that the isolated BLG was dried.

Palmer (Crystalline Globulin from Cow's Milk, J. Biol. Chem., Vol. 104, 1934, pages 359-372) reported a laborious and time-consuming process for producing protein crystals based on acid whey using several sequences of salt precipitation of unwanted proteins, pH-adjustments and dialysis to remove other unwanted proteins. Finally, when a highly purified BLG solution had been obtained, BLG was crystallized. The process lasted more than 12 days and required addition of toluene. The procedures disclosed in Palmer are therefore incompatible with safe food production and provides products that are clearly not edible. Palmer reported that the BLG crystals could be dried by means of alcohol and ether.

EP 0 604 684 A1 disclosed a process for the recovery of alpha-lactalbumin and/or beta-lactoglobulin enriched whey protein concentrate from a whey protein product. The process comprised a) incubating a solution comprising said whey protein product with a calcium-binding ionic exchange resin in its acid form to initiate the instabilization of alpha-lactalbumin, b) adjusting the pH of the treated protein product solution to a value between 4.3 and 4.8, after separation of said resin, c) incubating said protein product solution at a temperature between 10 and 50 DEG C to promote the flocculation of alpha-lactalbumin, d) fractionating the proteins in said protein product solution at pH 4.3-4.8, providing an alpha-lactalbumin enriched fraction and a beta-lactoglobulin enriched fraction, e) raising the pH of the alpha-lactalbumin enriched fraction sufficiently to solubilize the alpha-lactalbumin fraction, and f) optionally raising the pH of the beta-lactoglobulin enriched fraction sufficiently to neutralize the beta-lactoglobulin fraction.

WO2010/037736 A1 disclosed isolation of whey proteins and the preparation of a whey product and a whey isolate, and in particular the isolation of a beta-lactoglobulin product and the isolation of an alpha-lactalbumin-enriched whey protein isolate from whey obtained from an animal. The alpha-lactalbumin-enriched whey protein isolate provided by the present invention is besides from being low in beta-lactoglobulin also high in alpha-lactalbumin and immunoglobulin G.

WO2011/112695 (A1) disclosed nutritional compositions containing whey protein micelles and leucine. The nutritional compositions provide a sufficient amount of leucine to improve protein synthesis in humans, while also maintaining a low-viscosity fluid matrix and acceptable organoleptic properties.

## SUMMARY OF THE INVENTION

The present inventors have discovered that high functionality BLG isolate powders can be obtained by drying, and preferably spray-drying, a high BLG, liquid BLG isolate having a pH range of i) 2-4.9 or ii) 6.1-8.5, or iii) 5.0-6.0.

Thus, an aspect of the invention pertains to a BLG isolate powder, preferably prepared by spray-drying, having a pH in the range of i) 2.5-4.9, ii) 6.1-8.5, or iii) 5.0-6.0 and comprising:

- total protein in an amount of at least 30% w/w,

- beta-lactoglobulin (BLG) in an amount of at least 85% w/w relative to total protein
- water in an amount of at most 10% w/w,

said BLG isolate powder having one or more of the following:

- a bulk density of at least 0.2 g/cm$^3$,
- an intrinsic tryptophan fluorescence emission ratio (I330/I350) of at least 1.11,
- a degree of protein denaturation of at most 10%,
- a heat-stability at pH 3.9 of at most 200 NTU, and
- at most 15000 colony-forming units/g.

Another aspect of the invention pertains to a liquid BLG isolate having a pH in the range of i) 2-4.9, ii) 6.1-8.5, or iii) 5.0-6.0 and comprising:

- total protein in an amount of at least 10% w/w,
- beta-lactoglubulin (BLG) in an amount of at least 85% w/w relative to total protein said BLG isolate powder having one or more of the following:
- an intrinsic tryptophan fluorescence emission ratio (I330/I350) of at least 1.11,
- a degree of protein denaturation of at most 10%,
- a heat-stability at pH 3.9 of at most 200 NTU, and
- at most 1000 colony-forming units/g.

Yet aspect of the invention pertains to a method of producing a dried BLG isolate powder containing BLG in an amount of at least 85% w/w relative to total protein, the method comprising the steps of:

a) providing a liquid BLG isolate having

i) a pH in the range of 2-4.9,
ii) a pH of in the range of 6.1-8.5, or
iii) a pH of in the range of 5.0-6.0

said liquid BLG isolate containing BLG in an amount of at least 85% w/w relative to total protein,
b) optionally, subjecting the liquid BLG isolate to a physical microbial reduction,
c) drying the liquid BLG isolate, preferably by spray-drying.

A further aspect of the invention pertains to use of the BLG isolate powder or the liquid BLG isolate as defined herein as an ingredient for the production of a food product, e.g. a beverage or an instant beverage powder, having a pH in the range of 2-4.7 and furthermore having one or more of the following:

- a reduced level of drying mouthfeel,
- improved transparency, and/or
- increased content of protein, preferably heat-treated beverages containing a protein content in an amount of at least 3-45% w/w, more preferably 11-40% w/w, even more preferably 15-38% w/w and most preferably 20-36% w/w.

## BRIEF DESCRIPTION OF THE FIGURES

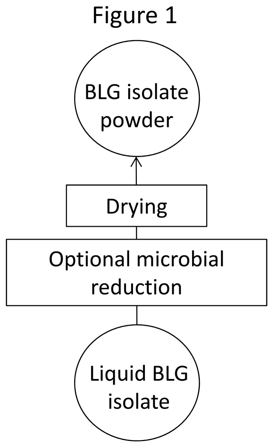

Figure 1 provides a schematic overview of the method of the invention for producing BLG isolate powder.

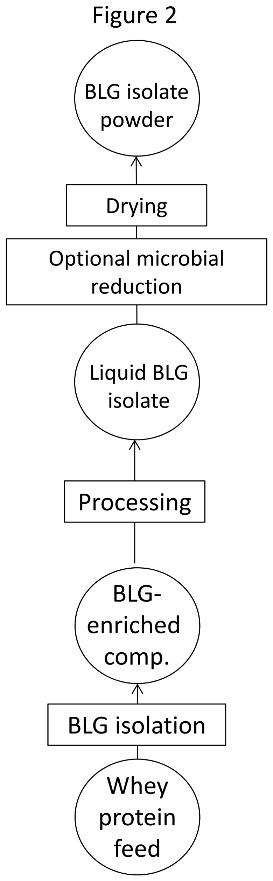

Figure 2 provides a schematic overview of the method of the invention for producing BLG isolate powder starting from a whey protein feed and illustrates the terminology used in the patent description.

Figure 3 is a microscope photo of BLG crystals.

Figure 4 shows a microscope photo of the BLG crystals, both whole and fragmented.

Figure 5 demonstrates that spray-dried BLG isolates have a higher bulk density than comparable WPIs dried at the same conditions.

Figure 6 demonstrates that high protein liquid BLG isolates have a lower viscosity than a comparable WPI solution.

## DETAILED DESCRIPTION

### DEFINITIONS

In the context of the present invention, the term "beta-lactoglobulin" or "BLG" pertains to beta-lactoglobulin from mammal species, e.g. in native, unfolded and/or glycosylated forms and includes the naturally occurring genetic variants. The term furthermore includes aggregated BLG, precipitated BLG and crystalline BLG. When referring to the amount of BLG reference is made to the total amount of BLG including aggregated BLG. The total amount of BLG is determined according to Example 1.31. The term "aggregated BLG" pertains to BLG which is at least partially unfolded and which furthermore has aggregated with other denatured BLG molecules and/or other denatured whey proteins, typically <qby means of hydrophobic interactions and/or covalent bonds.

BLG is the most predominant protein in bovine whey and milk serum and exists in several genetic variants, the main ones in cow milk being labelled A and B. BLG is a lipocalin protein, and can bind many hydrophobic molecules, suggesting a role in their transport. BLG has also been shown to be able to bind iron via siderophores and might have a role in combating pathogens. A homologue of BLG is lacking in human breast milk.

Bovine BLG is a relatively small protein of approx. 162 amino acid residues with a molecular weight of approx. 18.3-18.4 kDa. Under physiological conditions, it is predominantly dimeric, but dissociates to a monomer below about pH 3, preserving its native state as determined using Nuclear Magnetic Resonance spectroscopy. Conversely, BLG also occurs in tetrameric, octam-eric and other multimeric aggregation forms under a variety of natural conditions.

In the context of the present invention, the term "non-aggregated beta-lactoglobulin" or "non-aggregated BLG" also pertains to beta-lactoglobulin from mammal species, e.g. in native, unfolded and/or glycosylated forms and includes the naturally occurring genetic variants. However, the term does not include aggregated BLG, precipitated BLG or crystallised BLG. The amount or concentration of non-aggregated BLG is determined according to Example 1.6.

The percentage of non-aggregated BLG relative to total BLG is determined by calculate $(m_{total\ BLG} - m_{non-aggregate\ BLG})/m_{total\ BLG} *100\%$. $m_{total\ BLG}$ is the concentration or amount of BLG determined according to Example 1.31 and $m_{non-aggregated\ BLG}$ is the concentration or amount of non-aggregated BLG determined according to Example 1.6.

In the context of the present invention, the term "crystal" pertains to a solid material whose constituents (such as atoms, molecules or ions) are arranged in a highly ordered microscopic structure, forming a crystal lattice that extends in all directions.

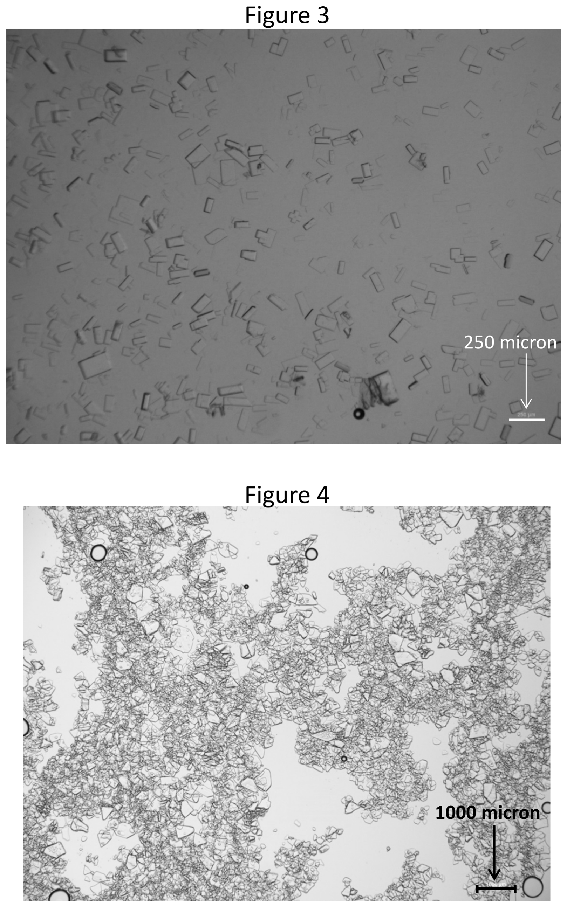

In the context of the present invention, the term "BLG crystal" pertains to protein crystals that primarily contain non-aggregated and preferably native BLG arranged in a highly ordered microscopic structure, forming a crystal lattice that extends in all directions. The BLG crystals may e.g. be monolithic or polycrystalline and may e.g. be intact crystals, fragments of crystals, or a combination thereof. Fragments of crystal are e.g. formed when intact crystals are subjected to mechanical shear during processing. Fragments of crystals also have the highly ordered microscopic structure of crystal but may lack the even surface and/or even edges or corners of an intact crystal. See e.g. Figure 3 for an example of many intact BLG crystals and Figure 4 for an example of fragments of BLG crystals. In both cases, the BLG crystal or crystal fragments can be identified visually as well-defined, compact and coherent structures using light microscopy. BLG crystal or crystal fragments are often at least partially transparent. Protein crystals are furthermore known to be birefringent and this optical property can be used to identify unknown particles having a crystal structure. Non-crystalline BLG aggregates, on the other hand, often appear as poorly defined, non-transparent, and as open or porous lumps of irregular size.

In the context of the present invention, the term "crystallise" pertains to the formation of protein crystals. Crystallisation may e.g. happen spontaneously or be initiated by the addition of crystallisation seeds.

"Mother liquor"

In the context of the present invention the term "mother liquor" pertains to the whey protein solution that remains after BLG has been crystallised and the BLG crystals have be at least partially removed. The mother liquor may still contain some BLG crystals but normally only small BLG crystals that have escaped the separation.

In the context of the present invention, the term "edible composition" pertains to a composition that is safe for human consumption and use as a food ingredient and that does not contain problematic amounts of toxic components, such as toluene or other unwanted organic solvents.

In the context of the present invention, the term "ALA" or "alpha-lactalbumin" pertains to alpha-lactalbumin from mammal species, e.g. in native and/or glycosylated forms and includes the naturally occurring genetic variants. The term furthermore includes aggregated ALA and precipitated BLG. When referring to the amount of ALA reference is made to the total amount of ALA including e.g. aggregated ALA. The total amount of ALA is determined according to Example 1.31. The term "aggregated ALA" pertains to ALA which typically is at least partially unfolded and which furthermore has aggregated with other denatured ALA molecules and/or other denatured whey proteins, typically by means of hydrophobic interactions and/or covalent bonds.

Alpha-lactalbumin (ALA) is a protein present in the milk of almost all mammalian species. ALA forms the regulatory subunit of the lactose synthase (LS) heterodimer and $\beta$-1,4-galactosyl-transferase (beta4Gal-T1) forms the catalytic component. Together, these proteins enable LS to produce lactose by transferring galactose moieties to glucose. One of the main structural differences with beta-lactoglobulin is that ALA does not have any free thiol group that can serve as the starting-point for a covalent aggregation reaction.

In the context of the present invention, the term "non-aggregated ALA" also pertains to ALA from mammal species, e.g. in native, unfolded and/or glycosylated forms and includes the naturally occurring genetic variants. However, the term does not include aggregated ALA or precipitated ALA. The amount or concentration of non-aggregated BLG is determined according to Example 1.6.

The percentage of non-aggregated ALA relative to total ALA is determined by calculate $(m_{total\ ALA} - m_{non\text{-}aggregate\ ALA})/m_{total\ ALA} *100\%$. $m_{total\ ALA}$ is the concentration or amount of ALA determined according to Example 1.31 and $m_{non\text{-}aggregated\ ALA}$ is the concentration or amount of non-aggregated ALA determined according to Example 1.6.

In the context of the present invention, the term "caseinomacropeptide" or "CMP" pertains to the hydrophilic peptide, residue 106-169, originated from the hydrolysis of "$\kappa$-CN" or "kappa-casein" from mammal species, e.g. in native and/or glycosylated forms and includes the naturally occurring genetic variants, by an aspartic proteinase, e.g. chymosin.

In the context of the present invention, the term "BLG isolate" means a composition that contains BLG in an amount of at least 85% w/w relative to total protein. A BLG isolate preferably has a total protein content of a least 30% w/w, and preferably at least 80% w/w relative to total solids.

In the context of the present invention, the term "BLG isolate powder" pertains to a BLG isolate in powder form and preferably a free-flowing powder.

In the context of the present invention, the term "BLG isolate liquid" pertains to a BLG isolate in liquid form and preferably an aqueous liquid.

The term "whey" pertains to the liquid phase that is left after the casein of milk has been precipitated and removed. Casein precipitation may e.g. be accomplished by acidification of milk and/or by use of rennet enzyme. Several types of whey exist, such as "sweet whey", which is the whey product produced by rennet-based precipitation of casein, and "acid whey" or "sour whey", which is the whey product produced by acid-based precipitation of casein. Acid-based precipitation of casein may e.g. be accomplished by addition of food acids or by means of bacterial cultures.

The term "milk serum" pertains to the liquid which remains when casein and milk fat globules have been removed from milk, e.g. by microfiltration or large pore ultrafiltration. Milk serum may also be referred to as "ideal whey".

The term "milk serum protein" or "serum protein" pertains to the protein which is present in the milk serum.

In the context of the present invention, the term "whey protein" pertains to protein that is found in whey or in milk serum. Whey protein may be a subset of the protein species found in whey or milk serum, and even a single whey protein species or it may be the complete set of protein species found in whey or/and in milk serum.

In the context of the present invention, the main non-BLG proteins of a standard whey protein concentrate from sweet whey are ALA, CMP, bovine serum albumin, immunoglobulin, osteopontin, lactoferrin, and lactoperoxidase. In the context of the present invention, the weight percentages of the main non-BLG whey proteins of a standard whey protein concentrate from sweet whey are:

ALA in an amount of 18% w/w relative to total protein,
CMP in an amount of 18% w/w relative to total protein,
BSA in an amount of 4% w/w relative to total protein,
Casein species in an amount of 5% w/w relative to total protein,
Immunoglobulin in an amount of 6% w/w relative to total protein,
Osteopontin in an amount of 0.5% w/w relative to total protein,
Lactoferrin in an amount of 0.1% w/w relative to total protein, and
Lactoperoxidase in an amount of 0.1% w/w relative to total protein.

The term casein pertains to casein protein found in milk and encompasses both native micellar casein as found in raw milk, the individual casein species, and caseinates.

In the context of the present invention, a liquid which is "supersaturated" or "supersaturated with respect to BLG" contains a concentration of dissolved, non-aggregated BLG which is above the saturation point of non-aggregated BLG in that liquid at the given physical and chemical conditions. The term "supersaturated" is well-known in the field of crystallisation (see e.g. Gér-ard Coquerela, "Crystallization of molecular systems from solution: phase diagrams, supersaturation and other basic concepts", Chemical Society Reviews, p. 2286-2300, Issue 7, 2014) and supersaturation can be determined by a number of different measurement techniques (e.g. by spectroscopy or particle size analysis). In the context of the present invention, supersaturation with respect to BLG is determined by the following procedure.

Procedure for testing whether a liquid at a specific set of conditions is supersaturated with respect to BLG:

a) Transfer a 50 ml sample of the liquid to be tested to a centrifuge tube (VWR Catalogue no. 525-0402) having a height of 115 mm, an inside diameter of 25 mm and a capacity of 50 mL. Care should be taken to keep the sample and subsequent fractions thereof at the original physical and chemical conditions of the liquid during steps a) - h).
b) The sample is immediately centrifuged at 3000 g for 3.0 minutes with max. 30 seconds acceleration and max 30 seconds deceleration.
c) Immediately after the centrifugation, transfer as much as possible of the supernatant (without disturbing the pellet if a pellet has formed) to a second centrifuge tube (same type as in step a)
d) Take a 0.05 mL subsample of the supernatant (subsample A)
e) Add 10 mg of BLG crystals (at least 98% pure, non-aggregated BLG relative to total solids) having a particle size of at most 200 micron to a second centrifuge tube and agitate the mixture.
f) Allow the second centrifuge tube to stand for 60 minutes at the original temperature.
g) Immediately after step f), centrifuge the second centrifuge tube at 500 g for 10 minutes and then take another 0.05 mL subsample of the supernatant (subsample B).
h) Recover the centrifugation pellet of step g) if there is one, resuspend it in milliQ water and immediately inspect the suspension for presence of crystals that are visible by microscopy.
i) Determine the concentration of non-aggregated BLG in subsamples A and B using the method outlined in Example 1.6 - the results are expressed as % BLG w/w relative to the total weight of the subsamples. The concentration of non-aggregated BLG of subsample A is referred to as $C_{BLG, A,}$ and the concentration of non-aggregated BLG of subsample B is referred to as $C_{BLG, B}$.
j) The liquid from which the sample of step a) was taken was supersaturated (at the specific conditions) if $C_{BLG, B}$ is lower than $C_{BLG, A}$ and if crystals are observed in step i).

In the context of the present invention, the terms "liquid" and "solution" encompass both compositions that are free of particulate matter and compositions that contain a combination of liquid and solid and/or semi-solid particles, such as e.g. protein crystals or other protein particles. A "liquid" or a "solution" may therefore be a suspension or even a slurry. However, a "liquid" and "solution" are preferably pumpable.
In the context of the present invention, the terms "whey protein concentrate" (WPC) and "serum protein concentrate" (SPC) pertain to dry or aqueous compositions which contain a total amount of protein of 20-89% w/w relative to total solids.
A WPC or an SPC preferably contains:

20-89% w/w protein relative to total solids,
15-70% w/w BLG relative to total protein,
8-50% w/w ALA relative to total protein, and
0-40% w/w CMP relative to protein.

Alternatively, but also preferred, a WPC or an SPC may contain:

20-89% w/w protein relative to total solids,
15-90% w/w BLG relative to total protein,
4-50% w/w ALA relative to total protein, and
0-40% w/w CMP relative to protein.

Preferably, a WPC or an SPC contains:

20-89% w/w protein relative to total solids,
15-80% w/w BLG relative to total protein,
4-50% w/w ALA relative to total protein, and
0-40% w/w CMP relative to protein.

More preferably a WPC or an SPC contains:

70-89% w/w protein relative to total solids,
30-90% w/w BLG relative to total protein,
4-35% w/w ALA relative to total protein, and
0-25% w/w CMP relative to protein.

SPC typically contain no CMP or only traces of CMP.

The terms "whey protein isolate" (WPI) and "serum protein isolate" (SPI) pertain to dry or aqueous compositions which contain a total amount of protein of 90-100% w/w relative to total solids.

A WPI or an SPI preferably contains:

90-100% w/w protein relative to total solids,
15-70% w/w BLG relative to total protein,
8-50% w/w ALA relative to total protein, and
0-40% w/w CMP relative to total protein.

Alternatively, but also preferred, a WPI or an SPI may contain:

90-100% w/w protein relative to total solids,
30-95% w/w BLG relative to total protein,
4-35% w/w ALA relative to total protein, and
0-25% w/w CMP relative to total protein.

More preferably a WPI or an SPI may contain:

90-100% w/w protein relative to total solids,
30-90% w/w BLG relative to total protein,
4-35% w/w ALA relative to total protein, and
0-25% w/w CMP relative to total protein.

SPI typically contain no CMP or only traces of CMP.

In the context of the present invention, the term "additional protein" means a protein that is not BLG. The additional protein that is present in the whey protein solution typically comprises one or more of the non-BLG proteins that are found in milk serum or whey. Non-limiting examples of such proteins are alpha-lactalbumin, bovine serum albumin, immunoglobulins, caseinomacropeptide (CMP), osteopontin, lactoferrin, and milk fat globule membrane proteins.

The terms "consists essentially of" and "consisting essentially of" mean that the claim or feature in question encompasses the specified materials or steps and those that do not materially affect the basic and novel characteristic(s) of the claimed invention.

In the context of the present invention, the phrase "Y and/or X" means "Y" or "X" or "Y and X". Along the same line of logic, the phrase "$n_1$, $n_2$, ..., $n_{i-1}$, and/or $n_i$" means "$n_1$" or "$n_2$" or ... or "$n_{i-1}$" or "$n_i$" or any combination of the components : $n_1$, $n_2$,...$n_{i-1}$, and $n_i$.

In the context of the present invention, the term "dry" or "dried" means that the composition or product in question comprises at most 10% w/w water, preferably at most 6% w/w and more preferably even less.

In the context of the present invention, the term "physical microbial reduction" pertains to physical interaction with a composition which results in reduction of the total amount of viable microorganisms of the composition. The term does not encompass addition of chemicals that result in killing of microorganisms. The term furthermore does not encompass the heat exposure to which the atomized droplets of liquid are exposed to during spray-drying but include possible pre-heating prior to spray-drying.

In the context of the present invention, the pH of a powder refers to the pH of 10 g of the powder mixed into 90 g demineralised water and is measured according to Example 1.16.

In the context of the present invention, the weight percentage (% w/w) of a component of a certain composition, product, or material means the weight percentage of that component relative to the weight of the specific composition, product, or material unless another reference (e.g. total solids or total protein) is specifically mentioned.

In the context of the present invention, the process step "concentration" and the verb "concentrate" pertain to concentration of protein and encompass both concentration of protein on total solids basis and concentration of protein on a total weight basis. This means e.g. that concentration does not necessarily require that the absolute concentration w/w of protein of a composition increases as long at the content of protein increases relative to total solids.

In the context of the present invention, the term "weight ratio" between component X and component Y means the value obtained by the calculation $m_X/m_Y$ wherein $m_X$ is the amount (weight) of components X and $m_Y$ is the amount (weight) of components Y.

In the context of the present invention, the term "at least pasteurisation" pertains to a heat-treatment which has microbial killing effect equal to or higher than a heat-treatment of 70 degrees C for 10 seconds. The reference for determining the bacteria killing effect is E. coli O157:H7.

In the context of the present invention, the term "whey protein feed" pertains to whey protein source from which the liquid BLG isolate is derived. The whey protein feed has a lower content of BLG relative to total protein than the liquid BLG isolate and is typically a WPC, a WPI, an SPC or an SPI.

In the context of the present invention, the term "BLG-enriched composition" pertains to the BLG-enriched composition resulting from isolating BLG from the whey protein feed. The BLG-enriched composition typically comprises the same whey proteins as the whey protein feed but BLG is present in significantly higher concentration relative to total protein than in whey protein feed. The BLG-enriched composition may e.g. be prepared from the whey protein feed by chromatography, protein crystallisation and/or membrane-based protein fractionation. The BLG-enriched composition comprises BLG in an amount of at least 85% w/w relative to total protein, and preferably at least 90% w/w. In some cases the BLG-enriched composition can be used directly as the liquid BLG isolate. However, often additional processing is required to convert the BLG-enriched composition to the liquid BLG isolate.

In the context of the present invention, the term "whey protein solution" is used to describe the special aqueous whey protein composition that is supersaturated with respect to BLG in salting-in mode and useful for preparing BLG crystals.

In the context of the present invention, the term "sterile" means that the sterile composition or product in question does not contain any viable microorganisms and therefore is devoid of microbial growth during storage at room temperature. A composition that has been sterilised is sterile.

When a liquid, such as a beverage preparation, is sterilized and packaged aseptically in a sterile container it typically has a shelf life of at least six months at room temperature. The sterilization treatment kills spores and microorganisms that could cause spoilage of the liquid.

In the context of the present invention the term "protein fraction" relates to proteins of the composition in question e.g. the proteins of a powder or a beverage preparation.

In the context of the present invention the term "drying mouthfeeling" relates to a feeling in the mouth, it feels like a drying of the mouth and teeth and results in minimization of the saliva production.

Thus drying mouthfeeling is not a taste as such, but a physical mouthfeeling and time-dependent feeling in the mouth.

In the context of the present invention the term "minerals" as used herein, unless otherwise specified, refers to any one of major minerals, trace or minor minerals, other minerals, and combinations thereof. Major minerals include calcium, phosphorus, potassium, sulfur, sodium, chlorine, magnesium. Trace or minor minerals include iron, cobalt, copper, zinc, molybdenum, iodine, selenium, manganese and other minerals include chromium, fluorine, boron, lithium, and strontium.

In the context of the present invention the terms "lipid", "fat", and "oil" as used herein unless otherwise specified, are used interchangeably to refer to lipid materials derived or processed from plants or animals. These terms also include synthetic lipid materials so long as such synthetic materials are suitable for human consumption.

In the context of the present invention the term "transparent" encompasses a beverage preparation having a visibly clear appearance and which allows light to pass and through which distinct images appear. A transparent beverage has a turbidity of at most 200 NTU.

In the context of the present invention the terms "opaque" encompasses a beverage preparation having a visibly unclear appearance and it has a turbidity of more than 200 NTU.

An aspect of the invention pertains to a beta-lactoglobulin (BLG) isolate powder, preferably prepared by spray-drying, having a pH in the range of i) 2-4.9, ii) 6.1-8.5, or iii) 5.0-6.0 and comprising:

- total protein in an amount of at least 30% w/w,
- BLG in an amount of at least 85% w/w relative to total protein, and
- water in an amount of at most 10% w/w.

The BLG isolate powder preferably has one or more of the following:

- a bulk density of at least 0.2 g/cm$^3$,
- an intrinsic tryptophan fluorescence emission ratio (I330/I350) of at least 1.11,
- a degree of protein denaturation of at most 10%,
- a heat-stability at pH 3.9 of at most 200 NTU, and
- at most 1000 colony-forming units/g.

The BLG isolate powder is preferably an edible composition.

In some preferred embodiments of the invention, the BLG isolate powder has a pH in the range of 2-4.9. Such powders are particularly useful for acidic food products and particularly acidic beverages.

In other preferred embodiments of the invention, BLG isolate powder has a pH in the range of 6.1-8.5.

In some preferred embodiments of the invention, the BLG isolate powder comprises total protein in an amount of at least 40% w/w, preferably at least 50% w/w, at least 60% w/w, more preferably at least 70% w/w, even more preferably at least 80% w/w.

Even higher protein contents may be required and in some preferred embodiments of the invention, the BLG isolate powder comprises total protein in an amount of at least 85% w/w, preferably at least 90% w/w, at least 92% w/w, more preferably at least 94% w/w, and even more preferably at least 95% w/w.

Total protein is measured according to Example 1.5.

In some preferred embodiments of the invention, the BLG isolate powder comprises BLG in an amount of at least 96% w/w relative to total protein, preferably at least 96.5% w/w, more preferably at least 97% w/w, even more preferably at least 98%, and most preferably BLG in an amount of at least 99.5% w/w relative to total protein.

In some preferred embodiments of the invention, the BLG isolate powder comprises BLG in an amount of at least 97.5% w/w relative to total protein, preferably at least 98.0% w/w, more preferably at least 98.5% w/w, even more preferably at least 99.0%, and most preferably BLG in an amount of at least 99.7% w/w relative to total protein, such as approx. 100.0% w/w relative to total protein.

In some preferred embodiments of the invention, the sum of alpha-lactalbumin (ALA) and caseinomacropeptide (CMP) comprises at least 40% w/w of the non-BLG protein of the powder, preferably at least 60% w/w, even more preferably at least 70% w/w, and most preferably at least 90% w/w of the non-BLG protein of the powder.

In other preferred embodiments of the invention, each main non-BLG whey protein is present in a weight percentage relative to total protein which is at most 25% of its weight percentage relative to total protein in a standard whey protein concentrate from sweet whey, preferably at most 20%, more preferably at most 15%, even more preferably at most 10%, most preferably at most 6%.

Even lower concentrations of the main non-BLG whey proteins may be desirable. Thus, in additional preferred embodiments of the invention, each main non-BLG whey protein is present in a weight percentage relative to total protein which is at most 4% of its weight percentage relative to total protein in a standard whey protein concentrate from sweet whey, preferably at most 3%, more preferably at most 2%, even more preferably at most 1%.

In some preferred embodiments of the invention, ALA comprises at most 80% w/w of the non-BLG protein of the BLG isolate powder, preferably at most 60% w/w, even more preferably at most 40% w/w, and most preferably at most 30% w/w of the non-BLG protein of the BLG isolate powder.

Even lower contents of ALA may be preferred, thus in some preferred embodiments of the invention, ALA comprises at most 20% w/w of the non-BLG protein of the BLG isolate powder, preferably at most 15% w/w, even more preferably at most 10% w/w, and most preferably at most 5% w/w of the non-BLG protein of the BLG isolate powder.

The inventors have seen indications that reduction of lactoferrin and/or lactoperoxidase is particularly advantageous for obtaining a colour-neutral whey protein product.

Thus in some preferred embodiments of the invention, lactoferrin is present in a weight percentage relative to total protein which is at most 25% of its weight percentage relative to total protein in a standard whey protein concentrate from sweet whey, preferably at most 20%, more preferably at most 15%, even more preferably at most 10%, most preferably at most 6%. Even lower concentrations of lactoferrin may be desirable. Thus, in additional preferred embodiments of the invention, lactoferrin is present in a weight percentage relative to total protein which is at most 4% of its weight percentage relative to total protein in a standard whey protein concentrate from sweet whey, preferably at most 3%, more preferably at most 2%, even more preferably at most 1%.

Similarly, in some preferred embodiments of the invention, lactoperoxidase is present in a weight percentage relative to total protein which is at most 25% of its weight percentage relative to total protein in a standard whey protein concentrate from sweet whey, preferably at most 20%, more preferably at most 15%, even more preferably at most 10%, most preferably at most 6%. Even lower concentrations of lactoperoxidase may be desirable. Thus, in additional preferred embodiments of the invention, lactoperoxidase is present in a weight percentage relative to total protein which is at most 4% of its weight percentage relative to total protein in a standard whey protein concentrate from sweet whey, preferably at most 3%, more preferably at most 2%, even more preferably at most 1%.

Lactoferrin and lactoperoxidase are quantified according to Example 1.29.

In some preferred embodiments of the invention, the BLG isolate powder has a water content in an amount of at most 10% w/w, preferably at most 7% w/w, more preferably at most 6% w/w, even more preferably at most 4% w/w, and most preferred at most 2% w/w.

In some preferred embodiments of the invention the BLG isolate powder comprises carbohydrate in an amount of at most 60% w/w, preferably at most 50% w/w, more preferably at most 20% w/w, even more preferably at most 10% w/w, even more preferably at most 1% w/w, and most preferably at most 0.1%. The BLG isolate powder may for example contain carbohydrates, such as e.g. lactose, oligosaccharides and/or hydrolysis products of lactose (i.e. glucose and galactose), sucrose, and/or maltodextrin.

In some preferred embodiments of the invention, the BLG isolate powder comprises lipid in an amount of at most 10% w/w, preferably at most 5% w/w, more preferably at most 2% w/w, and even more preferably at most 0.1% w/w.

The amount of lipid is determined according to ISO 1211:2010 (Determination of Fat Content - Röse-Gottlieb Gravimetric Method).

The present inventors have found that it can be advantageous to control the mineral content to reach some of the desired properties of the BLG isolate powder.

In some preferred embodiments of the invention, the sum of the amounts of Na, K, Mg, and Ca of the BLG isolate powder is at most 10 mmol/g protein. Preferably, the sum of the amounts of Na, K, Mg, and Ca of the BLG isolate powder is at most 6 mmol/g protein, more preferably at most 4 mmol/g protein, even more preferably at most 2 mmol/g protein.

In other preferred embodiments of the invention, the sum of the amounts of Na, K, Mg, and Ca of the BLG isolate powder is at most 1 mmol/g protein. Preferably, the sum of the amounts of Na, K, Mg, and Ca of the BLG isolate powder is at most 0.6 mmol/g protein, more preferably at most 0.4 mmol/g protein, even more preferably at most 0.2 mmol/g protein, and most preferably at most 0.1 mmol/g protein.

In other preferred embodiments of the invention, the sum of the amounts of Mg and Ca of the BLG isolate powder is at most 5 mmol/g protein. Preferably, the sum of the amounts of Mg and Ca of the BLG isolate powder is at most 3 mmol/g protein, more preferably at most 1.0 mmol/g protein, even more preferably at most 0.5 mmol/g protein.

In other preferred embodiments of the invention, the sum of the amounts of Mg and Ca of the BLG isolate powder is at most 0.3 mmol/g protein. Preferably, the sum of the amounts of Mg and Ca of the BLG isolate powder is at most 0.2 mmol/g protein, more preferably at most 0.1 mmol/g protein, even more preferably at most 0.03 mmol/g protein, and most preferably at most 0.01 mmol/g protein.

The inventors have found that it is possible to make low phosphorus/low potassium variants of the BLG isolate powder that are particularly useful to patients with kidney diseases. To make such a product, the BLG isolate powder has to have an equally low content of phosphorus and potassium.

Thus, in some preferred embodiments of the invention, the BLG isolate powder has a total content of phosphorus of at most 100 mg phosphorus per 100 g protein. Preferably, the BLG isolate powder has a total content of at most 80 mg phosphorus per 100 g protein. More preferably, the BLG isolate powder has a total content of at most 50 mg phosphorus per 100 g protein. Even more preferably, the BLG isolate powder has a total content of phosphorus of at most 20 mg phosphorus per 100 g protein. The BLG isolate powder has a total content of phosphorus of at most 5 mg phosphorus per 100 g protein.

In some preferred embodiments of the invention, the BLG isolate powder comprises at most 600 mg potassium per 100 g protein. More preferably, the BLG isolate powder comprise at most 500 mg potassium per 100 g protein. More preferably, the BLG isolate powder comprises at most 400 mg potassium per 100 g protein. More preferably, the BLG isolate powder comprises at most 300 mg potassium per 100 g protein. Even more preferably, the BLG isolate powder at most 200 mg potassium per 100 g protein. Even more preferably, the BLG isolate powder comprises at most 100 mg potassium per 100 g protein. Even more preferably, the BLG isolate powder comprises at most 50 mg potassium per 100 g protein and even more preferably, the BLG isolate powder comprises at most 10 mg potassium per 100 g protein.

The content of phosphorus relates to the total amount of elemental phosphorus of the composition in question and is determined according to Example 1.19. Similarly, the content of potassium relates to the total amount of elemental potassium of the composition in question and is determined according to Example 1.19.

In some preferred embodiments of the invention, the BLG isolate powder comprises at most 100 mg phosphorus/100 g protein and at most 700 mg potassium/100g protein, preferably at most 80mg phosphorus/100 g protein and at most 600mg potassium/ 100g protein, more preferably at most 60mg phosphorus/100 g protein and at most 500mg potassium/ 100g protein, more preferably at most 50mg phosphorus/100 g protein and at most 400mg potassium/ 100g protein, or more preferably at most 20mg phosphorus/100 g protein and at most 200mg potassium/100g protein, or even more preferably at most 10mg phosphorus/100 g protein and at most 50mg potassium/ 100g protein. In some preferred embodiments of the invention the BLG isolate powder comprises at most 100mg phosphor/100 g protein and at most 340mg potassium/100g protein.

The low phosphorus and/or low potassium compositions according to the present invention may be used as a food ingredient for the production of a food product for patients groups that have a reduced kidney function.

The present inventors have found that for some applications, e.g. acidic food products and particularly acidic beverages, it is particularly advantageous to have an acidic BLG isolate powder having a pH of at most 4.9 and even more preferably at most 4.3. This is especially true for high protein, transparent acidic beverages.

In the context of the present invention, a transparent liquid has a turbidity of at most 200 NTU measured according to Example 1.7.

Thus, in some preferred embodiments of the invention, the BLG isolate powder has a pH in the range of 2-4.9. Preferably, the BLG isolate powder has a pH in the range of 2.5-4.7, more preferably 2.8-4.3, even more preferably 3.2-4.0, and most preferably 3.4-3.9. Alternatively, but also preferred, the BLG isolate powder may have a pH in the range of 3.6-4.3.

The present inventors have found that for some applications, e.g. pH-neutral food products and particularly pH-neutral beverages, it is particularly advantageous to have a pH-neutral BLG isolate powder. This is especially true for high protein, transparent or opaque pH-neutral beverages.

Thus, in some preferred embodiments of the invention, BLG isolate powder has a pH in the range of 6.1-8.5. Preferably, the powder has a pH in the range of 6.1-8.5, more preferably 6.2-8.0, even more preferably 6.3-7.7, and most preferably 6.5-7.5.

In other preferred embodiments of the invention, BLG isolate powder has a pH in the range of 5.0-6.0. Preferably, the powder has a pH in the range of 5.1-5.9, more preferably 5.2-5.8, even more preferably 5.3-5.7, and most preferably 5.4-5.6.

Advantageously, the BLG isolate powder of the present invention may have bulk density of at least 0.20 g/cm$^3$, preferably at least 0.30 g/cm$^3$, more preferably at least 0.40 g/cm$^3$, even more preferably at least 0.45 g/cm$^3$, even more preferably at least 0.50 g/cm$^3$, and most preferably at least 0.6 g/cm$^3$.

Low density powders such as freeze-dried BLG isolates are fluffy and easily drawn into the air of the production site during use. This is problematic as it increases the risk of cross-contamination of the freeze-dried powder to other foods products and a dusty environment is known to be a cause of hygiene issues. In extreme cases, a dusty environment also increases the risk of dust explosions.

The high density variants of the present invention are easier to handle and less prone to flow into the surrounding air.

An additional advantage of the high density variants of the present invention is that they take up less space during transportation and thereby increase weight of BLG isolate powder that can be transported in one volume unit.

Yet an advantage of the high density variants of the present invention is that they are less prone to segregation when used in powder mixtures with other powdered food ingredients, such as e.g. powdered sugar (bulk density of approx. 0.56 g/cm$^3$), granulated sugar (bulk density of approx. 0.71 g/cm$^3$), powdered citric acid (bulk density of approx. 0.77 g/cm$^3$).

The BLG isolate powder of the present invention may e.g. have a bulk density in the range of 0.2-1.0 g/cm$^3$, preferably in the range of 0.30-0.9 g/cm$^3$, more preferably in the range of 0.40-0.8 g/cm$^3$, even more preferably in the range of 0.45-0.75 g/cm$^3$, even more preferably in the range of 0.50-0.75 g/cm$^3$, and most preferably in the range of 0.6-0.75 g/cm$^3$.

In some preferred embodiments of the invention the BLG isolate powder have a bulk density in the range of 0.45-1.2 g/cm$^3$, preferably in the range of 0.46-1.0 g/cm$^3$, more preferably in the range of 0.47-0.8 g/cm$^3$, even more preferably in the range of 0.48-0.75 g/cm$^3$, even more preferably in the range of 0.48-0.6 g/cm$^3$, and most preferably in the range of 0.50-0.6 g/cm$^3$.

The bulk density of a powder is measured according to Example 1.17.

The present inventors have found that it is advantageous to maintain the native conformation of BLG and have seen indications that increased unfolding of BLG gives rise to an increased level of drying mouthfeel when the BLG is used for acidic beverages.

The intrinsic tryptophan fluorescence emission ratio (I330/I350) is a measure of degree of unfolding of BLG and the inventors have found that at high intrinsic tryptophan fluorescence emission ratios, which correlate with low or no unfolding of BLG, less drying mouthfeel was observed. The intrinsic tryptophan fluorescence emission ratio (I330/I350) is measured according to Example 1.1.

In some preferred embodiments of the invention, the BLG isolate powder has an intrinsic tryptophan fluorescence emission ratio (I330/I350) of at least 1.11.

In some preferred embodiments of the invention, the BLG isolate powder has an intrinsic tryptophan fluorescence emission ratio (I330/I350) of at least 1.12, preferably at least 1.13, more preferably at least 1.15, even more preferably at least 1.17, and most preferably at least 1.19.

If BLG isolate powder contains considerable amounts of non-protein matter it is preferred to isolate the protein fraction before measuring the intrinsic tryptophan fluorescence emission ratio. Thus in some preferred embodiments of the invention, the protein fraction of the BLG isolate powder has an intrinsic tryptophan fluorescence emission ratio of at least 1.11.

In some preferred embodiments of the invention, the protein fraction of the BLG isolate powder has an intrinsic tryptophan fluorescence emission ratio (I330/I350) of at least 1.12, preferably at least 1.13, more preferably at least 1.15, even more preferably at least 1.17, and most preferably at least 1.19.

The protein fraction can e.g. be separated from the BLG isolate powder by dissolving the BLG isolate powder in demineralised water and subjecting the solution to dialysis or ultrafiltration-based diafiltration using a filter that retains the protein. If the BLG isolate powder contains interfering levels of lipid such lipid can e.g. be removed by microfiltration. Steps of microfiltration and ultrafiltration/diafiltration can be combined to remove both lipid and small molecules from the protein fraction.

It is often preferred that a substantial amount of the BLG of the BLG isolate powder is non-aggregated BLG. Preferably at least 50% of the BLG is non-aggregated BLG. More preferably at least at least 80% of the BLG is non-aggregated BLG. Even more preferred at least 90% of the BLG is non-aggregated BLG. Most preferred, at least 95% of the BLG is non-aggregated BLG. Even more preferred approx. 100% of the BLG of the BLG isolate powder is non-aggregated BLG.

In some preferred embodiments of the invention, the BLG isolate powder has a degree of protein denaturation of at most 10%, preferably at most 8%, more preferably at most 6%, even more preferably at most 3%, even more preferably at most 1%, and most preferably at most 0.2%.

However, it may also be preferred that the BLG isolate powder has a significant level of protein denaturation, e.g. if an opaque beverage is desired. Thus, in other preferred embodiments of the invention, the BLG isolate powder has a degree of protein denaturation of at least 11%, preferably at least 20%, more preferably at least 40%, even more preferably at least 50%, even more preferably at least 75%, and most preferably at least 90%.

If BLG isolate powder has a significant level of protein denaturation it is often preferred to keep a low level of insoluble protein matter, i.e. precipitated protein matter that would settle in a beverage during storage. The level of insoluble matter is measure according to Example 1.10.

In some preferred embodiments of the invention the BLG isolate powder comprises at most 20% w/w insoluble protein matter, preferably at most 10% w/w insoluble protein matter, more preferably at most 5% w/w insoluble protein matter, even more preferably at most 3% w/w insoluble protein matter, and most preferred at most 1% w/w insoluble protein matter. It may even be preferred that the BLG isolate powder does not contain any insoluble protein matter at all.

The present inventors have found that the heat-stability at pH 3.9 of a BLG isolate powder is a good indicator for its usefulness for transparent high protein beverages. The heat-stability at pH 3.9 is measured according to Example 1.2.

It is particularly preferred that the BLG isolate powder has a heat-stability at pH 3.9 of at most 200 NTU, preferably at most 100 NTU, more preferred at most 60 NTU, even more preferred at most 40 NTU, and most preferred at most 20 NTU. Even better heat-stabilities are possible and the BLG isolate powder preferably has a heat-stability at pH 3.9 of at most 10 NTU, preferably at most 8 NTU, more preferred at most 4NTU, even more preferred at most 2 NTU.

The content of microorganisms of the BLG isolate powder is preferably kept to a minimum. However, it is a challenge to obtain both a high degree of protein nativeness and a low content of microorganism as processes for microbial reduction tend to lead to protein unfolding and denaturation. The present invention makes it possible to obtain a very low content of microorganism while at the same time maintain a high level of the nativeness of BLG.

In some embodiments of the invention, the liquid BLG isolate contains at most 500.000 CFU/g, preferably at most 100.000 CFU/g, more preferably at most 50.000 CFU/g, even more preferably at most 25.000 CFU/g.

Even lower contents of microorganisms may be preferred, thus, in some preferred embodiments of the invention, the BLG isolate powder contains at most 15000 colony-forming units (CFU)/g. Preferably, the BLG isolate powder contains at most 10000 CFU/g. More preferably, the BLG isolate powder contains at most 5000 CFU/g. Even more preferably, the BLG isolate powder contains at most 1000 CFU/g. Even more preferably, the BLG isolate powder contains at most 300 CFU/g. Most preferably, the BLG isolate powder contains at most 100 CFU/g such as e.g. at most 10 CFU/g. In a particularly preferred embodiment the powder is sterile. A sterile BLG isolate powder may e.g. be prepared by combining several physical microbial reduction processes during the production of the BLG isolate powder, such as e.g. microfiltration and heat-treatment at acidic pH. The drying is preferably performed in an aseptic drying system, such as e.g. an aseptic spray-dryer.

In some preferred embodiments of the invention, the BLG isolate powder has a pH in the range of i) 2-4.9, ii) 6.1-8.5, or iii) 5.0-6.0 and comprises:

- total protein in an amount of at least 30% w/w, preferably at least 80% w/w, and even more preferably at least 90% w/w
- beta-lactoglobulin (BLG) in an amount of at least 85% w/w relative to total protein, preferably at least 90% w/w,
- water in an amount of at most 6% w/w,
- lipid in an amount of at most 2% w/w, preferably at most 0.5% w/w,

said BLG isolate powder having:

- an intrinsic tryptophan fluorescence emission ratio (I330/I350) of at least 1.11,
- a degree of protein denaturation of at most 10%, and
- a heat-stability at pH 3.9 of at most 200 NTU.

In some preferred embodiments of the invention, the BLG isolate powder has a pH in the range of i) 2-4.9 or ii) 6.1-8.5 and comprises:

- total protein in an amount of at least 30% w/w, preferably at least 80% w/w, and even more preferably at least 90% w/w
- beta-lactoglobulin (BLG) in an amount of at least 85% w/w relative to total protein, preferably at least 90% w/w, and more preferably at least 94% w/w relative to total protein
- water in an amount of at most 6% w/w,
- lipid in an amount of at most 2% w/w, preferably at most 0.5% w/w,

said BLG isolate powder having:

- an intrinsic tryptophan fluorescence emission ratio (I330/I350) of at least 1.11,
- a degree of protein denaturation of at most 10%, preferably at most 5%, and
- a heat-stability at pH 3.9 of at most 70 NTU, preferably at most 50 NTU and even more preferably at most 40 NTU.

In some preferred embodiments of the invention the BLG isolate powder has a pH in the range of i) 2-4.9 or ii) 6.1-8.5 and comprises:

- total protein in an amount of at least 30% w/w,
- beta-lactoglobulin (BLG) in an amount of at least 85% w/w relative to total protein, preferably at least 90% w/w,
- water in an amount of at most 6% w/w,

said BLG isolate powder having:

- a bulk density of at least 0.2 g/cm$^3$,
- an intrinsic tryptophan fluorescence emission ratio (I330/I350) of at least 1.11,
- a degree of protein denaturation of at most 10%, and
- a heat-stability at pH 3.9 of at most 200 NTU.

In other preferred embodiments of the invention, the BLG isolate powder has a pH in the range of 2-4.9 and comprises:

- total protein in an amount of at least 80% w/w, preferably at least 90% w/w, and even more preferably at least 94% w/w
- beta-lactoglobulin (BLG) in an amount of at least 85% w/w relative to total protein, preferably at least 90% w/w, and even more preferably at least 94% w/w relative to total protein,
- water in an amount of at most 6% w/w,
- lipid in an amount of at most 2% w/w, preferably at most 0.5% w/w,

said BLG isolate powder having:

- a bulk density of at least 0.2 g/cm$^3$, preferably at least 0.3 g/cm$^3$, and more preferably at least 0.4 g/cm$^3$,
- an intrinsic tryptophan fluorescence emission ratio (I330/I350) of at least 1.11,
- a degree of protein denaturation of at most 10%, preferably at most 5%, and more preferably at most 2%, and
- a heat-stability at pH 3.9 of at most 50 NTU, preferably at most 30 NTU and even more preferably at most 10 NTU.

In further preferred embodiments of the invention, the BLG isolate powder has a pH in the range of 3.0-4.3, preferably in the range of 3.6-4.1 and comprises:

- total protein in an amount of at least 80% w/w, preferably at least 90% w/w, and even more preferably at least 94% w/w
- beta-lactoglobulin (BLG) in an amount of at least 90% w/w relative to total protein, preferably at least 92% w/w, and even more preferably at least 94% w/w relative to total protein,
- water in an amount of at most 6% w/w,
- lipid in an amount of at most 2% w/w, preferably at most 0.5% w/w,

said BLG isolate powder having:

- a bulk density of at least 0.2 g/cm$^3$, preferably at least 0.3 g/cm$^3$, and more preferably at least 0.4 g/cm$^3$,
- an intrinsic tryptophan fluorescence emission ratio (I330/I350) of at least 1.11, preferably at least 1.13
- a degree of protein denaturation of at most 10%, preferably at most 5%, and more preferably at most 2%, and
- a heat-stability at pH 3.9 of at most 50 NTU, preferably at most 30 NTU and even more preferably at most 10 NTU.

In yet other preferred embodiments of the invention, the BLG isolate powder has a pH in the range of 6.1-8.5 and comprises:

- total protein in an amount of at least 80% w/w, preferably at least 90% w/w, and even more preferably at least 94% w/w
- beta-lactoglobulin (BLG) in an amount of at least 85% w/w relative to total protein, preferably at least 90% w/w, and even more preferably at least 94% w/w relative to total protein,
- water in an amount of at most 6% w/w,
- lipid in an amount of at most 2% w/w, preferably at most 0.5% w/w,

said BLG isolate powder having:

- a bulk density of at least 0.2 g/cm$^3$, preferably at least 0.3 g/cm$^3$, and more preferably at least 0.4 g/cm$^3$,
- a degree of protein denaturation of at most 10%, preferably at most 5%, and more preferably at most 2%, and
- a heat-stability at pH 3.9 of at most 50 NTU, preferably at most 30 NTU, and even more preferably at most 10 NTU.

In further preferred embodiments of the invention, the BLG isolate powder has a pH in the range of 6.1-8.5 and comprises:

- total protein in an amount of at least 80% w/w, preferably at least 90% w/w, and even more preferably at least 94% w/w
- beta-lactoglobulin (BLG) in an amount of at least 85% w/w relative to total protein, preferably at least 90% w/w, and even more preferably at least 94% w/w relative to total protein,
- water in an amount of at most 6% w/w,
- lipid in an amount of at most 2% w/w, preferably at most 0.5% w/w,

said BLG isolate powder having:

- a bulk density of at least 0.2 g/cm$^3$, preferably at least 0.3 g/cm$^3$, and more preferably at least 0.4 g/cm$^3$,
- a degree of protein denaturation of at most 10%, preferably at most 5%, and more preferably at most 2%, and
- a heat-stability at pH 3.9 of at most 50 NTU, preferably at most 30 NTU, and even more preferably at most 10 NTU.

In further preferred embodiments of the invention, the BLG isolate powder has a pH in the range of 5.0-6.0 and comprises:

- total protein in an amount of at least 80% w/w, preferably at least 90% w/w, and even more preferably at least 94% w/w,
- beta-lactoglobulin (BLG) in an amount of at least 85% w/w relative to total protein, preferably at least 90% w/w, and even more preferably at least 94% w/w relative to total protein,
- water in an amount of at most 6% w/w,
- lipid in an amount of at most 2% w/w, preferably at most 0.5% w/w,

said BLG isolate powder having:

- a bulk density of at least 0.2 g/cm$^3$, preferably at least 0.3 g/cm$^3$, and more preferably at least 0.4 g/cm$^3$,
- a degree of protein denaturation of at most 10%, preferably at most 5%, and more preferably at most 2%,
- a heat-stability at pH 3.9 of at most 50 NTU, preferably at most 30 NTU, and even more preferably at most 10 NTU, and
- preferably, a BLG crystallinity of less than 10%.

In some preferred embodiments of the invention, the BLG isolate powder has a pH in the range of i) 3.0-4.3, ii) 6.5-7.5, or iii) 5.0-6.0 and comprises:

- total protein in an amount of at least 90% w/w, preferably at least 92% w/w, and even more preferably at least 94% w/w
- beta-lactoglobulin (BLG) in an amount of at least 92% w/w relative to total protein, preferably at least 94% w/w,
- water in an amount of at most 6% w/w,
- lipid in an amount of at most 0.5% w/w, preferably at most 0.1% w/w,

said BLG isolate powder having:

- an intrinsic tryptophan fluorescence emission ratio (I330/I350) of at least 1.15,
- a degree of protein denaturation of at most 5%,

- a heat-stability at pH 3.9 of at most 40 NTU, and
- at most 15000 colony-forming units/g, preferably at most 1000 colony-forming units/g more preferably at most 100 colony-forming units/g, and most preferably said BLG isolate powder is sterile.

In some preferred embodiments of the invention, the BLG isolate powder has a pH in the range of i) 3.0-4.3, ii) 6.5-7.5, or iii) 5.0-6.0 and comprises:

- total protein in an amount of at least 90% w/w, preferably at least 92% w/w, and even more preferably at least 94% w/w
- beta-lactoglobulin (BLG) in an amount of at least 92% w/w relative to total protein, preferably at least 94% w/w,
- water in an amount of at most 6% w/w,
- lipid in an amount of at most 0.5% w/w, preferably at most 0.1% w/w,

said BLG isolate powder having:

- a degree of protein denaturation of at most 5%,
- a heat-stability at pH 3.9 of at most 40 NTU, and
- at most 15000 colony-forming units/g, preferably at most 1000 colony-forming units/g more preferably at most 100 colony-forming units/g, and most preferably said BLG isolate powder is sterile.

In some preferred embodiments of the invention, the BLG isolate powder has a pH in the range of i) 3.0-4.3, ii) 6.5-7.5, or iii) 5.0-6.0 and comprises:

- total protein in an amount of at least 90% w/w, preferably at least 92% w/w, and even more preferably at least 94% w/w
- beta-lactoglobulin (BLG) in an amount of at least 92% w/w relative to total protein, preferably at least 94% w/w,
- water in an amount of at most 6% w/w,
- lipid in an amount of at most 0.5% w/w, preferably at most 0.1% w/w,

said BLG isolate powder having:

- an intrinsic tryptophan fluorescence emission ratio (I330/I350) of at least 1.15,
- a heat-stability at pH 3.9 of at most 40 NTU, and
- at most 15000 colony-forming units/g, preferably at most 1000 colony-forming units/g more preferably at most 100 colony-forming units/g, and most preferably said BLG isolate powder is sterile.

In some preferred embodiments of the invention, the BLG isolate powder has a pH in the range of i) 3.0-4.3, ii) 6.5-7.5, or iii) 5.0-6.0 and comprises:

- total protein in an amount of at least 90% w/w, preferably at least 92% w/w, and even more preferably at least 94% w/w
- beta-lactoglobulin (BLG) in an amount of at least 92% w/w relative to total protein, preferably at least 94% w/w,
- water in an amount of at most 6% w/w,
- lipid in an amount of at most 0.5% w/w, preferably at most 0.1% w/w,

said BLG isolate powder having:

- an intrinsic tryptophan fluorescence emission ratio (I330/I350) of at least 1.15,
- a degree of protein denaturation of at most 5%, and
- at most 15000 colony-forming units/g, preferably at most 1000 colony-forming units/g more preferably at most 100 colony-forming units/g, and most preferably said BLG isolate powder is sterile.

In some preferred embodiments of the invention, the BLG isolate powder has a pH in the range of i) 3.0-4.3, ii) 6.5-7.5, or iii) 5.0-6.0 and comprises:

- total protein in an amount of at least 90% w/w, preferably at least 92% w/w, and even more preferably at least 94% w/w
- beta-lactoglobulin (BLG) in an amount of at least 92% w/w relative to total protein, preferably at least 94% w/w,
- water in an amount of at most 6% w/w,
- lipid in an amount of at most 0.5% w/w, preferably at most 0.1% w/w,

said BLG isolate powder having:

- an intrinsic tryptophan fluorescence emission ratio (I330/I350) of at least 1.15,
- a degree of protein denaturation of at most 5%,
- a heat-stability at pH 3.9 of at most 40 NTU.

In some preferred embodiments of the invention, the BLG isolate powder has a pH in the range of i) 3.0-4.3, ii) 6.5-7.5, or iii) 5.0-6.0 and comprises:

- total protein in an amount of at least 90% w/w, preferably at least 92% w/w, and even more preferably at least 94% w/w
- beta-lactoglobulin (BLG) in an amount of at least 92% w/w relative to total protein, preferably at least 94% w/w,
- water in an amount of at most 6% w/w,
- lipid in an amount of at most 0.5% w/w, preferably at most 0.1% w/w,

said BLG isolate powder having:

- an intrinsic tryptophan fluorescence emission ratio (I330/I350) of at least 1.15,
- a heat-stability at pH 3.9 of at most 40 NTU, and
- at most 15000 colony-forming units/g, preferably at most 1000 colony-forming units/g more preferably at most 100 colony-forming units/g, and most preferably said BLG isolate powder is sterile.

In some preferred embodiments of the invention, the BLG isolate powder has a pH in the range of i) 3.0-4.3 or ii) 6.3-7.5 and comprises:

- total protein in an amount of at least 30% w/w, preferably at least 50% w/w, and even more preferably at least 80% w/w
- beta-lactoglobulin (BLG) in an amount of at least 90% w/w, and more preferably at least 94% w/w relative to total protein
- water in an amount of at most 6% w/w,
- lipid in an amount of at most 0.5% w/w, preferably at most 0.1% w/w,

said BLG isolate powder having:

- an intrinsic tryptophan fluorescence emission ratio (I330/I350) of at least 1.15,
- a degree of protein denaturation of at most 5%, preferably at most 2%, and
- a heat-stability at pH 3.9 of at most 40 NTU, preferably at most 20 NTU and even more preferably at most 10 NTU.

In some preferred embodiments of the invention the BLG isolate powder has a pH in the range of i) 3.0-4.3 or ii) 6.3-7.5 and comprises:

- total protein in an amount of at least 30% w/w,
- beta-lactoglobulin (BLG) in an amount of at least 85% w/w relative to total protein, preferably at least 90% w/w,
- water in an amount of at most 6% w/w,

said BLG isolate powder having:

- an intrinsic tryptophan fluorescence emission ratio (I330/I350) of at least 1.15,
- a degree of protein denaturation of at most 5%, and
- a heat-stability at pH 3.9 of at most 40 NTU.

In other preferred embodiments of the invention, the BLG isolate powder has a pH in the range of 3.0-4.3 and comprises:

- total protein in an amount of at least 90% w/w, and preferably at least 94% w/w
- beta-lactoglobulin (BLG) in an amount of at least 94% w/w, and preferably at least 96% w/w relative to total protein,
- water in an amount of at most 6% w/w,
- lipid in an amount of at most 0.5% w/w, preferably at most 0.1% w/w,

said BLG isolate powder having:

- a bulk density of 0.45-0.8 $g/cm^3$, preferably 0.50-0.6 $g/cm^3$,

- an intrinsic tryptophan fluorescence emission ratio (I330/I350) of at least 1.15,
- a degree of protein denaturation of at most 5%, and more preferably at most 2%, and
- a heat-stability at pH 3.9 of at most 30 NTU and preferably at most 10 NTU.

In yet other preferred embodiments of the invention, the BLG isolate powder has a pH in the range of 6.3-7.5 and comprises:

- total protein in an amount of at least 90% w/w, and even more preferably at least 94% w/w
- beta-lactoglobulin (BLG) in an amount of at least 94% w/w, and even more preferably at least 96% w/w relative to total protein,
- water in an amount of at most 6% w/w,
- lipid in an amount of at most 0.5% w/w, preferably at most 0.1% w/w,

said BLG isolate powder having:

- a bulk density of 0.45-0.8 $g/cm^3$, preferably 0.50-0.6 $g/cm^3$,
- a degree of protein denaturation of at most 10%, preferably at most 5%, and more preferably at most 2%, and
- a heat-stability at pH 3.9 of at most 50 NTU, preferably at most 30 NTU, and even more preferably at most 10 NTU.

In further preferred embodiments of the invention, the BLG isolate powder has a pH in the range of 5.0-6.0 and comprises:

- total protein in an amount of at least 90% w/w, and even more preferably at least 94% w/w,
- beta-lactoglobulin (BLG) in an amount of at least 90% w/w, and even more preferably at least 94% w/w relative to total protein,
- water in an amount of at most 6% w/w,
- lipid in an amount of at most 0.5% w/w, preferably at most 0.1% w/w,

said BLG isolate powder having:

- a bulk density of 0.45-0.8 $g/cm^3$, preferably 0.50-0.6 $g/cm^3$,
- a degree of protein denaturation of at most 10%, preferably at most 5%, and more preferably at most 2%,
- a heat-stability at pH 3.9 of at most 50 NTU, preferably at most 30 NTU, and even more preferably at most 10 NTU, and
- preferably, a BLG crystallinity of less than 10%, and more preferably at most 1%.

Yet an aspect of the invention pertains to method of producing a dried BLG isolate powder containing BLG in an amount of at least 85% w/w relative to total protein, the method comprising the steps of:

a) providing a liquid BLG isolate having

i) a pH in the range of 2-4.9,
ii) a pH of in the range of 6.1-8.5, or
iii) a pH of in the range of 5.0-6.0

said liquid BLG isolate containing BLG in an amount of at least 85 w/w relative to total protein,
b) optionally, subjecting the liquid BLG isolate to a physical microbial reduction,
c) drying the liquid BLG isolate, preferably by spray-drying.

The liquid BLG isolate is preferably an edible composition.
The liquid BLG isolate is preferably prepared from mammal milk, and preferably from ruminant milk such as e.g. milk from cow, sheep, goat, buffalo, camel, llama, horse, and/or deer. Protein derived from bovine milk is particularly preferred. The BLG is therefore preferably bovine BLG.
In some preferred embodiments of the invention, the liquid BLG isolate comprises BLG in an amount of at least 92% w/w relative to total protein, preferably at least 95% w/w, more preferably at least 97% w/w, even more preferably at least 98%, and most preferably BLG in an amount of at least 99.5% w/w relative to total protein.
In some preferred embodiments of the invention, the liquid BLG isolate comprises BLG in an amount of at least 97.5% w/w relative to total protein, preferably at least 98.0% w/w, more preferably at least 98.5% w/w, even more preferably at least 99.0%, and most preferably BLG in an amount of at least 99.7% w/w relative to total protein, such as approx. 100.0% w/w relative to total protein.

In some preferred embodiments of the invention, the liquid BLG isolate comprises total protein in an amount of at least 5% w/w, preferably at least 10% w/w, more preferably at least 15% w/w, even more preferably at least 20%, and most preferably total protein in an amount of at least 30% w/w.

In some preferred embodiments of the invention, the liquid BLG isolate comprises total protein in an amount in the range of 5-40% w/w, preferably in the range of 10-35% w/w, more preferably in the range of 15-30% w/w, even more preferably in the range of 20-25% w/w.

The present inventors have observed that an increasing BLG concentration in the liquid BLG isolate gives rise to spray-dried powders having a higher bulk density and it is therefore preferred to have a relatively high concentration of BLG in the liquid BLG isolate.

Thus, in other preferred embodiments of the invention, the liquid BLG isolate comprises total protein in an amount in the range of 10-40% w/w, preferably in the range of 20-38% w/w, more preferably in the range of 24-36% w/w, even more preferably in the range of 28-34% w/w.

In some preferred embodiments of the invention, the sum of alpha-lactalbumin (ALA) and caseinomacropeptide (CMP) comprises at least 40% w/w of the non-BLG protein of the liquid BLG isolate, preferably at least 60% w/w, even more preferably at least 70% w/w, and most preferably at least 90% w/w of the non-BLG protein of the liquid BLG isolate.

In some preferred embodiments of the invention, ALA comprises at most 80% w/w of the non-BLG protein of the liquid BLG isolate, preferably at most 60% w/w, even more preferably at most 40% w/w, and most preferably at most 30% w/w of the non-BLG protein of the liquid BLG isolate.

Even lower contents of ALA may be preferred, thus in some preferred embodiments of the invention, ALA comprises at most 20% w/w of the non-BLG protein of the liquid BLG isolate, preferably at most 15% w/w, even more preferably at most 10% w/w, and most preferably at most 5% w/w of the non-BLG protein of the liquid BLG isolate.

In other preferred embodiments of the invention, each main non-BLG whey protein of the liquid BLG isolate is present in a weight percentage relative to total protein which is at most 25% of its weight percentage relative to total protein in a standard whey protein concentrate from sweet whey, preferably at most 20%, more preferably at most 15%, even more preferably at most 10%, most preferably at most 6%.

Even lower concentrations of the main non-BLG whey proteins may be desirable. Thus in additional preferred embodiments of the invention each main non-BLG whey protein of the liquid BLG isolate is present in a weight percentage relative to total protein which is at most 4% of its weight percentage relative to total protein in a standard whey protein concentrate from sweet whey, preferably at most 3%, more preferably at most 2%, even more preferably at most 1%.

The inventors have seen indications that a low level of lactoferrin and/or lactoperoxidase is particularly advantageous for obtaining a colour-neutral whey protein product.

Thus in some preferred embodiments of the invention, lactoferrin is present in the liquid BLG isolate in a weight percentage relative to total protein which is at most 25% of its weight percentage relative to total protein in a standard whey protein concentrate from sweet whey, preferably at most 20%, more preferably at most 15%, even more preferably at most 10%, most preferably at most 6%. Even lower concentrations of lactoferrin may be desirable. Thus, in additional preferred embodiments of the invention, lactoferrin is present in a weight percentage relative to total protein which is at most 4% of its weight percentage relative to total protein in a standard whey protein concentrate from sweet whey, preferably at most 3%, more preferably at most 2%, even more preferably at most 1%.

Similarly, in some preferred embodiments of the invention, lactoperoxidase is present in the liquid BLG isolate in a weight percentage relative to total protein which is at most 25% of its weight percentage relative to total protein in a standard whey protein concentrate from sweet whey, preferably at most 20%, more preferably at most 15%, even more preferably at most 10%, most preferably at most 6%. Even lower concentrations of lactoperoxidase may be desirable. Thus, in additional preferred embodiments of the invention, lactoperoxidase is present in a weight percentage relative to total protein which is at most 4% of its weight percentage relative to total protein in a standard whey protein concentrate from sweet whey, preferably at most 3%, more preferably at most 2%, even more preferably at most 1%.

In some preferred embodiments of the invention, the liquid BLG isolate comprises a total solids content in an amount in the range of 5-50% w/w, preferably in the range of 10-40% w/w, more preferably in the range of 15-35% w/w, even more preferably in the range of 20-30% w/w.

The fraction of the liquid BLG isolate that does not contribute to the total solids preferably essentially consists of, or even consists of, water.

In some preferred embodiments of the invention, the liquid BLG isolate comprises a water content in an amount in the range of 50-95% w/w, preferably in the range of 60-90% w/w, more preferably in the range of 65-85% w/w, even more preferably in the range of 70-80% w/w.

In some preferred embodiments of the invention, the liquid BLG isolate comprises carbohydrate in an amount of at most 60% w/w, preferably at most 50% w/w, more preferably at most 20% w/w, even more preferably at most 10% w/w, even more preferably at most 1% w/w, and most preferably at most 0.1%. The liquid BLG isolate may for example contain carbohydrates, such as e.g. lactose, oligosaccharides and/or hydrolysis products of lactose (i.e. glucose and galactose), sucrose, and/or maltodextrin.

In some preferred embodiments of the invention, the liquid BLG isolate comprises lipid in an amount of at most 10% w/w, preferably at most 5% w/w, more preferably at most 2% w/w, and even more preferably at most 0.1% w/w.

The present inventors have found that it can be advantageous to control the mineral content to reach some of the desired properties of the liquid BLG isolate.

In some preferred embodiments of the invention, the sum of the amounts of Na, K, Mg, and Ca of the liquid BLG isolate is at most 10 mmol/g protein. Preferably, the sum of the amounts of Na, K, Mg, and Ca of the liquid BLG isolate is at most 6 mmol/g protein, more preferably at most 4 mmol/g protein, even more preferably at most 2 mmol/g protein.

In other preferred embodiments of the invention, the sum of the amounts of Na, K, Mg, and Ca of the liquid BLG isolate is at most 1.0 mmol/g protein. Preferably, the sum of the amounts of Na, K, Mg, and Ca of the liquid BLG isolate is at most 0.6 mmol/g protein, more preferably at most 0.4 mmol/g protein, even more preferably at most 0.2 mmol/g protein, and most preferably at most 0.1 mmol/g protein.

In other preferred embodiments of the invention, the sum of the amounts of Mg and Ca of the liquid BLG isolate is at most 5 mmol/g protein. Preferably, the sum of the amounts of Mg and Ca of the liquid BLG isolate is at most 3 mmol/g protein, more preferably at most 1.0 mmol/g protein, even more preferably at most 0.5 mmol/g protein.

In other preferred embodiments of the invention, the sum of the amounts of Mg and Ca of the liquid BLG isolate is at most 0.3 mmol/g protein. Preferably, the sum of the amounts of Mg and Ca of the liquid BLG isolate is at most 0.2 mmol/g protein, more preferably at most 0.1 mmol/g protein, even more preferably at most 0.03 mmol/g protein, and most preferably at most 0.01 mmol/g protein.

The inventors have found that it is possible to make low phosphorus/low potassium variants of the BLG isolate powder that are particularly useful to patients with kidney diseases. To make such a product the liquid BLG isolate has to have an equally low content of phosphorus and potassium.

Thus, in some preferred embodiments of the invention, the liquid BLG isolate has a total content of phosphorus of at most 100 mg phosphorus per 100 g protein. Preferably, the liquid BLG isolate has a total content of at most 80 mg phosphorus per 100 g protein. More preferably, the liquid BLG isolate has a total content of at most 50 mg phosphorus per 100 g protein. Even more preferably, the liquid BLG isolate has a total content of phosphorus of at most 20 mg phosphorus per 100 g protein. The liquid BLG isolate has a total content of phosphorus of at most 5 mg phosphorus per 100 g protein.

In some preferred embodiments of the invention, the liquid BLG isolate comprises at most 600 mg potassium per 100 g protein. More preferably, the liquid BLG isolate comprise at most 500 mg potassium per 100 g protein. More preferably, the liquid BLG isolate comprises at most 400 mg potassium per 100 g protein. More preferably, the liquid BLG isolate comprises at most 300 mg potassium per 100 g protein. Even more preferably, the liquid BLG isolate at most 200 mg potassium per 100 g protein. Even more preferably, the liquid BLG isolate comprises at most 100 mg potassium per 100 g protein. Even more preferably, the liquid BLG isolate comprises at most 50 mg potassium per 100 g protein and even more preferably, the liquid BLG isolate comprises at most 10 mg potassium per 100 g protein.

The content of phosphorus relates to the total amount of elemental phosphorus of the composition in question and is determined according to Example 1.19. Similarly, the content of potassium relates to the total amount of elemental potassium of the composition in question and is determined according to Example 1.19.

In some preferred embodiments of the invention, the liquid BLG isolate comprises at most 100 mg phosphorus/100 g protein and at most 700 mg potassium/100g protein, preferably at most 80mg phosphorus/100 g protein and at most 600mg potassium/ 100g protein, more preferably at most 60mg phosphorus/100 g protein and at most 500mg potassium/ 100g protein, more preferably at most 50mg phosphorus/100 g protein and at most 400mg potassium/ 100g protein, or more preferably at most 20mg phosphorus/100 g protein and at most 200mg potassium/100g protein, or even more preferably at most 10mg phosphorus/100 g protein and at most 50mg potassium/ 100g protein. In some preferred embodiments of the invention, the liquid BLG isolate comprises at most 100mg phosphor/100 g protein and at most 340mg potassium/100g protein.

The low phosphorus and/or low potassium compositions according to the present invention may be used as a food ingredient for the production of a food product for patients groups that have a reduced kidney function.

In some preferred embodiments of the invention, the liquid BLG isolate has a pH in the range of 2-4.9, preferably 2.5-4.7, more preferably 2.8-4.4, even more preferably 3.0-4.0, and most preferably 3.4-3.9.

In other preferred embodiments of the invention, the liquid BLG isolate has a pH in the range of 6.1-8.5, preferably 6.2-8.0, more preferably 6.3-7.7, and even more preferably 6.5-7.5.

In yet other preferred embodiments of the invention, the liquid BLG isolate has a pH in the range of 5.0-6.0, preferably 5.1-5.9, more preferably 5.2-5.8, and even more preferably 5.3-5.7. When the liquid BLG isolate is in the pH range 5.0-6.0, it is often preferred that the liquid BLG isolate does not contain any BLG crystals. This may be accomplished by making sure that the liquid BLG isolate is below the saturation point for BLG, e.g. by increasing the temperature and/or by adding salt. Alternatively, it is possible to keep the liquid BLG isolate crystal-free even if it is supersaturated with respect to BLG as long as it is kept in the meta-stable zone and no crystallisation promoting agents are brought in contact with the liquid BLG isolate.

The liquid BLG isolate preferably has a low content of microorganisms and this is particularly possible if the BLG-enriched composition already is low in microorganisms.

In some embodiments of the invention, the liquid BLG isolate contains at most 500.000 CFU/g, preferably at most 100.000 CFU/g, more preferably at most 50.000 CFU/g, even more preferably at most 10.000 CFU/g.

Thus, in some preferred embodiments of the invention, the liquid BLG isolate contain at most 1000 colony-forming units (CFU)/g. Preferably, the liquid BLG isolate contains at most 600 CFU/g. More preferred, the liquid BLG isolate contains at most 300 CFU/g. Even more preferably, the liquid BLG isolate contains at most 100 CFU/g. Even more preferably, the liquid BLG isolate contains at most 50 CFU/g. Most preferably, the liquid BLG isolate contains at most 20 CFU/gm such as e.g. at most 10 CFU/g. In a particularly preferred embodiment the powder is sterile. A sterile liquid BLG isolate may e.g. be prepared by combining several physical microbial reduction processes during the production of the BLG isolate powder, such as e.g. microfiltration and heat-treatment at low pH (e.g. at most pH 4.0).

The preparation of a BLG isolate powder with a low degree of protein unfolding requires that the liquid BLG isolate already has a low degree of protein unfolding, as the unfolding of BLG appears to be an irreversible process.

If a BLG isolate powder or liquid BLG isolate with a low degree of BLG unfolding is required, the liquid BLG isolate preferably has an intrinsic tryptophan fluorescence emission ratio (I330/I350) of at least 1.11.

In some preferred embodiments of the invention, the liquid BLG isolate has an intrinsic tryptophan fluorescence emission ratio (I330/I350) of at least 1.12, preferably at least 1.13, more preferably at least 1.15, even more preferably at least 1.17, and most preferably at least 1.19.

If liquid BLG isolate contains considerable amounts of non-protein matter, it is preferred to isolate the protein fraction before measuring the intrinsic tryptophan fluorescence emission ratio. Thus in some preferred embodiments of the invention the protein fraction of the liquid BLG isolate has an intrinsic tryptophan fluorescence emission ratio (I330/I350) of at least 1.11.

Preferably the protein fraction of the liquid BLG isolate may have an intrinsic tryptophan fluorescence emission ratio (I330/I350) of at least 1.12, more preferably at least 1.13, even more preferably at least 1.15, even more preferably at least 1.17, and most preferably at least 1.19.

The protein fraction can e.g. be separated from the BLG isolate powder by subjecting it to dialysis or ultrafiltration-based diafiltration using a filter that retains the protein.

The preparation of a BLG isolate powder having a low degree of protein unfolding requires that the liquid BLG isolate already has a low degree of protein denaturation, as protein denaturation of BLG appears to be an irreversible process. Thus, in some preferred embodiments of the invention, the liquid BLG isolate has a degree of protein denaturation of at most 10% w/w, preferably at most 6% w/w, more preferably at most 4% w/w, even more preferably at most 2, and most preferably at most 1% w/w.

It is often preferred that a substantial amount of the BLG of the liquid BLG isolate is non-aggregated BLG. Preferably at least 50% of the BLG is non-aggregated BLG. More preferably at least at least 80% of the BLG is non-aggregated BLG. Even more preferred at least 90% of the BLG is non-aggregated BLG. Most preferred, at least 95% of the BLG is non-aggregated BLG. Even more preferred approx. 100% of the BLG of the liquid BLG isolate is non-aggregated BLG.

However, it may also be preferred that the liquid BLG isolate has a significant level of protein denaturation, e.g. if an opaque beverage is desired. Thus, in other preferred embodiments of the invention, the BLG isolate powder has a degree of protein denaturation of at least 11%, preferably at least 20%, more preferably at least 40%, even more preferably at least 50%, even more preferably at least 75%, and most preferably at least 90%.

If liquid BLG isolate has a significant level of protein denaturation it is often preferred to keep a low level of insoluble protein matter, i.e. precipitated protein matter that would settle in a beverage during storage. The level of insoluble matter is measure according to Example 1.10.

In some preferred embodiments of the invention the liquid BLG isolate comprises at most 20% w/w insoluble protein matter, preferably at most 10% w/w insoluble protein matter, more preferably at most 5% w/w insoluble protein matter, even more preferred at most 3% w/w insoluble protein matter, and most preferred at most 1% w/w insoluble protein matter. It may even be preferred that the liquid BLG isolate does not contain any insoluble protein matter at all.

As mentioned above, the present inventors have found that the heat-stability at pH 3.9 of a liquid BLG isolate is a good indicator for its usefulness for transparent high protein beverages. The heat-stability at pH 3.9 is measured according to Example 1.2.

It is particularly preferred that the liquid BLG isolate has a heat-stability at pH 3.9 of at most 200 NTU, preferably at most 100 NTU, more preferred at most 60 NTU, even more preferred at most 40 NTU, and most preferred at most 20 NTU. Even better heat-stabilities are possible and the liquid BLG isolate preferably has a heat-stability at pH 3.9 of at most 10 NTU, preferably at most 8 NTU, more preferred at most 4NTU, even more preferred at most 2 NTU.

In some preferred embodiments of the invention the liquid BLG isolate has turbidity of at most 200 NTU, preferably at most 100 NTU, more preferably at most 50 NTU, even more preferably at most 20 NTU, even more preferably at most 10 NTU, and most preferably at most 2 NTU.

In other preferred embodiments of the invention the liquid BLG isolate has turbidity of at more than 200 NTU, preferably at least 400 NTU, more preferably at least 800 NTU, even more preferably at least 1000 NTU, even more preferably at least 2000 NTU, and most preferably at least 5000 NTU. Such liquid BLG isolates are particularly preferred for production of opaque beverages.

The present inventors have observed that the present liquid BLG isolate surprisingly has a lower viscosity than a comparable liquid WPI. The inventors have found that this makes the liquid BLG isolate particularly suitable as a high protein beverage as it makes it possible to obtain a high protein content without experiencing an unpleasantly high viscosity.

In some preferred embodiments of the invention the liquid BLG isolate has a viscosity at 15 degrees C and a shear rate of 300 s$^{-1}$ of visc(p) $\pm$ 50%, more preferably visc(p) $\pm$ 40%, even more preferably visc(p) $\pm$ 30%, and most preferably visc(p) $\pm$ 25%.

visc(p) is defined as:

$$\text{visc(p) = For } p \leq 23\%: 0.3556e^{0.1262*p} \text{ ; For } p>23\%: 0.0254*e^{0.24*p}$$

p is the total protein content of the liquid BLG isolate expressed in % w/w, so if the protein content e.g. is 31 % w/w, then p is 31.

This means that if the liquid BLG isolate (having the protein content p) e.g. has a viscosity at 15 degrees C and a shear rate of 300 s$^{-1}$ of visc(p) $\pm$ 25%, then the viscosity of the liquid BLG isolate is at least visc(p)-25% and at most visc(p) +25%. If the protein content, p, of the liquid BLG isolate e.g. is 31% w/w the minimum and maximum viscosities of this example are:

$$\text{Minimum viscosity (in cP): } 0.0254*e^{0.24*31} - 25\% = 43 \text{ cP} - 25\% = 32 \text{ cP}$$

$$\text{Maximum viscosity (in cP): } 0.0254*e^{0.24*31} + 25\% = 43 \text{ cP} - 25\% = 54 \text{ cP}$$

In other preferred embodiments of the invention the liquid BLG isolate has a viscosity at 15 degrees C and a shear rate of 300 s$^{-1}$ of visc(p) $\pm$ 20%, more preferably visc(p) $\pm$ 15%, even more preferably visc(p) $\pm$ 10%, and most preferably visc(p) $\pm$ 5%.

Viscosity of a liquid BLG isolate is measured according to Example 1.8, however using a temperature of 15 degrees C and a shear rate of 300 s$^{-1}$.

In some preferred embodiments of the invention the liquid BLG isolate has a viscosity at 15 degrees C and a shear rate of 300 s$^{-1}$ of:

- at least visc(p) - 20%, and
- at most visc$_{max}$(p) - 20%.

In other preferred embodiments of the invention the liquid BLG isolate has a viscosity at 15 degrees C and a shear rate of 300 s$^{-1}$ of:

- at least visc(p) - 10%, and
- at most visc$_{max}$(p) - 40%.

In other preferred embodiments of the invention the liquid BLG isolate has a viscosity at 15 degrees C and a shear rate of 300 s$^{-1}$ of:

- at least visc(p) - 10%, and
- at most visc$_{max}$(p) - 50%.

Visc$_{max}$(p) is defined as: visc$_{max}$(p) $\leq$ 0.611*e$^{(0.1494*p)}$ cP.

In other preferred embodiments of the invention the liquid BLG isolate has a viscosity at 15 degrees C and a shear rate of 300 s$^{-1}$ of at most visc$_{max}$(p) - 10%, more preferably at most visc$_{max}$(p) - 20%, even more preferred visc$_{max}$(p) - 30%, and most preferred visc$_{max}$(p) - 50%.

In some preferred embodiments of the invention the liquid BLG isolate has a pH in the range of 2.8-4.3, and preferably 3.0-4.0 and comprises:

- total protein in an amount of 20-34% w/w, more preferably in the range of 24-32% w/w, even more preferably in the range of 28-32% w/w,
- beta-lactoglobulin (BLG) in an amount of at least 90% w/w relative to total protein, more preferably at least 94% w/w,

said BLG isolate powder preferably having one or more of the following:

- an intrinsic tryptophan fluorescence emission ratio (I330/I350) of at least 1.15,
- a degree of protein denaturation of at most 2%,
- a heat-stability at pH 3.9 of at most 20 NTU,
- is sterile, and
- a viscosity at 15 degrees C of visc(p) $\pm$ 25%, where p is the protein content in the unit % w/w and visc(p) = For p$\leq$23%: $0.3556e^{0.1262*p}$ ; For p>23%: $0.0254*e^{0.24*p}$.

In other preferred embodiments of the invention the liquid BLG isolate has a pH in the range of 6.3-8.0, and more preferably 6.5-7.5 and comprises:

- total protein in an amount of 20-34% w/w, more preferably in the range of 24-32% w/w, even more preferably in the range of 28-30% w/w,
- beta-lactoglobulin (BLG) in an amount of at least 90% w/w relative to total protein, more preferably at least 94% w/w,

said BLG isolate powder preferably having one or more of the following:

- an intrinsic tryptophan fluorescence emission ratio (I330/I350) of at least 1.15,
- a degree of protein denaturation of at most 5%,
- a heat-stability at pH 3.9 of at most 40 NTU,
- is sterile, and
- a viscosity at 15 degrees C of visc(p) $\pm$ 25%, where p is the protein content in the unit % w/w and visc(p) = For p$\leq$23%: $0.3556e^{0.1262*p}$ ; For p>23%: $0.0254*e^{0.24*p}$.

Such acidic high protein liquid BLG isolates are particularly useful for production of high quality BLG isolate powders and have both a surprisingly low viscosity relative to comparable WPIs which makes processing such as microfiltration less energy-consuming. The acidic, high protein liquid BLG isolates furthermore make it possible to produce acidic whey protein powder with a much higher bulk density than can be achieved for a traditional WPI having the same protein content (see e.g. Example 7).

The liquid BLG isolate may be provided in a number of different ways.

Typically, the provision of the liquid BLG isolate involves, or even consists of, isolating BLG from a whey protein feed to provide a BLG-enriched composition by one or more of the following methods:

- crystallisation or precipitation of BLG by salting-in,
- crystallisation or precipitation of BLG of BLG by salting-out,
- ion exchange chromatography, and
- fractionation of whey proteins by ultrafiltration.

A particularly preferred way of providing the BLG-enriched composition is by crystallisation of BLG, preferably by salting-in or alternatively by salting-out.

The whey protein feed is preferably a WPC, a WPI, an SPC, an SPI, or a combination thereof.

The term "whey protein feed" pertains to the composition from which the BLG-enriched composition and subsequently the liquid BLG isolate are derived. The chemical features and embodiments described in the context of the whey protein solution also applies to the whey protein feed with the exception that the whey protein feed typically is not supersaturated with respect to BLG and that the pH of feed is not limited to the range 5-6.

In some embodiments of the invention, the preparation of the BLG-enriched composition includes, or even consists of, high salt BLG crystallisation in the pH range 3.6-4.0 according to US 2,790,790 A1.

In other embodiments of the invention the preparation of the BLG-enriched composition includes, or even consists of, the method described by de Jongh et at (Mild Isolation Procedure Discloses New Protein Structural Properties of β-Lactoglobulin, J Dairy Sci., vol. 84(3), 2001, pages 562-571) or by Vyas et at (Scale-Up of Native β-Lactoglobulin Affinity Separation Process, J. Dairy Sci. 85:1639-1645, 2002).

However, in particularly preferred embodiments of the invention, the BLG-enriched composition is prepared by crystallisation at pH 5-6 under salting-in conditions as described in the PCT application PCT/EP2017/084553, which is incorporated herein by reference for all purposes.

In some preferred embodiments of the invention, the BLG-enriched composition is an edible BLG composition according to PCT/EP2017/084553 containing at least 90% BLG relative to total protein and preferably containing BLG crystals.

Preferably the BLG-enriched composition is prepared by a process comprising the steps of

1) providing a whey protein solution comprising non-aggregated BLG and at least one additional whey protein, said whey protein solution being supersaturated with respect to BLG and having a pH in the range of 5-6,

2) crystallising non-aggregated BLG in the supersaturated whey protein solution, and

3) separating BLG crystals from the remaining whey protein solution,

4) optionally washing BLG crystals, e.g. the separated BLG crystals obtained from step 3) or 5), and

5) optionally, re-crystallising BLG crystals, e.g. the BLG crystals obtained from step 3) or 4).

This process for preparing the BLG-enriched composition comprises the mandatory steps 1), 2), and 3) and in that sequence and may optionally contain steps 4) and/or 5) in any sequence and number of iterations. However, steps 4) and 5) often follow step 3). Alternatively or additionally, washing water may be added to the crystal-containing whey protein solution prior to separation.

The process may furthermore comprise a step of drying the BLG-enriched composition. However, it is presently preferred to use the BLG-enriched composition without drying it, to avoid the risk of damaging the protein during drying.

As said, step 1) of the crystallisation process involves providing a whey protein solution which comprises non-aggregated BLG and at least an additional whey protein.

The whey protein solution preferably contains at least one additional non-aggregated whey protein selected from the group consisting of alpha-lactalbumin, bovine serum albumin, immunoglobulins, caseinomacropeptide (CMP), osteopontin, lactoferrin, lactoperoxidase, milk fat globule membrane proteins, and combinations thereof.

In some embodiments of the invention, the whey protein solution comprises at most 10% w/w casein relative to the total amount of protein, preferably at most 5% w/w, more preferably at most 1% w/w, and even more preferably at most 0.5% casein relative to the total amount of protein. In some preferred embodiments of the invention, the whey protein solution does not contain any detectable amount of casein.

In some preferred embodiments of the invention, the whey protein solution of step 1) comprises at least 5% w/w additional whey protein relative to the total amount of protein. Preferably, the whey protein solution of step 1) comprises at least 10% w/w additional whey protein relative to the total amount of protein. More preferably, the whey protein solution of step 1) comprises at least 15% w/w additional whey protein relative to the total amount of protein. Even more preferably, the whey protein solution of step 1) comprises at least 20% w/w additional whey protein relative to the total amount of protein. Most preferably, the whey protein solution of step 1) may comprise at least 30% w/w additional whey protein relative to the total amount of protein.

In other preferred embodiments of the invention, the whey protein solution of step 1) comprises at least 1% w/w additional whey protein relative to the total amount of protein.

Preferably, the whey protein solution of step 1) comprises at least 2% w/w additional whey protein relative to the total amount of protein. Even more preferably, the whey protein solution of step 1) comprises at least 3% w/w additional whey protein relative to the total amount of protein. Most preferably, the whey protein solution of step 1) may comprise at least 4% w/w additional whey protein relative to the total amount of protein.

In yet other preferred embodiments of the invention, the whey protein solution of step 1) comprises at least 35% w/w additional whey protein relative to the total amount of protein. Preferably, the whey protein solution of step 1) may comprise at least 40% w/w additional whey protein relative to the total amount of protein. More preferably, the whey protein solution of step 1) may e.g. comprise at least 45% w/w additional whey protein relative to the total amount of protein. Even more preferably, the whey protein solution of step 1) may comprise at least 50% w/w additional whey protein relative to the total amount of protein.

In some preferred embodiments of the invention, the whey protein solution of step 1) comprises in the range of 5-90% w/w additional whey protein relative to the total amount of protein. Preferably, the whey protein solution of step 1) may comprise in the range of 10-80% w/w additional whey protein relative to the total amount of protein. The whey protein solution of step 1) may e.g. comprise in the range of 20-70% w/w additional whey protein relative to the total amount of protein. Preferably, the whey protein solution of step 1) comprises in the range of 30-70% w/w additional whey protein relative to the total amount of protein.

As said, the present inventors have found that it is possible to crystallize non-aggregated BLG without the use of organic solvents. This purification approach can also be used to refine preparations containing whey protein, which preparations have already been subjected to some BLG purification and provides simple process of increasing the purity of non-aggregated BLG even further. Thus, in some preferred embodiments of the invention, the whey protein solution of step 1) comprises in the range of 1-20% w/w additional whey protein relative to the total amount of protein. Preferably, the whey protein solution of step 1) may comprise in the range of 2-15% w/w additional whey protein relative to the total amount of protein. Even more preferably, the whey protein solution of step 1) may e.g. comprise in the range of 3-10% w/w additional whey protein relative to the total amount of protein.

In some embodiments of the invention, the whey protein solution of step 1) comprises at least 5% w/w non-aggregated ALA relative to the total amount of protein. Preferably, the whey protein solution of step 1) comprises at least 10% w/w non-aggregated ALA relative to the total amount of protein. Even more preferably, the whey protein solution of step 1) comprises at least 15% w/w non-aggregated ALA relative to the total amount of protein. Alternatively, the whey protein solution of step 1) may comprise at least 20% w/w non-aggregated ALA relative to the total amount of protein.

In some preferred embodiments of the invention, the whey protein solution of step 1) comprises at least 25% w/w non-aggregated ALA relative to the total amount of protein. Preferably, the whey protein solution of step 1) comprises at least 30% w/w non-aggregated ALA relative to the total amount of protein. The whey protein solution of step 1) preferably comprises at least 35% w/w non-aggregated ALA relative to the total amount of protein. Even more preferably, the whey protein solution of step 1) may comprise at least 40% w/w non-aggregated ALA relative to the total amount of protein.

In some preferred embodiments of the invention, the whey protein solution of step 1) comprises in the range of 5-95% w/w non-aggregated ALA relative to the total amount of protein. Preferably, the whey protein solution of step 1) comprises in the range of 5-70% w/w non-aggregated ALA relative to the total amount of protein. Even more preferably, the whey protein solution of step 1) may comprise in the range of 10-60% w/w non-aggregated ALA relative to the total amount of protein. The whey protein solution of step 1) preferably comprises in the range of 12-50% w/w non-aggregated ALA relative to the total amount of protein. Even more preferably, the whey protein solution of step 1) may comprise in the range of 20-45% w/w non-aggregated ALA relative to the total amount of protein.

In some preferred embodiments of the invention, the whey protein solution of step 1) has a weight ratio between non-aggregated BLG and non-aggregated ALA of at least 0.01. Preferably, the whey protein solution of step 1) has a weight ratio between non-aggregated BLG and non-aggregated ALA of at least 0.5. Even more preferably, the whey protein solution of step 1) has a weight ratio between non-aggregated BLG and non-aggregated ALA of at least 1, such as e.g. at least 2. For example, the whey protein solution of step 1) may have a weight ratio between non-aggregated BLG and non-aggregated ALA of at least 3.

In some preferred embodiments of the invention, the whey protein solution of step 1) has a weight ratio between non-aggregated BLG and non-aggregated ALA in the range of 0.01-20. Preferably, the whey protein solution of step 1) has a weight ratio between non-aggregated BLG and non-aggregated ALA in the range of 0.2-10. Even more preferably, the whey protein solution of step 1) has a weight ratio between non-aggregated BLG and non-aggregated ALA in the range of 0.5-4. For example, the whey protein solution of step 1) may have a weight ratio between non-aggregated BLG and non-aggregated ALA in the range of 1-3.

In some preferred embodiments of the invention, the whey protein solution of step 1) comprises at least 1% w/w non-aggregated BLG relative to the total amount of protein. Preferably, the whey protein solution of step 1) comprises at least 2% w/w non-aggregated BLG relative to the total amount of protein. Even more preferably, the whey protein solution of step 1) comprises at least 5% w/w non-aggregated BLG relative to the total amount of protein. Preferably, the whey protein solution of step 1) may comprise at least 10% w/w non-aggregated BLG relative to the total amount of protein.

In some preferred embodiments of the invention, the whey protein solution of step 1) comprises at least 12% w/w non-aggregated BLG relative to the total amount of protein. For example, the whey protein solution of step 1) may comprise at least 15% w/w non-aggregated BLG relative to the total amount of protein. The whey protein solution of step 1) may e.g. comprise at least 20% w/w non-aggregated BLG relative to the total amount of protein. Alternatively, the whey protein solution of step 1) may comprise at least 30% w/w non-aggregated BLG relative to the total amount of protein.

In some particularly preferred embodiments of the invention, the whey protein solution of step 1) comprises at most 95% w/w non-aggregated BLG relative to the total amount of protein. Preferably, the whey protein solution of step 1) may comprise at most 90% w/w non-aggregated BLG relative to the total amount of protein. More preferably, the whey protein solution of step 1) may e.g. comprise at most 85% w/w non-aggregated BLG relative to the total amount of protein. Even more preferably, the whey protein solution of step 1) may e.g. comprise at most 80% w/w non-aggregated BLG relative to the total amount of protein. Preferably, the whey protein solution of step 1) may comprise at most 78% w/w non-aggregated BLG relative to the total amount of protein. Preferably, the whey protein solution of step 1) may comprise at most 75% w/w non-aggregated BLG relative to the total amount of protein.

In some preferred embodiments of the invention, the whey protein solution of step 1) comprises in the range of 1-95% w/w non-aggregated BLG relative to the total amount of protein. Preferably, the whey protein solution of step 1) may comprise in the range of 5-90% w/w non-aggregated BLG relative to the total amount of protein. More preferably, the whey protein solution of step 1) comprises in the range of 10-85% w/w non-aggregated BLG relative to the total amount of protein. Even more preferably, the whey protein solution of step 1) comprises in the range of 10-80% w/w non-aggregated BLG relative to the total amount of protein. Most preferably, the whey protein solution of step 1) may comprise in the range of 20-70% w/w non-aggregated BLG relative to the total amount of protein.

In other preferred embodiments of the invention, the whey protein solution of step 1) comprises in the range of 10-95% w/w non-aggregated BLG relative to the total amount of protein. Preferably, the whey protein solution of step 1) may comprise in the range of 12-90% w/w non-aggregated BLG relative to the total amount of protein. More preferably, the whey protein solution of step 1) comprises in the range of 15-85% w/w non-aggregated BLG relative to the total amount of protein. Even more preferably, the whey protein solution of step 1) comprises in the range of 15-80% w/w non-aggregated BLG relative to the total amount of protein. Most preferably, the whey protein solution of step 1) may comprise in the range of 30-70% w/w non-aggregated BLG relative to the total amount of protein.

In some preferred embodiments of the invention, the whey protein solution of step 1) comprises at least 0.4% w/w non-aggregated BLG relative to the weight of the whey protein solution. Preferably, the whey protein solution comprises at least 1.0% w/w non-aggregated BLG. More preferably, the whey protein solution comprises at least 2.0% w/w non-aggregated BLG. It is even more preferred that the whey protein solution comprises at least 4% w/w non-aggregated BLG.

Higher concentrations of non-aggregated BLG are even more preferred and preferably the whey protein solution comprises at least 6% w/w non-aggregated BLG. More preferably, the whey protein solution comprises at least 10% w/w non-aggregated BLG. It is even more preferred that the whey protein solution comprises at least 15% w/w non-aggregated BLG.

In some preferred embodiments of the invention, the whey protein solution of step 1) comprises in the range of 0.4-45% w/w non-aggregated BLG relative to the weight of the whey protein solution. Preferably, the whey protein solution comprises in the range of 1-35% w/w non-aggregated BLG. More preferably, the whey protein solution comprises in the range of 4-30% w/w non-aggregated BLG. It is even more preferred that the whey protein solution comprises in the range of 10-25% w/w non-aggregated BLG.

Higher contents of BLG are particularly preferred, and thus, in some preferred embodiments of the invention, the whey protein solution of step 1) comprises in the range of 10-45% w/w non-aggregated BLG relative to the weight of the whey protein solution. Preferably, the whey protein solution comprises in the range of 15-40% w/w non-aggregated BLG. More preferably, the whey protein solution comprises in the range of 20-39% w/w non-aggregated BLG. It is even more preferred that the whey protein solution comprises in the range of 25-38% w/w non-aggregated BLG.

It is often preferred that a substantial amount of the BLG of the whey protein solution is non-aggregated BLG. Preferably at least 50% of the BLG is non-aggregated BLG. More preferably at least at least 80% of the BLG is non-aggregated BLG. Even more preferred at least 90% of the BLG is non-aggregated BLG. Most preferred, at least 95% of the BLG is non-aggregated BLG. Even more preferred approx. 100% of the BLG of the whey protein solution is non-aggregated BLG.

Any suitable whey protein source may be used to prepare the whey protein solution. In some preferred embodiments of the invention, the whey protein solution comprises, or even consists of, a milk serum protein concentrate, whey protein concentrate, milk serum protein isolate, whey protein isolate, or a combination thereof.

It is preferred that the whey protein solution is a demineralised whey protein solution.

In this context, the term demineralised means that the conductivity of the whey protein solution is at most 15 mS/cm, and preferably at most 10 mS/cm, and even more preferably at most 8 mS/cm. The UF permeate conductivity of a demineralised whey protein solution is preferably at most 7 mS/cm, more preferably at most 4 mS/cm, and even more preferably at most 1 mS/cm.

It is particularly preferred that the whey protein solution is a demineralised milk serum protein concentrate, a demineralised milk serum protein isolate, a demineralised whey protein concentrate, or a demineralised whey protein isolate.

In some particularly preferred embodiments of the invention, the whey protein solution comprises, or even consists of, a demineralised and pH adjusted milk serum protein concentrate, whey protein concentrate, milk serum protein isolate, whey protein isolate, or a combination thereof.

The whey protein solution may for example comprise, or even consist of, a demineralised milk serum protein concentrate. Alternatively, the whey protein solution may comprise, or even consist of, a demineralised whey protein concentrate. Alternatively, the whey protein solution may comprise, or even consist of, a demineralised milk serum protein isolate. Alternatively, the whey protein solution may comprise, or even consist of, a demineralised whey protein isolate.

The BLG-enriched composition is preferably prepared from mammal milk, and preferably from ruminant milk, such as e.g. milk from cow, sheep, goat, buffalo, camel, llama, mare and/or deer. Protein derived from bovine milk is particularly preferred.

The protein of the whey protein solution is preferably as close to its native state as possible and preferably, it has only been subjected to gentle heat-treatments, if any at all.

In some preferred embodiments of the invention, the whey protein solution has a furosine value of at most 80 mg/100 g protein. Preferably, the whey protein solution has a furosine value of at most 40 mg/100 g protein. More preferably, the whey protein solution has a furosine value of at most 20 mg/100 g protein. Even more preferably, the whey protein solution has a furosine value of at most 10 mg/100 g protein. Most preferably, the whey protein solution has a furosine value of at most 5 mg/100 g protein, such as e.g. preferably a furosine value of 0 mg/100 g protein.

The whey protein solution typically contains other components in addition to protein. The whey protein solution may contain other components that are normally found in whey or milk serum, such as e.g. minerals, carbohydrate, and/or lipid. Alternatively or additionally, the whey protein solution may contain components that are not native to the whey or milk serum. However, such non-native components should preferably be safe for use in food production and preferably also for human consumption.

The present process is particularly advantageous for separating BLG from crude whey protein solutions that contain other solids than BLG.

The whey protein solution may for example contain carbohydrates, such as e.g. lactose, oligosaccharides and/or hydrolysis products of lactose (i.e. glucose and galactose). The whey protein solution may e.g. contain carbohydrate in the range of 0-40% w/w, such as in the range of 1-30% w/w, or in the range of 2-20% w/w.

In some preferred embodiments of the invention, the whey protein solution contains at most 20% w/w carbohydrate, preferably at most 10% w/w carbohydrate, more preferably at most 5% w/w carbohydrate, and even more preferably at most 2% w/w carbohydrate.

The whey protein solution may also comprise lipid, e.g. in the form of triglyceride and/or other lipid types such as phospholipids.

In some embodiments of the invention, the whey protein solution of step 1) comprises a total amount of lipid of at most 15% w/w relative to total solids. Preferably, the whey protein solution of step 1) comprises a total amount of lipid of at most 10% w/w relative to total solids. More preferably, the whey protein solution of step 1) comprises a total amount of lipid of at most 6% w/w relative to total solids. Even more preferably, the whey protein solution of step 1) comprises a total amount of lipid of at most 1.0% w/w relative to total solids. Most preferably, the whey protein solution of step 1) comprises a total amount of lipid of at most 0.5% w/w relative to total solids.

The total amount of protein of the whey protein solution is typically at least 1% w/w relative to the weight of the whey protein solution. Preferably, the total amount of protein of the whey protein solution is at least 5% w/w. More preferably, the total amount of protein of the whey protein solution is at least 10% w/w. Even more preferably, the total amount of protein of the whey protein solution is at least 15% w/w.

In some preferred embodiments of the invention, the total amount of protein of the whey protein solution is in the range of 1-50% w/w. Preferably, the total amount of protein of the whey protein solution is in the range of 5-40% w/w. More preferably, the total amount of protein of the whey protein solution is in range of 10-30% w/w. Even more preferably, the total amount of protein of the whey protein solution is in the range of 15-25% w/w.

The total amount of protein of the whey protein solution is determined according to Example 1.5.

The whey protein solution is typically prepared by subjecting a whey protein feed to one or more adjustments which form the whey protein solution which is supersaturated with respect to BLG.

The whey protein feed is preferably a WPC, a WPI, an SPC, an SPI, or a combination thereof.

The term "whey protein feed" pertains to the composition from which the BLG-enriched composition and subsequently the liquid BLG isolate are derived. The whey protein feed may e.g. be transformed to the whey protein solution supersaturated with respect to BLG. The whey protein feed is typically an aqueous liquid comprising BLG and at least one additional whey protein, but is normally not supersaturated with respect to BLG.

The embodiments relating to the chemical composition of the whey protein solution equally apply to the whey protein feed. However, typically at least one parameter of the whey protein feed is set to avoid supersaturation or at least spontaneous crystallisation.

In some preferred embodiments of the invention, the supersaturated whey protein solution is prepared by subjecting the whey protein feed to one or more of the following adjustments:

- Adjusting the pH,
- Reducing the conductivity
- Reducing the temperature
- Increasing the protein concentration
- Adding an agent that reduces the water activity
- Modifying the ion composition

In some preferred embodiments of the invention, the preparation of the whey protein solution involves adjusting the pH of the whey protein feed to a pH in the range of 5-6.

All pH values are measured using a pH glass electrode and are normalised to 25 degrees C. The normalisation to 25 degrees C is typically performed by the pH meter. Alternatively the temperature of the sample is adjusted to 25 degrees C.

The whey protein solution may for example have a pH in the range of 4.9-6.1. The pH of the whey protein solution may e.g. be in the range of 5.0-6.1. Alternatively, the pH of the whey protein solution may be in the range of 5.1-6.1. Preferably, the pH of the whey protein solution is in the range of 5.1-6.0.

In some preferred embodiments of the invention, the pH of the whey protein solution is in the range of 5.0-6.0. Preferably, the pH of the whey protein solution is in the range of 5.1-6.0. More preferably, the pH of the whey protein solution is in the range of 5.1-5.9. Even more preferably, the pH of the whey protein solution may be in the range of 5.2-5.9. Most preferably, the pH of the whey protein solution is in the range of 5.2-5.8.

The pH is preferably adjusted using food acceptable acids and/or bases. Food acceptable acids are particularly preferred, such as e.g. carboxylic acids. Useful examples of such acids are e.g. acetic acid, adipic acid, ascorbic acid, benzoic acid, butyric acid, citric acid, folic acid, fumaric acid, gluconic acid, hydrochloric acid, lactic acid, malic acid, phosphoric acid, propionic acid, sorbic acid, succinic acid, sulfuric acid, tartaric acid, and/or mixtures thereof.

In some preferred embodiments of the invention, the pH is adjusted using a lactone, such as e.g. D-glucono-delta-lactone, which slowly hydrolyses and at the same time reduces the pH of the aqueous liquid containing it. The target pH after the hydrolysis of the lactone has ended can be calculated precisely.

Useful examples of food acceptable bases are e.g. hydroxide sources such as e.g. sodium hydroxide, potassium hydroxide, calcium hydroxide, salts of food acids such as e.g. tri-sodium citrate, and/or combinations thereof.

In other preferred embodiments of the invention, the pH is adjusted by addition of cation exchange material on its $H^+$ form. Bead-type/large particle type cation exchange material is easily removed from the whey protein solution prior to the crystallisation or even after the crystallisation. Adjustment of pH by addition of cation exchange material on its $H^+$ form is particularly advantageous in the present invention as it reduced the pH without adding negative counter ions that significantly affects the conductivity of the whey protein feed.

In some preferred embodiments of the invention, the preparation of the whey protein solution involves reducing the conductivity of the whey protein feed.

Conductivity values mentioned herein have been normalised to 25 degrees C unless it is specified otherwise.

It has been found that reducing the conductivity of the whey protein solution leads to a higher yield of BLG crystals. The minimum obtainable conductivity of the whey protein solution depends on the composition of the protein fraction and the lipid fraction (if any). Some protein species, such as e.g. caseinomacropeptide (CMP), contribute more to the conductivity than other protein species. It is therefore preferable that the conductivity of the whey protein feed is brought near to the level where protein and the counter ions of the protein are the main contributors to the conductivity. The reduction of conductivity often involves removal of at least some of the small, free ions that are present in liquid phase and not tightly bound to the proteins.

It is often preferred that the whey protein solution has a conductivity of at most 10 mS/cm. In some preferred embodiments of the invention, the whey protein solution has a conductivity of at most 5 mS/cm. Preferably, the whey protein solution has a conductivity of at most 4 mS/cm.

Lower conductivities are even more preferably and give rise to higher yields of BLG crystals. Thus, the whey protein solution preferably has a conductivity of at most 3 mS/cm. In some preferred embodiments of the invention, the whey protein solution has a conductivity of at most 1 mS/cm. Preferably, the whey protein solution has a conductivity of at most 0.5 mS/cm.

The conductivity of the whey protein feed is preferably reduced by dialysis or diafiltration. Diafiltration by ultrafiltration is particularly preferred as it allows for washing out salts and small charged molecules while proteins are retained. In some preferred embodiments of the invention, the same UF unit is used for UF/diafiltration and subsequent concentration of the whey protein feed.

The ratio between the conductivity (expressed in mS/cm) and the total amount of protein in the whey protein solution (expressed in % wt. total protein relative to the total weight of the whey protein solution) can advantageously be kept at or below a certain threshold to facilitate the crystallisation of BLG.

In some preferred embodiments of the invention, the ratio between the conductivity and the total amount of protein of the whey protein solution is at most 0.3. Preferably, the ratio between the conductivity and the total amount of protein of the whey protein solution is at most 0.25. Preferably, the ratio between the conductivity and the total amount of protein of the whey protein solution is at most 0.20. More preferably, the ratio between the conductivity and the total amount of protein of the whey protein solution is at most 0.18. Even more preferably, the ratio between the conductivity and the total amount of protein of the whey protein solution is at most 0.12. Most preferably, the ratio between the conductivity and the total amount of protein of the whey protein solution is at most 0.10.

It is for example preferred that the ratio between the conductivity and the total amount of protein of the whey protein solution is approx. 0.07, or even lower.

The present inventors have furthermore found that the whey protein feed advantageously may be conditioned to provide a whey protein solution having a UF permeate conductivity of at most 10 mS/cm. The UF permeate conductivity is a measure of the conductivity of the small molecule fraction of a liquid. When the term "conductivity" is used herein as such, it refers to the conductivity of the liquid in question. When the term "UF permeate conductivity" is used, it refers to the conductivity of the small molecule fraction of a liquid and is measured according to Example 1.23.

Preferably, the UF permeate conductivity of the whey protein solution is at most 7 mS/cm. More preferably, the UF permeate conductivity of the whey protein solution may be at most 5 mS/cm. Even more preferably, the UF permeate conductivity of the whey protein solution may be at most 3 mS/cm.

Even lower UF permeate conductivities may be used and are particularly preferred if a high yield of BLG should be obtained. Thus, preferably, the UF permeate conductivity of the whey protein solution is at most 1.0 mS/cm. More preferably, the UF permeate conductivity of the whey protein solution may be at most 0.4 mS/cm. Even more preferably, the UF permeate conductivity of the whey protein solution may be at most 0.1 mS/cm. Most preferably, the UF permeate conductivity of the whey protein solution may be at most 0.04 mS/cm.

Even lower UF permeate conductivities may be reached, e.g. if MilliQ water is used as a diluent during diafiltration (MilliQ water has a conductivity of approx. 0.06 $\mu$S/cm) Thus, the UF permeate conductivity of the whey protein solution may be at most 0.01 mS/cm. Alternatively, the UF permeate conductivity of the whey protein solution may be at most 0.001 mS/cm. Alternatively, the UF permeate conductivity of the whey protein solution may be at most 0.0001 mS/cm.

In some preferred embodiments of the invention, the preparation of the whey protein solution involves reducing the temperature of the whey protein feed.

For example, the preparation of the whey protein solution may involve reducing the temperature of the whey protein feed to at least 5 degrees C, preferably at least 10 degrees C and even more preferably at least 15 degrees C. For example, the preparation of the whey protein solution may involve reducing the temperature of the whey protein feed to at least 20 degrees C.

The temperature of the whey protein feed may e.g. be reduced to at most 30 degrees C, preferably at most 20 degrees C, and even more preferably to at most 10 degrees C. The inventors have found that even lower temperatures provide higher degree of supersaturation, and thus, the temperature of the whey protein feed may e.g. be reduced to at most 5 degrees C, preferably at most 2 degrees C, and even more preferably to at most 0 degrees C. The temperature may even be lower than 0 degrees C. However, preferably the whey protein solution should remain pumpable, e.g. in the form of an ice slurry.

In some preferred embodiments of the invention, the whey protein solution is an ice slurry before the initialisation of BLG crystallisation. Alternatively or additionally, crystallising whey protein solution may be converted into or maintained as an ice slurry during the BLG crystallisation of step 2).

In some particularly preferred embodiments of the invention, the preparation of the whey protein solution involves increasing the total protein concentration of the whey protein feed. The whey protein feed may e.g. be subjected to one or more protein concentration steps, such as ultrafiltration, nanofiltration, reverse osmosis, and/or evaporation and thereby concentrated to obtain the whey protein solution.

Ultrafiltration is particularly preferred as it allows for selective concentration of protein while the concentrations of salts and carbohydrates are nearly unaffected. As mentioned above, ultrafiltration is preferably used both for diafiltration and concentration of the whey protein feed.

In some preferred embodiments of the invention, the concentration of BLG in the whey protein solution is below the level where spontaneous crystallisation of BLG occurs. It is therefore often preferred to stop the modifications of the whey protein feed when the whey protein solution is in the meta-stable region, i.e. in the supersaturated region where BLG crystals can grow when seeding is used but where crystallisation does not start spontaneously.

In some preferred embodiments of the invention, the preparation of the whey protein solution involves addition of one or more water activity reducing agent(s) to the whey protein feed.

Useful, but non-limiting, examples of such water activity reducing agents are polysaccharides and/or poly-ethylene glycol (PEG).

In some preferred embodiments of the invention, the preparation of the whey protein solution involves modifying the ion composition of the whey protein feed, e.g. by ion exchange, by adding new ion species, by dialysis or by diafiltration.

Typically, the whey protein solution is prepared by combining two or more of the above process steps for creating supersaturation.

In some preferred embodiments of the invention, the preparation of the whey protein solution involves subjecting the whey protein feed to at least:

- concentration, e.g. using ultrafiltration, nanofiltration or reverse osmosis, at a temperature above 10 degrees C, and
- subsequently cooling to a temperature below 10 degrees C.

In other preferred embodiments of the invention, the preparation of the whey protein solution involves subjecting the whey protein feed to at least

- concentration at a pH above 6.0, and
- subsequently reducing the pH by addition of an acid (e.g. GDL or cation exchange material in H$^+$ form)

In yet other preferred embodiments of the invention, the preparation of the whey protein solution involves subjecting the whey protein feed to at least:

- reducing the conductivity, e.g. by diafiltration using a membrane that retains at least non-aggregated BLG.

In further preferred embodiments of the invention, the preparation of the whey protein solution involves subjecting the whey protein feed to a combination at least:

- adjusting the pH to 5-6,
- reducing the conductivity by diafiltration using a membrane that retains at least non-aggregated BLG,
- concentrating protein, e.g. using ultrafiltration, nanofiltration or reverse osmosis, at a temperature above 10 degrees C, and
- finally, cooling to a temperature below 10 degrees C.

The present inventors have furthermore found that the BLG yield of the present process may be improved by controlling the molar ratio between the sum of sodium+potassium vs. the sum of calcium and magnesium. A higher relative amount of calcium and magnesium surprisingly seems to increase the yield of non-aggregated BLG and therefore increases the efficiency of the BLG recovery of the present process.

In some preferred embodiments of the present invention, the whey protein solution of step 1) has a molar ratio between Na + K and Ca + Mg of at most 4. More preferably, the whey protein solution of step 1) has a molar ratio between Na + K and Ca + Mg of at most 2. Even more preferably, the whey protein solution of step 1) has a molar ratio between Na + K and Ca + Mg of at most 1.5, and even more preferably at most 1.0. Most preferably, the whey protein solution of step 1) has a molar ratio between Na + K and Ca + Mg of at most 0.5, such as e.g. at most 0.2.

The molar ratio between Na + K and Ca + Mg it calculated as $(m_{Na}+m_K)/(m_{Ca}+m_{Mg})$ wherein $m_{Na}$ is the content of elemental Na in mol, $m_K$ is the content of elemental K in mol, $m_{Ca}$ is the content of elemental Ca in mol, and $m_{Mg}$ is the content of elemental Mg in mol.

It is particularly preferred that the whey protein solution has been supersaturated with respect to BLG by salting-in and that BLG therefore can be crystallised from the whey protein solution by salting-in.

In some embodiments of the invention, the whey protein solution has a low content of denatured protein, particularly if the edible BLG product of the present invention should have a degree of protein denaturation too. Preferably, the whey protein solution has a degree of protein denaturation of at most 2%, preferably at most 1.5%, more preferably at most 1.0%, and most preferably at most 0.8%.

Step 2) of the process involves crystallising at least some of the BLG of the supersaturated whey protein solution.

It is particularly preferred that the crystallisation of step 2) takes place by salting-in, i.e. in a liquid that has a low ionic strength and conductivity. This is contrary to the salting-out mode, wherein significant amounts of salts are added to a solution in order to provoke crystallisation.

The crystallisation of BLG of step 2) may e.g. involve one or more of the following:

- Waiting for crystallisation to take place,
- Addition of crystallisation seeds,
- Increasing the degrees of supersaturation of BLG even further, and/or
- Mechanical stimulation.

In some preferred embodiments of the invention, step 2) involves adding crystallisation seeds to the whey protein solution. The inventors have found that addition of crystallisation seeds makes it possible to control when and where the BLG crystallisation takes place to avoid sudden clogging of process equipment and unintentional stops during production. It is for example often desirable to avoid onset crystallisation while concentrating the whey protein feed.

It is particularly preferred that the whey protein solution does not contact an UF membrane or MF membrane in operation during step 2) unless ceramic membranes or high shear systems, such as DCF, are employed.

In principle, any seed material which initiates the crystallisation of BLG may be used. However, it is preferred that hydrated BLG crystals or dried BLG crystals are used for seeding to avoid adding additional impurities to the whey protein solution.

The crystallisation seeds may be on dry form or may form part of a suspension when added to the whey protein solution. Adding a suspension containing the crystallisation seeds, e.g. BLG crystals, is presently preferred as it appears to provide a faster onset of crystallisation. It is preferred that such a suspension contains crystallisation seeds having a pH in the range of 5-6 and a conductivity of at most 10 mS/cm.

We note that the conductivity unit "mS/cm" means milliSiemens per cm and 1.00 mS correspond to 1000 microS.

It is particularly preferred that the crystallisation seeds are added via a suspension of BLG crystals that have not been dried after BLG crystallisation. Such a suspension could for example be a portion of BLG crystals and mother liquor obtained from step 2) of a previous batch or portion of wet BLG crystals obtained from step 3), 4) or 5) of a previous batch.

The present inventors have observed that the use of wet BLG crystals for crystallisation seeds provides much larger BLG crystals during step 2) than if dry or poorly hydrated BLG crystals are used, which again makes the separation of BLG from the mother liquor more efficient. In an experiment in which the whey protein feed, crystallisation conditions, mass and particle size of seeding material, cooling profile, and separation method was the same the inventers found that seeding with non-dried BLG crystals provided a 100% increase in the particle size of the obtained crystals (obtained particle size: 100-130 microns) relative to BLG crystals obtained by seeding with rehydrated, dried BLG crystals (obtained particle size: 40-60 microns).

Alternatively, if the crystallisation seeds are based on dried BLG crystals, it is preferred to resuspend the crystals in an aqueous liquid, e.g. water, and allow the dried BLG crystals to rehydrate for at least 30 minutes, preferably at least 1.0 hour, and even more preferably at least 1.5 hours before the resulting BLG crystal suspension is used for initiating crystallisation.

In some embodiments of the invention, at least some of the crystallisation seeds are located on a solid phase which is brought in contact with the whey protein solution.

The crystallisation seeds preferably have a smaller particle size than the desired size of the BLG crystals. The size of the crystallisation seeds may be modified by removing the largest seeds by sieving or other size fractionation processes. Particle size reduction, e.g. by means of grinding, may also be employed prior to the particle size fractionation.

In some embodiments of the invention, at least 90% w/w of the crystallisation seeds have a particle size (measured by sieving analysis) in the range of 0.1-600 microns. For example, at least 90% w/w of the crystallisation seeds may have a particle size in the range of 1-400 microns. Preferably, at least 90% w/w of the crystallisation seeds may have a particle size in the range of 5-200 microns. More preferably, at least 90% w/w of the crystallisation seeds may have a particle size in the range of 5-100 microns.

The particle size and dosage of crystallisation seeds may be tailored to provide the optimal crystallisation of BLG.

In some preferred embodiments of the invention, the crystallisation seeds are added to the whey protein feed prior to obtaining supersaturation with respect to BLG but preferably in a way that at least some crystallisation seeds are still present when supersaturation is reached. This may e.g. be accomplished by adding crystallisation seeds when the whey protein feed is close to supersaturation, e.g. during cooling, concentration, and/or pH adjustment and to reach supersaturation before the crystallisation seeds are completely dissolved.

In some preferred embodiments of the invention, step 2) involves increasing the degree of supersaturation of BLG even further, preferably to a degree where crystallisation of BLG initiates immediately, i.e. in at most for 20 minutes, and preferably in at most for 5 minutes. This is also referred to as the nucleation zone wherein crystallites form spontaneously and start the crystallisation process.

The degree of supersaturation may e.g. be increased by one or more of the following:

- increasing the protein concentration of the whey protein solution further
- cooling the whey protein solution further
- bringing the whey protein solution closer to the optimum pH for BLG crystallisation
- reducing the conductivity even further.

In some preferred embodiments of the invention, step 2) involves waiting for the BLG crystals to form. This may take several hours and is typically for a whey protein solution which is only slightly supersaturated with respect to BLG and to which no crystallisation seeds have been added.

In some preferred embodiments of the invention, the provision of the whey protein solution (step 1) and the crystallisation of BLG (step 2) take place as two separate steps.

However, in other preferred embodiments of the invention, step 2) involves additional adjustment of the crystallising whey protein solution to raise the degree of supersaturation of BLG, or at least maintain supersaturation. The additional adjustment results in an increased yield of BLG crystals.

Such additional adjustment may involve one or more of:

- increasing the protein concentration of the crystallising whey protein solution even further
- cooling the crystallising whey protein solution to an even lower temperature
- bringing the crystallising whey protein solution even closer to the optimum pH for BLG crystallisation
- reducing the conductivity of the crystallising whey protein solution even further.

In some preferred embodiments of the invention, the crystallising whey protein solution is maintained in the meta-stable zone during step 2) to avoid spontaneous formation of new crystallites.

In some preferred embodiments of the invention, at least some of the BLG crystals obtained during step 2) have an orthorhombic space group P $2_1$ $2_1$ $2_1$.

Preferably, at least some of the obtained BLG crystals have an orthorhombic space group P $2_1$ $2_1$ $2_1$ and the unit cell dimensions a=68.68 ($\pm$5%) Å, b = 68.68 ($\pm$5%) Å, and c = 156.65 ($\pm$5%) Å; and unit cell integral angles $\alpha$=90°, $\beta$=90°, and $\gamma$=90°.

In some preferred embodiments of the invention, at least some of the obtained BLG crystals have an orthorhombic space group P $2_1$ $2_1$ $2_1$ and the unit cell dimensions a=68.68 ($\pm$2%) Å, b = 68.68 ($\pm$2%) Å, and c = 156.65 ($\pm$2%) Å; and the unit cell integral angles $\alpha$=90°, $\beta$=90°, and $\gamma$=90°.

Even more preferred, at least some of the obtained BLG crystals may have an orthorhombic space group P $2_1$ $2_1$ $2_1$ and the unit cell dimensions a=68.68 ($\pm$1%) Å, b = 68.68 ($\pm$1%) Å, and c = 156.65 ($\pm$1%) Å; and the unit cell integral angles $\alpha$=90°, $\beta$=90°, and $\gamma$=90°.

Most preferably, at least some of the obtained BLG crystals have an orthorhombic space group P $2_1$ $2_1$ $2_1$ and the unit cell dimensions a=68.68 Å, b = 68.68 Å, and c = 156.65 Å; and the unit cell integral angles $\alpha$=90°, $\beta$=90°, and $\gamma$=90°.

In some particularly preferred embodiments of the invention, the process contains a step 3) of separating at least some of the BLG crystals from the remaining whey protein solution. This is especially preferred when purification of BLG is desired.

Step 3) may for example comprise separating the BLG crystals to a solids content of at least 30% w/w. Preferably, step 3) comprises separating the BLG crystals to a solids content of at least 40% w/w. Even more preferably step 3) comprises separating the BLG crystals to a solids content of at least 50% w/w.

The inventors have found that the high solids content is advantageous for the purification of BLG, as the aqueous portion that adhere to the separated BLG crystals typically contains the impurities that should be avoided. Additionally, the high solids content reduces the energy consumption for converting the separated BLG crystals to a dry product, such as e.g. a powder, and it increases the BLG yield obtained from a drying unit with a given capacity.

In some preferred embodiments of the invention, step 3) comprises separating the BLG crystals to a solids content of at least 60%. Preferably, step 3) comprises separating the BLG crystals to a solids content of at least 70%. Even more preferably, step 3) comprises separating the BLG crystals to a solids content of at least 80%.

In some preferred embodiments of the invention, the separation of step 3) involves one or more of the following operations:

- centrifugation,
- decantation,
- filtration,
- sedimentation,
- combinations of the above.

These unit operations are well-known to the person skilled in the art and are easily implemented. Separation by filtration may e.g. involve the use of vacuum filtration, dynamic cross-flow filtration (DCF), a filtrate press or a filter centrifuge.

Different pore sizes for filtration may be employed based on the desired outcome. Preferably, the filter allows native whey protein and small aggregates to pass but retains the BLG crystals. The filter preferably has a nominal pore size of at least 0.1 micron. The filter may e.g. have a nominal pore size of at least 0.5 micron. Even more preferably, the filter may have a nominal pore size of at least 2 micron.

Filters having larger pore sizes can also be used and are in fact preferred if primarily the large crystals should be separated from a liquid containing BLG crystals. In some embodiments of the invention, the filter has a nominal pore size of at least 5 micron. Preferably, the filter has a nominal pore size of at least 20 micron. Even more preferably, the filter may have a pore size of at least 40 micron.

The filter may e.g. have a pore size in the range of 0.03-5000 micron, such as e.g. 0.1-5000 micron. Preferably, the filter may have a pore size in the range of 0.5-1000 micron. Even more preferably, the filter may have a pore size in the range of 5-800 micron, such as e.g. in the range of 10-500 micron or in the range of 50-500 microns.

In some preferred embodiments of the invention, the filter has a pore size in the range of 0.03-100 micron. Preferably, the filter may have a pore size in the range of 0.1-50 micron. More preferably, the filter may have a pore size in the range of 4-40 micron. Even more preferably, the filter may have a pore size in the range of 5-30 micron such as in the range of 10-20 micron.

An advantage of using filters having a pore size larger than 1 micron is that bacteria and other microorganisms also are at least partly removed during separation and optionally also during washing and/or recrystallisation. The present process therefore makes it possible to produce high purity BLG with both a very low bacterial load yet avoiding heat-damage of the protein.

Another advantage of using filters having a pore size larger than 1 micron is that removal of water and subsequent drying becomes easier and less energy consuming.

The remaining whey protein solution which is separated from the BLG crystals may be recycled to the whey protein feed during preparation of the whey protein solution.

In some preferred embodiments of the invention, step 3) employs a filter centrifuge. In other preferred embodiments of the invention, step 3) employs a decanter centrifuge. Initial results have shown that use of a filter centrifuge and/or a decanter centrifuge for separating BLG crystals from the mother liquor provides more robust operation of the process than e.g. vacuum filtration.

Often it is preferred to dry a formed filter cake with a drying gas to reduce the moisture content of the filter cake and preferably to make it possible to peel the filter cake off the filter. The use of a drying gas may form part of the separation step or alternatively, the final drying step, if the filter cake is converted directly to a dry edible BLG composition.

In some preferred embodiments of the invention, step 3) employs a dynamic cross-flow filtration (DCF) unit.

Initial tests have shown that using a DCF unit with a membrane pore size in the range of 0.03-5 micron, and preferably in the range of 0.3-1.0 microns, offers an efficient separation of BLG crystals, and the inventors have observed that the DCF unit can be run for a duration sufficient to separate crystals from even large batches of whey protein solution containing BLG crystals.

In some preferred embodiments of the invention, step 3) is performed using a DCF unit equipped with a membrane capable of retaining BLG crystals, the DCF permeate is recycled to form part of the whey protein solution or whey protein feed, and DCF retentate may be recovered or returned to the crystallisation tank. Preferably, the DCF permeate is treated, e.g. by ultra-/diafiltration to make it supersaturated with respect to BLG prior to mixing it with the whey protein solution or whey protein feed.

Advantageously, these embodiments do not require that the temperature of the liquid streams are raised above 15 degrees C and are therefore less prone to microbial contamination than process variants that require higher temperatures. Another industrial advantage of these embodiments is that the level of supersaturation is easily controlled and can be kept at a level where unwanted, spontaneous crystallisation does not occur. The temperature of the liquid streams during these embodiments of the process is therefore preferably at most 15 degrees C, more preferably at most 12 degrees C, and even more preferably at most 10 degrees C, and most preferably at most 5 degrees C.

These embodiments are exemplified in Example 10 and illustrated in Figure 26 of the PCT application PCT/EP2017/084553. These embodiments may be implemented as a batch process or a continuous process.

In some preferred embodiments of the invention, the process comprises a step 4) of washing BLG crystals, e.g. the separated BLG crystals of 3). The washing may consist of a single wash or of multiple washing steps.

The washing of step 4) preferably involves contacting the BLG crystals with a washing liquid without completely dissolving the BLG crystals and subsequently separating the remaining BLG crystals from the washing liquid.

The washing liquid is preferably selected to avoid complete dissolution of the BLG crystals and may e.g. comprise, or even consist essentially of, cold demineralised water, cold tap water, or cold reverse osmosis permeate.

The washing liquid may e.g. comprise, or even consist essentially of, cold demineralised water, cold tap water, or cold reverse osmosis permeate.

The washing liquid may have a pH in the range of 5-6, preferably in the range of 5.0-6.0, and even more preferably in the range of 5.1-6.0, such as e.g. in the range of 5.1-5.9.

Alternatively, the washing liquid may have a pH in the range of 6.1-8, preferably in the range of 6.4-7.6, and even more preferably in the range of 6.6-7.4, such as e.g. in the range of 6.8-7.2. This is typically the pH of the washing liquid when demineralised water, tap water, or reverse osmosis permeate. It is generally preferred that the washing liquid is low in minerals and has a low buffer capacity.

The washing liquid may have a conductivity of at most 0.1 mS/cm, preferably at most 0.02 mS/cm, and even more preferably at most 0.005 mS/cm.

Washing liquids having even lower conductivities may be used. For example, the washing liquid may have a conductivity of at most 1 microS/cm. Alternatively, the washing liquid may have a conductivity of at most 0.1 microS/cm, such as e.g. approx. 0.05 microS/cm.

A washing step is preferably performed at low temperature to limit the dissolution of crystallised BLG. The temperature of the washing liquid is preferably at most 30 degrees C, more preferably at most 20 degrees C and even more preferably at most 10 degrees C.

A washing step may e.g. be performed at at most 5 degrees C, more preferably at at most 2 degrees C, such as e.g. approx. 0 degrees C. Temperatures lower than 0 degrees C may be used in so far as the washing liquid does not freeze at that temperature, e.g. due to the presence of one or more freezing point depressant(s).

In some embodiments of the invention, the washing liquid contains BLG, e.g. in an amount of at least 1% w/w, and preferably in an amount of at least 3% w/w, such as e.g. in an amount of 4% w/w.

The washing of step 4) typically dissolves at most 80% w/w of the initial amount of BLG crystals, preferably at most 50% w/w, and even more preferably at most 20% w/w of the initial amount of BLG crystals. Preferably, the washing of step 4) dissolves at most 15% w/w of the initial amount of BLG crystals, more preferably at most 10% w/w, and even more preferably at most 5% w/w of the initial amount of BLG crystals.

The weight ratio between the total amount of washing liquid and the initial amount of separated BLG crystals is often at least 1, preferably at least 2 and more preferably at least 5. For example, the weight ratio between the amount of washing liquid and the initial amount of separated BLG crystals may be at least 10. Alternatively, the weight ratio between the amount total of washing liquid and the initial amount of separated BLG crystals may be at least 20, such as e.g. at least 50 or at least 100.

The term "total amount of washing liquid" pertains to the total amount of washing liquid used during the entire process.

In some preferred embodiments of the invention, the one or more washing sequences take place in the same filter arrangement or in a similar filter arrangement as the BLG crystal separation. A filter cake primarily containing BLG crystals is added one or more sequences of washing liquid which is removed through the filter while the remaining part of the BLG crystals stays in the filter cake.

In particularly preferred embodiments of the invention, the separation of step 3) is performed using a filter that retains BLG crystals. Subsequently, the filter cake is contacted with one or more quantities of washing liquid which moves through the filter cake and the filter. It is often preferred that each quantity of washing liquid is at most 10 times the volume of the filter cake, preferably at most 5 times the volume of the filter cake, more preferably at most 1 times the volume of the filter cake, even more preferably at most 0.5 times the volume of the filter cake, such as e.g. at most 0.2 times the volume of the filter cake. The volume of the filter cake includes both solids and fluids (liquids and gasses) of the filter cake. The filter cake is preferably washed this way at least 2 times, preferably at least 4 times and even more preferably at least 6 times.

The used washing liquid from step 4) may e.g. be recycled to the whey protein feed or the whey protein solution where washed out BLG may be isolated again.

The process may furthermore comprise a step 5) which involves a recrystallisation step comprising:

- dissolving the separated BLG crystals in a recrystallisation liquid,
- adjusting the recrystallisation liquid to obtain supersaturation with respect to BLG,
- crystallising BLG in the supersaturated, adjusted recrystallisation liquid, and
- separating BLG crystals from the remaining adjusted recrystallisation liquid.

Step 5) may comprise either a single re-crystallisation sequence or multiple re-crystallisation sequences.

In some embodiments of the invention, the BLG crystals of step or 3) or 4) are recrystallized at least 2 times. For example, the BLG crystals may be recrystallized at least 3 times, such as e.g. at least 4 times.

The washing and re-crystallisation steps may be combined in any sequence and may be performed multiple times if required.

The separated BLG crystals of step 3) may e.g. be subjected to the process sequence:

- One or more steps of washing (step 4), followed by
- One or more steps of re-crystallisation (step 5).

Alternatively, the separated BLG crystals of step 3) may be subjected to the process sequence:

- One or more steps of re-crystallisation (step 5), followed by
- One or more steps of washing (step 4).

It is also possible to combine multiple steps of washing and re-crystallisation, e.g. in the sequence:

- One or more steps of washing (step 4),
- One or more steps of re-crystallisation (step 5),
- One or more steps of washing (step 4), and
- One or more steps of re-crystallisation (step 5).

Or e.g. in the sequence:

- One or more steps of re-crystallisation (step 5),
- One or more steps of washing (step 4),
- One or more steps of re-crystallisation (step 5).
- One or more steps of washing (step 4)

The present inventors have noticed that the crystallisation process including the preparation of the whey protein solution is prone to microbial growth and have found it advantageous to modify the process to address this problem.

It is particularly preferred that the total amount of time that a BLG molecule is at a temperature above 12 degrees C from the provision of whey protein feed to the BLG molecules has been separated in step c) is at most 24 hours, preferably 20 hours, more preferred at most 12, even more preferably at most 6 hours, and most preferably at most at most 3 hours.

It is possible and often preferably to reduce the duration even further, and thus, in some preferred embodiments of the invention, the total amount of time that a BLG molecule is at a temperature above 12 degrees C from the provision of whey protein feed to the BLG molecules has been separated in step c) is at most 2 hours, preferably 1 hour, more preferably at most 0.5, even more preferably at most 0.3 hours, and most preferably at most at most 0.1 hours.

In some embodiments of the invention, the process furthermore involves subjecting the separated BLG to additional BLG enrichments steps, e.g. based on chromatography or selective filtration. However, in other preferred embodiments of the invention the process does not contain additional BLG enrichment steps after step 2). By the term "additional BLG enrichment step" is meant a process step which enriches BLG relative to the total amount of protein, which step is not related to crystallisation of BLG or handling of BLG crystals. An example of such an additional BLG enrichment step is ion exchange chromatography. Washing of BLG crystals and/or recrystallisation of BLG is not considered "additional BLG enrichment steps".

In some preferred embodiments of the invention, the process involves a drying step wherein a BLG-enriched composition derived from steps 3), 4), or 5) is converted to a dry composition.

In particularly preferred embodiments of the invention, the process of preparing the BLG-enriched composition comprises the steps of:

1) providing a whey protein solution comprising BLG and at least one additional whey protein, said whey protein solution being supersaturated with respect to BLG and having a pH in the range of 5-6, said whey protein solution comprising:

- 70-100% w/w protein relative to total solids,
- 30-90% w/w non-aggregated BLG relative to total protein, and preferably 30-70%
- 4-50% w/w non-aggregated ALA relative to total protein, and preferably 8-35%
- 0-25% w/w CMP relative to total protein.
- at least 10% w/w protein relative to the total weight of the whey protein solution,

2) crystallising BLG in the supersaturated whey protein solution, preferably by addition of crystallisation seeds,

3) separating BLG crystals from the remaining whey protein solution,

4) optionally, washing the separated BLG crystals obtained from step 3),

5) optionally, re-crystallising BLG crystals obtained from step 3) or 4).

The whey protein solution is preferably a demineralised whey protein solution and preferably has a ratio between the conductivity and the total amount of protein of at most 0.3 and/or a UF permeate conductivity of at most 7 mS/cm.

These embodiments are particularly useful for making low mineral and low phosphorus BLG isolates.

In some preferred embodiments, the process is implemented as batch process. Alternatively, and sometimes preferably, the process may be implemented as semi-batch process. In other preferred embodiments, the process is implemented as a continuous process.

An advantage of the present process is that it is much faster than comparable processes for BLG crystallisation of the prior art. The duration from the initial adjustment of the whey protein feed to the completion of the separation of step 3) may be at most 10 hours, preferably at most 4 hours, more preferably at most 2 hours, and even more preferably at most 1 hour.

Generally, it is preferred to prepare the BLG-enriched composition using mild temperatures that do not damage the nutritional value of neither non-aggregated BLG or the other whey proteins of the whey protein feed.

In some preferred embodiments of the invention, the non-aggregated BLG is not subjected to a temperature above 90 degrees C during the process. Preferably, the BLG is not subjected to a temperature above 80 degrees C during the process. Even more preferably, the non-aggregated BLG is not subjected to a temperature above 75 degrees C during the process. It should be noted that even though spray-drying often employs temperatures in the excess of 150 degree C, the short exposure time and the concurrent evaporation of water means that the spray-dried proteins do not experience temperatures above 50-70 degrees C.

No matter which process has been used to prepare the BLG-enriched composition, it may contain a step of drying the BLG-enriched composition. However it is presently preferred to use the BLG-enriched composition without drying it in order to avoid the risk of damaging the protein during drying.

If it does not already have the required characteristics to be used as liquid BLG isolate, the BLG-enriched composition which has been isolated from whey protein feed may be subjected to one or more steps selected from the group of:

- demineralisation,
- addition of minerals
- dilution,
- concentration,
- physical microbial reduction, and
- pH adjustment

as part of providing the liquid BLG isolate.

Non-limiting examples of demineralisation include e.g. dialysis, gel filtration, UF/diafiltration, NF/diafiltration, and ion exchange chromatography.

Non-limiting examples of addition of minerals include addition of soluble, food acceptable salts, such as e.g. salts of Na, K, Ca, and/or Mg. Such salts may e.g. be phosphate-salts, chloride salts or salts of food acids, such as e.g. citrate salt or lactate salt. The minerals may be added in solid, suspended, or dissolved form.

Non-limiting examples of dilution include e.g. addition of liquid diluent such as water, demineralised water, or aqueous solutions of minerals, acids or bases.

Non-limiting examples of concentration include e.g. evaporation, reverse osmosis, nanofiltration, ultrafiltration and combinations thereof.

If the concentration has to increase the concentration of protein relative to total solids, it is preferred to use concentration steps such as ultrafiltration or alternatively dialysis. If the concentration does not have to increase the concentration of protein relative to total solids, methods such as e.g. evaporation, nanofiltration and/or reverse osmosis can be useful.

Non-limiting examples of physical microbial reduction include e.g. heat-treatment, germ filtration, UV radiation, high pressure treatment, pulsed electric field treatment, and ultrasound. These methods are well-known to the person skilled in the art.

Germ filtration typically involves microfiltration or large pore ultrafiltration and requires a pore size that is capable of retaining microorganisms but allow the proteins and other components of interest to pass. Useful pore sizes are typically at most 1.5 micron, preferably at most 1.0 micron, more preferably at most 0.8 micron, even more preferably at most 0.5 micron, and most preferably at most 0.2 micron. The pore size for germ filtration is normally at least 0.1 micron.

The germ filtration may for example involve a membrane having a pore size of 0.02-1 micron, preferably 0.03-0.8 micron, more preferably 0.04-0.6 micron, even more preferably 0.05-0.4 micron, and most preferably 0.1-0.2 micron.

In some preferred embodiments of the invention the liquid BLG isolate is subjected to a germ filtration and subsequently to the heat-treatment using a temperature of at most 80 degrees C, and preferably at most 75 degrees C. The combination of temperature and duration of this heat-treatment of preferably chosen to provide a sterile beverage preparation.

In other preferred embodiments of the invention the liquid BLG isolate is subjected to a germ filtration and subsequently to the heat-treatment using a temperature of at least 150 degrees C for a duration of at most 0.2 seconds, and preferably at most 0.1 seconds. The combination of temperature and duration of this heat-treatment of preferably chosen to provide a sterile beverage preparation.

Non-limiting examples of pH adjustment include e.g. addition of bases and/or acids, and preferably food acceptable bases and/or acids. It is particularly preferred to employ acids and/or bases that are capable of chelating divalent metal cations. Examples of such acids and/or bases are citric acid, citrate salt, EDTA, lactic acid, lactate salt, phosphoric acid, phosphate salt, and combinations thereof.

In the following a number of preferred embodiments of the provision of the liquid BLG isolate of step a) from BLG-enriched composition. The process steps mentioned in this context are applied to the BLG-containing product stream following the BLG-enriched composition.

In some preferred embodiments of the invention, which e.g. are useful if the BLG-enriched composition comprises BLG crystals from the salting-in process described above, the provision of the liquid BLG isolate of step a) comprises subjecting the BLG-enriched composition to the following steps in the following sequence:

- pH-adjustment to i) 2-4.9 or ii) 6.1-8.5, e.g. to dissolve the BLG crystals of the BLG-enriched composition
- optionally, demineralization or addition of mineral, and
- either:

- concentration to the desired protein content followed by physical microbial reduction, or
- physical microbial reduction followed by concentration to the desired protein content.

In yet other preferred embodiments of the invention, which e.g. are useful if the BLG-enriched composition comprises BLG crystals from the salting-in process described above, the provision of the liquid BLG isolate of step a) comprises subjecting the BLG-enriched composition to the following steps in the following sequence:

- pH-adjustment to i) 2-4.9 or ii) 6.1-8.5, e.g. to dissolve the BLG crystals of the BLG-enriched composition
- optionally, demineralization or addition of mineral,
- concentration to the desired protein content.

In other preferred embodiments of the invention, which e.g. are useful if the BLG-enriched composition comprises BLG crystals from the salting-in process described above, the provision of the liquid BLG isolate of step a) comprises subjecting the BLG-enriched composition to the following steps in the following sequence:

- addition of minerals and preferably soluble salts to dissolve the BLG crystals of the BLG-enriched composition, preferably while the pH is in the range of 5.0-6.0, and
- either:

    - concentration to the desired protein content followed by physical microbial reduction, or
    - physical microbial reduction followed by concentration to the desired protein content.

It is particularly advantageous to use heat-treatment as physical microbial reduction, either alone or in combination with one or more of the other processes for physical microbial reduction mentioned herein, when handling the acidic, dissolved BLG-enriched composition. The present inventors have found that the use of mild heat-treatment under acidic conditions is especially beneficial as it allows BLG to stay in its native, folded state but still contributes to the reduction of the microbial load of the processed streams.

It is particularly preferred to subject the acidic, dissolved BLG-enriched composition to a germ filtration step while it has a concentration of total protein of at most 27% w/w, preferably at most 22% w/w and even more preferably at most 17% w/w, and subsequently subjecting the germ filtered BLG-enriched composition or the liquid BLG isolate to a heat-treatment step.

It is even more preferred to subject the acidic, dissolved BLG-enriched composition to a germ filtration step while it has a concentration of total protein of 5-27% w/w, preferably 10-22% w/w and even more preferably at most 12-17% w/w, and subsequently subjecting the germ filtered BLG-enriched composition or the liquid BLG isolate to a heat-treatment step.

The heat-treatment step is preferably performed on the liquid BLG isolate and preferably as the final step prior to spray-drying.

It is often preferred to avoid or at least limit the unfolding of the BLG during the provision of the liquid BLG isolate. If heat-treatment is used in the pH-range 2-4.9, it is preferred that the temperature is kept at at most 82 degrees C, preferably at at most 80 degrees C, and more preferably at at most 78 degrees C to limit or even avoid unfolding of BLG.

The heat-treatment is preferably at least pasteurization.

In some preferred embodiments of the invention, the temperature of the heat-treatment is in the range 70-80 degrees C, preferably in the range 70-79 degrees C, more preferably in the range 71-78 degrees C, even more preferably in the range 72-77 degrees C, and most preferably in the range 73-76 degrees C, such as approx. 75 degrees C.

Preferably, the duration of the heat-treatment, when performed in the temperature range 70-80, is 1 second to 30 minutes. The highest exposure times are best suited for the lowest temperatures of the temperature range and *vice versa*.

In particularly preferred embodiments of the invention, the heat-treatment provides 70-78 degrees C for 1 second to 30 minutes, more preferably 71-77 degrees C for 1 minute to 25 minutes, and even more preferably 72-76 degrees C for 2 minute to 20 minutes.

Higher temperatures may also be preferred in some embodiments, especially if unfolding, and optionally also aggregation of BLG is required prior to drying. For example, the temperature of the heat-treatment may be at least 81 degrees C, preferably at least 91 degrees C, more preferably at least 100 degrees C, even more preferably at least 120 degrees C, and most preferably at least 140 degrees C.

The heat-treatment may for example be a UHT-type treatment, which typically involves a temperature in the range of 135-144 degrees C and a duration in the range of 2-10 seconds.

Alternatively, but also preferred, the heat-treatment may involve a temperature in the range of 145-180 degrees C and a duration in the range of 0.01-1 seconds, and more preferably a temperature in the range of 150-180 degrees C and a duration in the range of 0.01-0.2 seconds.

In some preferred embodiments of the invention, the provision of the liquid BLG isolate of step a) comprises subjecting microbial reduction by heat-treatment while the pH is in the range of 2-4.9, preferably 2.5-4.7, more preferably 2.8-4.3, and even more preferably 3.2-4.0.

In other preferred embodiments of the invention, the provision of the liquid BLG isolate of step a) from the BLG-enriched composition comprises physical microbial reduction by heat-treatment while the pH is in the range of 2-4.9, preferably 2.5-4.7, more preferably 2.8-4.3, and even more preferably 3.2-4.0.

In yet other preferred embodiments of the invention, the provision of the liquid BLG isolate of step a) from the BLG-enriched composition comprises demineralization while the pH is in the range of 6.1-8.5, preferably in the range of 6.3-8.0, and more preferably in the range of 6.5-7.5. The present inventors have found that such demineralization is advantageous to improve the heat-stability of BLG isolate powder prepared by the method.

In particularly preferred embodiments of the invention, the provision of the liquid BLG isolate of step a) from the BLG-enriched composition comprises:

- physical microbial reduction by heat-treatment while the pH is in the range of 2-4.9, preferably 2.5-4.7, more preferably 2.8-4.3, and even more preferably 3.2-4.0, and
- demineralization while the pH is in the range of 6.1-8.5, preferably in the range of 6.3-8.0, and more preferably in the range of 6.5-7.5.

In some preferred embodiments of the invention, which e.g. are useful if the BLG-enriched composition comprises BLG crystals from the salting-in process described above, the provision of the liquid BLG isolate of step a) comprises subjecting the BLG-enriched composition to the following steps in the following sequence:

- pH-adjustment to 2-4.9 to dissolve the BLG crystals of the BLG-enriched composition
- optionally, concentration to the desired protein content while the pH is in the range of 2-4.9, preferably 2.5-4.0, and more preferably 3.0-3.9, and
- physical microbial reduction by heat-treatment while the pH is in the range of 2-4.9, preferably 2.5-4.7, more preferably 2.8-4.3, and even more preferably 3.2-4.0.

If the BLG-enriched composition already has a pH in the range of 2-4.9, the provision of the liquid BLG isolate of step a) preferably comprises subjecting the BLG-enriched composition to following steps in the following sequence:

- demineralization while the pH is in the range of 2-4.9, preferably 2.5-4.7, more preferably 2.8-4.3, and even more preferably 3.2-4.0,
- optionally, concentration to the desired protein content while the pH is in the range of 2-4.9, preferably 2.5-4.7, more preferably 2.8-4.3, and even more preferably 3.2-4.0, and
- physical microbial reduction by heat-treatment while the pH is in the range of 2-4.9, preferably 2.5-4.7, more preferably 2.8-4.3, and even more preferably 3.2-4.0.

In other preferred embodiments of the invention, which are particularly suitable for providing a liquid BLG isolate having a pH in the range of 5.0-8.5, preferably in the range of 6.1-8.5, and more preferably in the range of 6.5-8.0, the provision of the liquid BLG isolate of step a) comprises subjecting the BLG-enriched composition to the following steps in the following sequence:

- pH-adjustment to 2-4.9, e.g. to dissolve the BLG crystals of the BLG-enriched composition,
- optionally, concentration to the desired protein content,
- physical microbial reduction by heat-treatment while the pH is in the range of 2-4.9, preferably 2.5-4.7, more preferably 2.8-4.3, and even more preferably 3.2-4.0,
- pH-adjustment to a pH in the range of 5.0-8.5, preferably in the range of 6.1-8.5, and more preferably in the range of 6.5-8.0.

In other preferred embodiments of the invention, which are particularly suitable for providing a liquid BLG isolate having a pH in the range of 6.1-8.5, preferably in the range of 6.3-8.0, and more preferably in the range of 6.5-7.5, the provision of the liquid BLG isolate of step a) comprises subjecting the BLG-enriched composition to the following steps in the following sequence:

- pH-adjustment to 2-4.9, e.g. to dissolve the BLG crystals of the BLG-enriched composition,
- optionally, concentration to the desired protein content while the pH is in the range of 2-4.9, preferably 2.5-4.7, more preferably 2.8-4.3, and even more preferably 3.2-4.0,,

- physical microbial reduction by heat-treatment while the pH is in the range of 2-4.9, preferably 2.5-4.7, more preferably 2.8-4.3, and even more preferably 3.2-4.0,
- pH-adjustment to a pH in the range of 6.1-8.5, preferably in the range of 6.3-8.0, and more preferably in the range of 6.5-7.5, and
- demineralization at a pH of 6.1-8.5, preferably in the range of 6.3-8.0, and more preferably in the range of 6.5-7.5.

In further preferred embodiments of the invention, which are particularly suitable for providing a liquid BLG isolate having a pH in the range of 6.1-8.5, preferably in the range of 6.1-8.5, and more preferably in the range of 6.5-8.0, the provision of the liquid BLG isolate of step a) comprises subjecting the BLG-enriched composition to the following steps in the following sequence:

- pH-adjustment to a pH of at least 6.1 to dissolve the BLG crystals of the BLG-enriched composition,
- demineralization while the pH is in the range of 6.1-8.5, preferably 6.5-8.0, and more preferably 6.5-7.5,
- optionally, concentration to the desired protein content while the pH is 6.1-8.5, preferably 6.5-8.0, and more preferably 6.5-7.5,
- optionally, physical microbial reduction by heat-treatment while the pH is in the range of 6.1-8.5, preferably 6.5-8.0, and more preferably 6.5-7.5.

The temperature during the conversion of the BLG-enriched composition to the liquid BLG isolate is typically in the range 0-82 degrees C and may involve even higher temperatures for heat-treatment. If the BLG streams during processing have a pH above 4.9, it is preferred that the temperature is in the range of 0-65 degrees C, and more preferably in the range of 0-15 degrees C or in the range of 50-65 degrees C to reduce microbial growth.

If the liquid BLG isolate has a pH of at most 4.9 and even more preferably at most 4.1, it is preferred that the temperature is in the range of 0-82 degrees C, and advantageously in the range of 0-15 degrees C or in the range of 50-80 degrees C to reduce microbial growth. The present inventors have found that it is particularly advantageous to perform the process, or at least the concentration step, if required, at a temperature in the range of 50-80 degrees C, and more preferably in the range in the range of 60-80 degrees C, and even more preferably in the range in the range of 65-78 degrees C as the high temperature increases the efficiency of the concentration step and at the same time contributes to the microbial reduction. This embodiment makes it possible to produce liquid BLG isolates having a high content BLG and very low microbial content while maintaining the nativeness of BLG. The resulting spray-dried powder has a high bulk density and a high level of nativeness and a very favourable microbial profile.

In some preferred embodiments of the invention the process of converting the BLG-enriched composition to the liquid BLG isolate is performed at a temperature in the range of 0-15 degrees C, and preferably in the range 1-10 degrees C.

In other preferred embodiments of the invention, at least some of the process of converting the BLG-enriched composition to the liquid BLG isolate, and preferably the entire process, is performed at a temperature in the range of 50-82 degrees C, and preferably in the range 55-80 degrees C, and more preferably in the range 60-78 degrees C. This is particularly preferred when the pH of the protein stream during processing is at most 4.9, and preferably at most 4.3, more preferably at most 3.7, and preferably in the range 3.0-4.3.

In some preferred embodiments of the invention, at least some of the process of converting the BLG-enriched composition to the liquid BLG isolate, and preferably the entire process, is performed at a temperature in the range of 78-82 degrees C while the pH is in the range 3.0-3.7 In other preferred embodiments of the invention, at least some of the process of converting the BLG-enriched composition to the liquid BLG isolate, and preferably the entire process, is performed at a temperature in the range of 60-78 degrees C while the pH is in the range 3.7-4.3

The BLG-enriched composition preferably has a protein composition which is substantially the same as the liquid BLG isolate and normally, it is not required to apply additional protein fraction while converting the BLG-enriched composition to the liquid BLG isolate.

In some preferred embodiments of the invention, the BLG-enriched composition comprises BLG in an amount of at least 85% w/w relative to total protein, preferably at least 88% w/w, more preferably at least 90% w/w, even more preferably at least 92%, and most preferably BLG in an amount of at least 95% w/w relative to total protein. It is sometimes particularly preferred that the BLG-enriched composition comprises BLG in an amount of at least 97% w/w relative to total protein, and more preferably at least 99% w/w, such as preferably approx. 100% w/w relative to total protein.

In some preferred embodiments of the invention, the BLG-enriched composition comprises total protein in an amount of at least 5% w/w, preferably at least 10% w/w, more preferably at least 15% w/w, even more preferably at least 20%, and most preferably total protein in an amount of at least 30% w/w relative to total protein.

In some preferred embodiments of the invention, the BLG-enriched composition comprises total protein in an amount in the range of 5-45% w/w, preferably in the range of 10-40% w/w, more preferably in the range of 15-38% w/w, even more preferably in the range of 20-35% w/w.

In some preferred embodiments of the invention, the sum of alpha-lactalbumin (ALA) and caseinomacropeptide (CMP) comprises at least 40% w/w of the non-BLG protein of the BLG-enriched composition, preferably at least 60% w/w, even more preferably at least 70% w/w, and most preferably at least 90% w/w of the non-BLG protein of the BLG-enriched composition.

In other preferred embodiments of the invention each main, non-BLG whey protein of the BLG-enriched composition is present in a weight percentage relative to total protein which is at most 25% of its weight percentage relative to total protein in a standard whey protein concentrate from sweet whey, preferably at most 20%, more preferably at most 15%, even more preferably at most 10%, most preferably at most 6%.

Even lower concentrations of the main non-BLG whey proteins may be desirable. Thus in additional preferred embodiments of the invention, each main non-BLG whey protein of the BLG-enriched composition is present in a weight percentage relative to total protein which is at most 4% of its weight percentage relative to total protein in a standard whey protein concentrate from sweet whey, preferably at most 3%, more preferably at most 2%, even more preferably at most 1%.

The inventors have seen indications that a low level of lactoferrin and/or lactoperoxidase is particularly advantageous for obtaining a colour-neutral whey protein product.

Thus in some preferred embodiments of the invention, lactoferrin is present in the BLG-enriched composition in a weight percentage relative to total protein which is at most 25% of its weight percentage relative to total protein in a standard whey protein concentrate from sweet whey, preferably at most 20%, more preferably at most 15%, even more preferably at most 10%, most preferably at most 6%. Even lower concentrations of lactoferrin may be desirable. Thus, in additional preferred embodiments of the invention lactoferrin is present in a weight percentage relative to total protein which is at most 4% of its weight percentage relative to total protein in a standard whey protein concentrate from sweet whey, preferably at most 3%, more preferably at most 2%, even more preferably at most 1%.

Similarly, in some preferred embodiments of the invention, lactoperoxidase is present in the BLG-enriched composition in a weight percentage relative to total protein which is at most 25% of its weight percentage relative to total protein in a standard whey protein concentrate from sweet whey, preferably at most 20%, more preferably at most 15%, even more preferably at most 10%, most preferably at most 6%. Even lower concentrations of lactoperoxidase may be desirable. Thus, in additional preferred embodiments of the invention lactoperoxidase is present in a weight percentage relative to total protein which is at most 4% of its weight percentage relative to total protein in a standard whey protein concentrate from sweet whey, preferably at most 3%, more preferably at most 2%, even more preferably at most 1%.

In some preferred embodiments of the invention, the BLG-enriched composition comprises a total solids content in an amount in the range of 5-50% w/w, preferably in the range of 10-45% w/w, more preferably in the range of 15-40% w/w, even more preferably in the range of 20-35% w/w.

In some preferred embodiments of the invention, the BLG-enriched composition comprises a water content in an amount in the range of 50-95% w/w, preferably in the range of 55-90% w/w, more preferably in the range of 60-85% w/w, even more preferably in the range of 65-80% w/w.

In some preferred embodiments of the invention, the BLG-enriched composition comprises carbohydrate in an amount of at most 60% w/w, preferably at most 50% w/w, more preferably at most 20% w/w, even more preferably at most 10% w/w, even more preferably at most 1% w/w, and most preferably at most 0.1%.

In some preferred embodiments of the invention, the BLG-enriched composition comprises lipid in an amount of at most 10% w/w, preferably at most 5% w/w, more preferably at most 2% w/w, and even more preferably at most 0.1% w/w.

In some preferred embodiments of the invention, the BLG-enriched composition is used directly as the liquid BLG isolate, e.g. if it already has the desired pH and chemical composition.

While some embodiments of the invention do not require the physical microbial reduction of step b) and therefore only require steps a) and c), other preferred embodiments do and therefore contain all three steps a), b) and c).

Thus, in some preferred embodiments of the invention, the liquid BLG isolate is subjected to physical microbial reduction.

Useful examples of physical microbial reduction involves one or more of heating, germ filtration, UV radiation, high pressure treatment, pulsed electric field treatment, and ultrasound.

In some preferred embodiments of the invention, the physical microbial reduction involves or even consists of heat-treatment.

Preferably, the heat-treatment involves at least pasteurisation.

In particularly embodiments, heat-treatment involves heating the liquid BLG isolate to a temperature in the range of 70-82 degrees C.

In some preferred embodiments of the invention, the temperature of the heat-treatment is in the range 70-80 degrees C, preferably in the range 70-79 degrees C, more preferably in the range 71-78 degrees C, even more preferably in the range 72-77 degrees C, and most preferably in the range 73-76 degrees C, such as approx. 75 degrees C.

Preferably, the duration of the heat-treatment, when performed in the temperature range 70-80, is 1 second to 30 minutes. The highest exposure times are best suited for the lowest temperatures of the temperature range and *vice versa*.

In particularly preferred embodiments of the invention, the heat-treatment provides 70-78 degrees C for 1 second to 30 minutes, more preferably 71-77 degrees C for 1 minute to 25 minutes, and even more preferably 72-76 degrees C for 2 minute to 20 minutes.

Higher temperatures may also be preferred in some embodiments, especially if unfolding and optionally also aggregation for BLG is required prior to drying. For example, the temperature of the heat-treatment may be at least 81 degrees C, preferably at least 91 degrees C, more preferably at least 100 degrees C, even more preferably at least 120 degrees C, and most preferred at least 140 degrees C.

The heat-treatment may for example involve a temperature in the range of 90-130 degrees C and a duration in the range of 5 seconds - 30 minutes. The heat-treatment may e.g. involve heating to a temperature in the range of 90-95 degrees C for a duration of 1-30 minutes, e.g. approx. 120 degrees C for 20 approx. seconds. Alternatively, the heat-treatment may involve heating to a temperature in the range of 115-125 degrees C for a duration of 5-30 seconds, e.g. approx. 120 degrees C for 20 approx. seconds.

Alternatively, the heat-treatment may for example be a UHT-type treatment, which typically involves a temperature in the range of 135-144 degrees C and a duration in the range of 2-10 seconds.

Alternatively, but also preferred, the heat-treatment may involve a temperature in the range of 145-180 degrees C and a duration in the range of 0.01-2 seconds, and more preferably a temperature in the range of 150-180 degrees C and a duration in the range of 0.01-0.3 seconds.

The implementation of the heat-treatment may involve the use of conventional equipment such as a plate or tubular heat exchanger, scraped surface heat exchanger or a retort system. Alternatively, and particularly preferred for heat-treatments above 95 degrees C, direct steam-based heating may be employed, e.g. using direct steam injection, direct steam infusion, or spray-cooking. Additionally, such direct steam-based heating is preferably used in combination with flash cooling. Suitable examples of implementation of spray-cooking are found in WO2009113858A1, which are incorporated herein for all purposes. Suitable examples of implementation of direct steam injection and direct steam infusion are found in WO2009113858A1 and WO 2010/085957 A3, which are incorporated herein for all purposes. General aspects of high temperature treatment are e.g. found in "Thermal technologies in food processing" ISBN 185573558 X, which is incorporated herein by reference for all purposes.

In some preferred embodiments of the invention the physical microbial reduction of step b) is a sterilization resulting in a sterile liquid BLG isolate. Such a sterilisation may e.g. be obtained by combining germ filtration and pasteurisation.

In some preferred embodiments of the invention the liquid BLG isolate, preferably having a pH in the range of 2-4.9, is subjected to a germ filtration and subsequently to the heat-treatment using a temperature of at most 80 degrees C, and preferably at most 75 degrees C. The combination of temperature and duration of this heat-treatment of preferably chosen to provide a liquid BLG isolate.

In other preferred embodiments of the invention the liquid BLG isolate is subjected to a germ filtration and subsequently to the heat-treatment using a temperature of at least 150 degrees C for a duration of at most 0.2 seconds, and preferably at most 0.1 seconds. The combination of temperature and duration of this heat-treatment of preferably chosen to provide a sterile liquid BLG isolate.

Step c) of the method preferably involves spray-drying or freeze drying. Spray-drying is particularly preferred.

The inventors have found that it is particularly advantageous to avoid exposing the liquid BLG isolate to a heat-treatment regime that unfolds or denatures a significant amount of the BLG. Thus, if pre-heating of the liquid BLG isolate is used prior to spraying, it is preferred to carefully control the heat-load.

In some embodiments of the invention, the liquid BLG isolate has a temperature of at most 70 degrees C when reaching the exit of the spray device (e.g. a nozzle or an atomizer), preferably at most 60 degrees C, more preferably at most 50 degrees C. In some preferred embodiments of the invention, the liquid BLG isolate has a temperature of at most 40 degrees C when reaching the exit of the spray-device, preferably at most 30 degrees C, more preferably at most 20 degrees C, even more preferably at most 10 degrees C, and most preferably at most 5 degrees C.

The spray-device of the spray-dryer is the device, e.g. the nozzle or the atomizer, which converts the solution or suspension to be dried into droplets that enter the drying chamber of the spray drier.

In some embodiments of the invention, it is particularly preferred that the liquid BLG isolate has a temperature in the range of 0-60 degrees C when reaching the exit of the spray-device, preferably in the range of 2-40 degrees C, more preferably in the range of 4-35 degrees C, and most preferably in the range of 5-10 degrees C when reaching the exit of the spray-device.

The inlet temperature of gas of the spray drier is preferably in the range of 140-220 degrees C, more preferably in the range of 160-200 degrees C, and even more preferably in the range of 170-190 degrees C, such as e.g. preferably approximately 180 degrees C. The exit temperature of the gas from the spray drier is preferably in the range of 50-95 degrees C, more preferably in the range of 70-90 degrees C, and even more preferably in the range of 80-88 degrees C, such as e.g. preferably approximately 85 degrees C. As a rule of thumb, the solids that are subjected to spray-drying are said to be heated to a temperature which is 10-15 degrees C less than the gas exit temperature.

In some preferred embodiments of the invention, the exit temperature of the spray drier is preferably in the range of 50-85 degrees C, more preferably in the range of 60-80 degrees C, and even more preferably in the range of 65-75 degrees C, such as e.g. preferably approximately 70 degrees C.

An advantage of the present method is that the liquid BLG isolate to be dried may have a very high solids content prior to the drying step and therefore less water has to be removed and less energy is consumed in the drying operation.

The inventors have found that the lower the degree of unfolding of the BLG, the higher a concentration of BLG can be handled prior to spray-drying.

In some preferred embodiments of the invention, the liquid BLG isolate has a solids content of at least 10% w/w. Preferably, the liquid BLG isolate has a solids content of at least 20% w/w. More preferably, the liquid BLG isolate has a solids content of at least 25% w/w. Even more preferably, the liquid BLG isolate has a solids content of at least 30% w/w. Most preferably, the liquid BLG isolate has a solids content of at least 35% w/w.

In other preferred embodiments of the invention, the liquid BLG isolate has a solids content of in the range of 10-60% w/w. Preferably, the liquid BLG isolate has a solids content in the range of 15-50% w/w. More preferably, the liquid BLG isolate has a solids content in the range of 20-45% w/w. Even more preferably, the liquid BLG isolate has a solids content in the range of 25-40% w/w, such as e.g. approx. 35% w/w.

In some preferred embodiments of the invention, the liquid BLG isolate contains a total amount of protein of at least 10% w/w. Preferably, the liquid BLG isolate contains a total amount of protein of at least 20% w/w. More preferably, the liquid BLG isolate contains a total amount of protein of at least 25% w/w. Even more preferably, the liquid BLG isolate contains a total amount of protein of at least 30% w/w. Most preferably, the liquid BLG isolate contains a total amount of protein of at least 35% w/w.

In other preferred embodiments of the invention, the liquid BLG isolate contains a total amount of protein of in the range of 10-50% w/w. Preferably, the liquid BLG isolate contains a total amount of protein in the range of 15-45% w/w. More preferably, the liquid BLG isolate contains a total amount of protein in the range of 20-40% w/w. Even more preferably, the liquid BLG isolate contains a total amount of protein in the range of 25-38% w/w, such as e.g. approx. 35% w/w.

The inventors have found that the protein content reduces the energy consumption for converting the liquid BLG isolate to a powder, and it increases the BLG yield obtained from a drying unit with a given capacity.

The method of the present invention is preferably operated using mild temperatures that do not damage the nutritional value of neither non-aggregated BLG or the other whey proteins of the whey protein solution.

In some preferred embodiments of the invention, the non-aggregated BLG is not subjected to a temperature above 90 degrees C during the method. Preferably, the non-aggregated BLG is not subjected to a temperature above 80 degrees C during the method. Even more preferably, the non-aggregated BLG is not subjected to a temperature above 75 degrees C during the method. It should be noted that even though spray-drying often employs temperatures in the excess of 150 degree C, the short exposure time and the concurrent evaporation of water means that the spray-dried proteins do not experience temperatures above 40-70 degrees C.

The inventors have seen indications that extended heating during the drying step reduces the amount of BLG that is in undenatured form. In some preferred embodiments of the invention, the heat exposure during the drying step is kept sufficiently low in order to provide a degree of denaturation of BLG of at most 5%, preferably at most 4%, more preferably at most 2%, even more preferably at most 0.5% and even more preferably at most 0.1%. Most preferably, the drying step does not result in detectable denaturation of BLG at all.

The drying step may furthermore involve fluid bed drying, e.g. integrated in the spray-drying device or as a separate unit operation performed after the spray-drying.

The combination of spray-drying and fluid bed drying makes it possible to reduce the amount of water that is removed while the droplet of liquid to be dried moves the spray-drying chamber and instead removes residual water from the moist powder by fluid bed drying. This solution requires less energy for drying than drying by spray-drying alone and furthermore makes it possible to modify the powder by e.g. instantization and/or agglomeration. Instantization is preferably performed by applying lecithin or another useful wetting agent to the surface of the powder. When instantization is applied the instantisation agent, e.g. lecithin dissolved in an edible oil, is typically added in an amount in the range of 0.5-2% w/w relative to the total final powder weight, and preferably in the range of 1.0-1.5% w/w relative to the total final powder weight.

The method furthermore preferably comprises a step of packaging the BLG isolate powder. A packaged BLG isolate powder product comprising a container containing the BLG isolate powder as described herein, and preferably a sealed container.

In some embodiments of the invention the BLG isolate powder is hermetically sealed in the container, optionally packaged with an inert gas.

A wide range of different containers may be used to store the BLG isolate powder. Preferred containers are e.g. a bag, a barrel, a pouch, a box, a can, and a sachet.

A particularly preferred embodiment of the invention pertains to a method of producing a dried BLG isolate powder containing BLG in an amount of at least 85% w/w relative to total protein, preferably at least 90% w/w, and even more preferably at least 94% w/w relative to total protein, the method comprising the steps of:

a) providing a liquid BLG isolate having

    i) a pH in the range of 2-4.9,
    ii) a pH of in the range of 6.1-8.5, or
    iii) a pH of in the range of 5.0-6.0,

said liquid BLG isolate containing BLG in an amount of at least 85% w/w relative to total protein, preferably at least 90% w/w, and even more preferably at least 94% w/w relative to total protein,
b) optionally, subjecting the liquid BLG isolate to a physical microbial reduction,
c) drying the liquid BLG isolate, preferably by spray-drying, wherein the provision of the liquid BLG isolate of step a) involves

- preparing BLG-enriched composition by a process comprising the steps of

    1) providing a whey protein solution comprising non-aggregated BLG and at least one additional whey protein, said whey protein solution is supersaturated with respect to BLG and has a pH in the range of 5-6,

    2) crystallising BLG in the supersaturated whey protein solution, and

    3) separating BLG crystals from the remaining whey protein solution,

    4) optionally, washing BLG crystals, e.g. the separated BLG crystals obtained from step 3) or 5), and

    5) optionally, re-crystallising BLG crystals, e.g. the BLG crystals obtained from step 3) or 4), and

- processing the BLG-enriched composition to at least dissolve the BLG crystals, thereby obtaining the liquid BLG isolate.

In the above embodiment, the BLG-enriched composition contains the separated BLG crystals from step 3) which are subsequently dissolved by an appropriate pH-adjustment or alternatively by increasing the conductivity and/or increasing the temperature.

A particularly preferred embodiment of the invention pertains to a method of producing a dried BLG isolate powder containing BLG in an amount of at least 85% w/w relative to total protein, preferably at least 90% w/w, and even more preferably at least 94% w/w relative to total protein, the method comprising the steps of:

a) providing a liquid BLG isolate having a pH in the range of 2.5-4.9, preferably 2.5-4.0, and even more preferably 3.0-3.9
said liquid BLG isolate containing BLG in an amount of at least 85% w/w relative to total protein, preferably at least 90% w/w, and even more preferably at least 94% w/w relative to total protein,
b) optionally, subjecting the liquid BLG isolate to a physical microbial reduction,
c) drying the liquid BLG isolate, preferably by spray-drying,

wherein the provision of the liquid BLG isolate of step a) comprises:

- preparing a BLG-enriched composition by a process comprising the steps of

    1) providing a whey protein solution comprising non-aggregated BLG and at least one additional whey protein, said whey protein solution being supersaturated with respect to BLG and having a pH in the range of 5-6,

    2) crystallising BLG in the supersaturated whey protein solution, and

    3) separating BLG crystals from the remaining whey protein solution,

    4) optionally, washing BLG crystals, e.g. the separated BLG crystals obtained from step 3) or 5), and

    5) optionally, re-crystallising BLG crystals, e.g. the BLG crystals obtained from step 3) or 4), and

- at least adjusting the pH of the BLG-enriched composition to a pH in the range of 2.5-4.9, preferably 2.5-4.0, and even more preferably 3.0-3.9.

Another particularly preferred embodiment of the invention pertains to a method of producing a dried BLG isolate powder containing BLG in an amount of at least 85% w/w relative to total protein, preferably at least 90% w/w, and even more preferably at least 94% w/w relative to total protein, the method comprising the steps of:

a) providing a liquid BLG isolate having a pH in the range of 6.1-8.5, preferably in the range of 6.3-8.0, and more preferably in the range of 6.5-7.5,
said liquid BLG isolate containing BLG in an amount of at least 85% w/w relative to total protein, preferably at least 90% w/w, and even more preferably at least 94% w/w relative to total protein,
b) optionally, subjecting the liquid BLG isolate to a physical microbial reduction,
c) drying the liquid BLG isolate, preferably by spray-drying,

wherein the provision of the liquid BLG isolate of step a) comprises:

- preparing a BLG-enriched composition by a process comprising the steps of

1) providing a whey protein solution comprising non-aggregated BLG and at least one additional whey protein, said whey protein solution being supersaturated with respect to BLG and having a pH in the range of 5-6,

2) crystallising BLG in the supersaturated whey protein solution, and

3) separating BLG crystals from the remaining whey protein solution,

4) optionally, washing BLG crystals, e.g. the separated BLG crystals obtained from step 3) or 5), and

5) optionally, re-crystallising BLG crystals, e.g. the BLG crystals obtained from step 3) or 4), and

- at least adjusting the pH of the BLG-enriched composition to a pH in the range of 6.1-8.5, preferably in the range of 6.3-8.0, and more preferably in the range of 6.5-7.5.

Alternative, but also preferred embodiments of the invention pertain to a method of producing a dried BLG isolate powder containing BLG in an amount of at least 85% w/w relative to total protein, preferably at least 90% w/w, and even more preferably at least 94% w/w relative to total protein, the method comprising the steps of:

a) providing a liquid BLG isolate having a pH in the range of 5.0-8.5, preferably 6.1-8.5, more preferably in the range of 6.3-8.0, and even more preferably in the range of 6.5-7.5,
said liquid BLG isolate containing BLG in an amount of at least 85% w/w relative to total protein, preferably at least 90% w/w, and even more preferably at least 94% w/w relative to total protein,
b) optionally, subjecting the liquid BLG isolate to a physical microbial reduction,
c) drying the liquid BLG isolate, preferably by spray-drying,

wherein the provision of the liquid BLG isolate of step a) comprises the following steps in the following sequence:

- preparing a BLG-enriched composition by a process comprising the steps of

1) providing a whey protein solution comprising non-aggregated BLG and at least one additional whey protein, said whey protein solution being supersaturated with respect to BLG and having a pH in the range of 5-6,

2) crystallising BLG in the supersaturated whey protein solution, and

3) separating BLG crystals from the remaining whey protein solution,

4) optionally, washing BLG crystals, e.g. the separated BLG crystals obtained from step 3) or 5), and

5) optionally, re-crystallising BLG crystals, e.g. the BLG crystals obtained from step 3) or 4), and

- adjusting the pH of the BLG-enriched composition to a pH in the range of 2.5-4.9, preferably 2.5-4.0, and even more preferably 3.0-3.9,
- physical microbial reduction which involves at least pasteurization, preferably using a temperature in the range of 70-82 degrees C, and even more preferably in the range of 70-80 degrees C, while the pH is in the range of 2.5-4.9, preferably 2.5-4.0, and even more preferably 3.0-3.9,
- pH adjustment to a pH in the range of 5.0-8.5, preferably 6.1-8.5, more preferably in the range of 6.3-8.0, and even more preferably in the range of 6.5-7.5,
- optionally, demineralisation.

In some preferred embodiments, the method of the present invention is implemented as a batch process. Alternatively, and sometimes preferably, the method may be implemented as a semi-batch process. In other preferred embodiments, the method is implemented as a continuous process.

Yet an aspect of the invention pertains to the liquid BLG isolate as described herein. The liquid BLG isolate is particularly useful for obtaining spray-dried BLG isolate powders and may furthermore be used as a liquid ingredient in the production of liquid or non-liquid food products. Alternatively but also preferred, the liquid BLG isolate may be used as a beverage as such.

In some preferred embodiments of the invention the BLG isolate powder of the present invention is obtainable by the method described herein.

In some preferred embodiments of the invention the liquid BLG isolate of the present invention is obtainable by the method described herein with the exception that the drying step is omitted.

An aspect of the invention pertains to the use of the BLG isolate powder or the liquid BLG isolate as defined herein as an ingredient for the production of a food product. The food product may e.g. be a beverage or an instant beverage powder.

The use of the BLG isolate powder or the liquid BLG isolate preferably provides one more of the following effect:

- Reduced level of drying mouthfeeling
- Improved transparency of the obtained liquid containing the BLG isolate powder or the liquid BLG isolate
- Reduced viscosity
- Possibility to increase the protein concentration of food products that are heat-treated beverages
- Has a lower color contribution (the inventors have observed that the present BLG isolate provides less color to e.g. protein beverages than corresponding WPI solution)

In some preferred embodiments of the invention the use of BLG isolate powder is as an ingredient for preparing a beverage having a protein content of at least 10-36% w/w, more preferably at least 15-35% w/w, even more preferably 20-34% w/w, and most preferably 25-33% w/w, and wherein the BLG isolate powder contributes with at least 90% w/w of the total protein of the beverage, more preferable at least 95% w/w and most preferably with all the protein of the beverage.

The BLG powders of the present invention are furthermore particularly suitable for high protein beverages, or shake powders for preparing high protein beverages as the present BLG powders contribute less to the viscosity than traditional WPIs and hence provides more drinkable beverages.

The food product may e.g. be a beverage or an instant beverage powder, having a pH in the range of 2-4.7 and furthermore having one or more of the following:

- a reduced level of drying mouthfeel,
- improved transparency, and/or
- increased content of protein, preferably heat-treated beverages containing a protein content in an amount of at least 3-45% w/w, more preferably 11-40% w/w, even more preferably 15-38% w/w and most preferably 20-36% w/w.

Preferred, numbered embodiments of the invention are described in the following:
Preferred, numbered embodiment 1. A BLG isolate powder, preferably prepared by spray-drying, having a pH in the range of i) 2.5-4.9, ii) 6.1-8.5, or iii) 5.0-6.0 and comprising:

- total protein in an amount of at least 30% w/w,
- beta-lactoglobulin (BLG) in an amount of at least 85% w/w relative to total protein
- water in an amount of at most 10% w/w,

said BLG isolate powder having one or more of the following:

- a bulk density of at least 0.2 g/cm$^3$,
- an intrinsic tryptophan fluorescence emission ratio (I330/I350) of at least 1.11,

- a degree of protein denaturation of at most 10%,
- a heat-stability at pH 3.9 of at most 200 NTU, and
- at most 15000 colony-forming units/g.

Preferred, numbered embodiment 2. The BLG isolate powder according to preferred, numbered embodiment 1 having a pH in the range of 2.5-4.9.

Preferred, numbered embodiment 3. The BLG isolate powder according to preferred, numbered embodiment 1 having a pH in the range of 6.1-8.5.

Preferred, numbered embodiment 4. The BLG isolate powder according to preferred, numbered embodiment 1 having a pH in the range of 5.0-6.0.

Preferred, numbered embodiment 5. The BLG isolate powder according to any of the preceding preferred, numbered embodiments comprising total protein in an amount of at least 40% w/w, preferably at least 50% w/w, at least 60% w/w, more preferably at least 70% w/w, even more preferably at least 80% w/w, even more preferably at least 90% w/w, and most preferably at least 92% w/w.

Preferred, numbered embodiment 6. The BLG isolate powder according to any of the preceding preferred, numbered embodiments comprising BLG in an amount of at least 88% w/w relative to total protein, preferably at least 90% w/w relative to total protein.

Preferred, numbered embodiment 7. The BLG isolate powder according to any of the preceding preferred, numbered embodiments wherein the powder comprises lipid in an amount of at most 10% w/w, preferably at most 5% w/w, more preferably at most 2% w/w, and even more preferably at most 0.1% w/w.

Preferred, numbered embodiment 8. The BLG isolate powder according to any of the preceding preferred, numbered embodiments having a bulk density of at least 0.20 $g/cm^3$, preferably at least 0.30 $g/cm^3$.

Preferred, numbered embodiment 9. The BLG isolate powder according to any of the preceding preferred, numbered embodiments having an intrinsic tryptophan fluorescence emission ratio (I330/I350) of at least 1.11.

Preferred, numbered embodiment 10. The BLG isolate powder according to any of the preceding preferred, numbered embodiments having a degree of protein denaturation of at most 10%.

Preferred, numbered embodiment 11. A liquid BLG isolate having a pH in the range of i) 2-4.9, ii) 6.1-8.5, or iii) 5.0-6.0 and comprising:

- total protein in an amount of at least 10% w/w,
- beta-lactoglobulin (BLG) in an amount of at least 85% w/w relative to total protein said BLG isolate powder having one or more of the following:
- an intrinsic tryptophan fluorescence emission ratio (I330/I350) of at least 1.11,
- a degree of protein denaturation of at most 10%,
- a heat-stability at pH 3.9 of at most 200 NTU, and
- at most 15000 colony-forming units/g, preferably at most 1000 colony-forming units/g.

Preferred, numbered embodiment 12. A method of producing a dried BLG isolate powder according to preferred, numbered embodiment 1 containing BLG in an amount of at least 85% w/w relative to total protein, the method comprising the steps of:

a) providing a liquid BLG isolate having

i) a pH in the range of 2-4.9,
ii) a pH of in the range of 6.1-8.5, or
iii) a pH of in the range of 5.0-6.0

said liquid BLG isolate containing BLG in an amount of at least 85% w/w relative to total protein,
b) optionally, subjecting the liquid BLG isolate to a physical microbial reduction,
c) drying the liquid BLG isolate by spray-drying.

Preferred, numbered embodiment 13. The method according to preferred, numbered embodiment 12 wherein the liquid BLG isolate comprises a total solids content in an amount in the range of 5-50% w/w.

Preferred, numbered embodiment 14. The method according to any of the preferred, numbered embodiments 12-13 wherein the protein fraction of the liquid BLG isolate has an intrinsic tryptophan fluorescence emission ratio (I330/I350) of at least 1.11.

Preferred, numbered embodiment 15. The method according to any of the preferred, numbered embodiments 12-14 wherein the protein of liquid BLG isolate has a degree of protein denaturation of at most 10% w/w.

Preferred, numbered embodiment 16. The method according to any of the preferred, numbered embodiments 12-15, wherein the provision of the liquid BLG isolate involves isolating BLG from whey protein feed, and optionally subjecting the resulting BLG-enriched composition to one or more steps selected from the group of:

- demineralisation,
- addition of minerals,
- dilution,
- physical microbial reduction,
- concentration, and
- pH-adjustment.

Preferred, numbered embodiment 17. The method according to any of the preferred, numbered embodiments 12-16, wherein the liquid BLG isolate is subjected to physical microbial reduction, which preferably involves or even consists of heat-treatment.

18. Use of the BLG isolate powder according to any of preferred, numbered embodiments 1-10 or the liquid BLG isolate according to preferred, numbered embodiment 11 as an ingredient for the production of a food product, e.g. a beverage or an instant beverage powder, having a pH in the range of 2-4.7 and furthermore having one or more of the following:

- a reduced level of drying mouthfeel,
- improved transparency, and/or
- increased content of protein, preferably heat-treated beverages containing a protein content in an amount of at least 3-45% w/w, more preferably 11-40% w/w, even more preferably 15-38% w/w and most preferably 20-36% w/w.

It should be noted that the embodiments and features described in the context of one of the aspects of the present invention also apply to the other aspects of the invention.

All patent and non-patent references cited in the present application are hereby incorporated by reference in their entirety.

The invention will now be described in further details in the following non-limiting examples.

## EXAMPLES

### EXAMPLE 1: METHODS OF ANALYSIS

### EXAMPLE 1.1: DETERMINATION OF PROTEIN NATIVENESS BY INTRINSIC TRYPTOPHAN FLUORESCENCE

Tryptophan (Trp) fluorescence spectroscopy is a well-described tool to monitor protein folding and unfolding. Trp residues buried within native proteins typically display highest fluorescence emission around 330nm than when present in more solvent exposed positions such as unfolded proteins. In unfolded proteins, the wavelengths for Trp fluorescence emission typically shift to higher wavelengths and are often measured around 350nm. We here exploit this transition to monitor thermally induced unfolding by calculating the ratio between fluorescence emission at 330nm and 350nm to investigate the influence of heating temperature.

The analysis comprises the following steps:

- Beverage compositions were diluted to 0.6mg/ml in MQ water.
- 300$\mu$l sample was transferred to white 96-well plate avoiding bubbles or 3mL was transferred to 10mm quartz cuvette.
- The tryptophan fluorescence emission intensity between 310 and 400nm was recorded from the top by excitation at 295 using 5nm slits.
- Samples were measured at 22°C using a Cary Eclipse fluorescence spectrophotometer equipped with a plate reader accessory (G9810A) or single cuvette holder.
- The emission intensity ratio was calculated by dividing the measured fluorescence emission intensity at 330nm with the emission intensity at 350nm, R = I330/I350, and used as a measure of protein nativity.

  ○ R of at least 1.11 describes a predominant native BLG conformation and
  ○ R of less than 1.11 reports on at least partial unfolding and aggregation.

### EXAMPLE 1.2: HEAT-STABILITY AT PH 3.9

**Heat-stability at pH 3.9:**

The heat-stability at pH 3.9 is a measure of the ability of protein composition to stay clear upon prolonged pasteurization at pH 3.9.

The heat-stability at pH 3.9 is determined by forming an aqueous solution having a pH of 3.9 and comprising 6.0% w/w protein by mixing a sample of the powder or liquid to be tested with water (or alternatively concentrating it by low temperature evaporation if it is a dilute liquid) and adjusting the pH to 3.9 with the minimum amount of 0.1 M NaOH or 0.1 M HCl required.

The pH-adjusted mixture is allowed to rest for 30 minutes after which 25 mL of the mixture is transferred to a 30 mL thin-walled glass test tube. It is heated to 75.0 degrees C for 300 seconds by immersion into a water-bath having a temperature of 75.0 degrees C. Immediately after the heating, the glass test tube is cooled to 1-5 degrees C by transferring it to an ice bath and the turbidity of the heat-treated sample is measured according to Example 1.7.

## EXAMPLE 1.3: DETERMINATION OF THE DEGREE OF PROTEIN DENATURATION OF A WHEY PROTEIN COMPOSITION

Denatured whey protein is known to have a lower solubility at pH 4.6 than at pH values below or above pH 4.6, therefore the degree of denaturation of a whey protein composition is determined by measuring the amount of soluble protein at pH 4.6 relative to the total amount of protein at a pH where the proteins in the solution are stable.

More specifically for whey proteins, the whey protein composition to be analysed (e.g. a powder or an aqueous solution) is converted to:

- a first aqueous solution containing 5.0% (w/w) total protein and having a pH of 7.0 or 3.0, and
- a second aqueous solution containing 5.0% (w/w) total protein and having a pH of 4.6.

pH adjustments are made using 3% (w/w) NaOH (aq) or 5% (w/w) HCl (aq).

The total protein content ($P_{pH\ 7.0\ or\ 3.0}$) of the first aqueous solution is determined according to example 1.5.

The second aqueous solution is stored for 2 h at room temperature and subsequently centrifuged at 3000 g for 5 minutes. A sample of the supernatant is recovered and analysed according to Example 1.5 to give the protein concentration in the supernatant ($S_{pH4.6}$).

The degree of protein denaturation, D, of the whey protein composition is calculated as:

$$D = ((P_{pH\ 7.0\ or\ 3.0} - S_{pH\ 4.6})/\ P_{pH\ 7.0\ or\ 3.0}) * 100\%$$

## EXAMPLE 1.4 DETERMINATION OF PROTEIN DENATURATION (WITH PH 4.6 ACID PRECIPITATION) USING REVERSE PHASE UPLC ANALYSIS.

BLG samples (such as non-heated reference and heated BLG beverage compositions) were diluted to 2% in MQ water. 5mL protein solution, 10mL Milli-Q, 4mL 10% acetic acid and 6mL 1.0M NaOAc are mixed and stirred for 20 minutes to allow precipitation agglomeration of denatured protein around pH 4.6. The solution is filtered through $0.22\mu m$ filter to remove agglomerates and non-native proteins.

All samples were subjected to the same degree of dilution by adding polished water.

For each sample, the same volume was loaded on an UPLC system with a UPLC column (Protein BEH C4; 300Å; 1.7 $\mu m$; 150 x 2.1 mm) and detected at 214nm.

The samples were run using the following conditions:

Buffer A: Milli-Q water, 0.1%w/w TFA
Buffer B: HPLC grade acetonitrile, 0.1%w/w TFA
Flow: 0.4ml/min
Gradient: 0-6.00 minutes 24-45%B; 6.00-6.50 minutes 45-90%B; 6.50-7.00 minutes 90%B; 7.00-7.50 minutes 90-24%B and 7.50-10.00 minutes 24%B.

The area of BLG peaks against a protein standard (Sigma L0130) was used to determine the concentration of native BLG in samples (5 level calibration curve)

Samples were diluted further and reinjected if outside linear range.

**EXAMPLE 1.5: DETERMINATION TOTAL PROTEIN**

The total protein content (true protein) of a sample is determined by:

1) Determining the total nitrogen of the sample following ISO 8968-1/2|IDF 020-1/2- Milk - Determination of nitrogen content - Part 1/2: Determination of nitrogen content using the Kjeldahl method.

2) Determining the non-protein nitrogen of the sample following ISO 8968-4|IDF 020-4- Milk - Determination of nitrogen content - Part 4: Determination of non-protein-nitrogen content.

3) Calculating the total amount protein as ($m_{total\ nitrogen}$ - $m_{non-protein-nitrogen}$)*6.38.

**EXAMPLE 1.6: DETERMINATION OF NON-AGGREGATED BLG, ALA, AND CMP**

The content of non-aggregated alpha-lactalbumin (ALA), beta-lactoglobulin (BLG) and caseinomacropeptide (CMP), respectively was analysed by HPLC analysis at 0.4mL/min. 25 microL filtered sample is injected onto 2 TSKgel3000PWxl (7.8 mm 30 cm, Tosohass, Japan) columns connected in series with attached pre-column PWxl (6 mm x 4 cm, Tosohass, Japan) equilibrated in the eluent (consisting of 465g Milli-Q water, 417.3 g acetonitrile and 1mL triflouroace-tic acid) and using a UV detector at 210nm.
Quantitative determination of the contents of native alpha-lactalbumin ($C_{alpha}$), beta-lactoglobulin ($C_{beta}$), and caseinomacropeptide ($C_{CMP}$) was performed by comparing the peak areas obtained for the corresponding standard proteins with those of the samples.
The total amount of additional protein (non-BLG protein) was determined by subtracting the amount of BLG from the amount of total protein (determined according to Example 1.5)

**EXAMPLE 1.7: DETERMINATION OF TURBIDITY**

Turbidity is the cloudiness or haziness of a fluid caused by large number of particles that are generally invisible to the naked eye, similar to smoke in air.
Turbidity is measured in nephelometric turbidity units (NTU).
20mL beverages/samples were added to NTU-glass and placed in the Turbiquant® 3000 IR Tur-bidimeter. The NTU-value was measured after stabilisation and repeated twice.

**EXAMPLE 1.8: DETERMINATION OF VISCOSITY**

The viscosity of a liquid is measured using a Viscoman viscometer by Gilson or a comparable viscometer and reported at a shear rate of $300s^{-1}$. Unless stated otherwise the samples are equilibrated to 15 °C prior to the measurement and measured at that temperature.
The viscosity is presented in the unit centipoise (cP) at a shear rate of $300\ s^{-1}$ unless otherwise stated. The higher the measured cP values, the higher the viscosity.

**EXAMPLE 1.9: DETERMINATION OF COLOUR**

The colour was measured using a Chroma Meter (Konica Minolta, CR-400). 15 g sample was added to a small petri dish (55x14.2mm, VWR Cat# 391-0895) avoiding bubble formation. The protein content of the samples was standardised to 6.0w/w% protein or less.
The Chroma Meter was calibrated to a white calibration plate (No. 19033177). The illuminant was set to D65 and the observer to 2 degree. The color (CIELAB colour space, a*-,b*-, L*-value) was measured with lids covering the suspension, as the average of three individual readings in different places of the petri dish.
Demineralised water reference has the following values:

L* 39.97±0.3

a* 0.00 ± 0.06

b* -0.22±0.09

The measurements were converted to delta/difference values based on demineralised water measurement.

$$delta\ L* = L_{sample\ standardised\ to\ 6.0\ w/w\%\ protein}* - L_{demin.\ water}*\ ,$$

measured at room temperature.

$$delta\ a* = a_{sample\ standardised\ to\ 6.0\ w/w\%\ protein}* - a_{demin.\ water}*\ ,$$

measured at room temperature.

$$delta\ b* = b_{sample\ standardised\ to\ 6.0\ w/w\%\ protein}* - b_{demin.\ water}*\ ,$$

measured at room temperature.

The sample is standardized to 6.0w/w% protein or below.

The L\*a\*b\* colour space (also referred to as the CIELAB space) is one of the uniform colour spaces defined by the International Commission on Illumination (CIE) in 1976 and was used to quantitatively report lightness and hue (ISO 11664-4:2008(E)/CIE S 014- 4/E:2007).

In this space, L\* indicates lightness (value from 0-100), the darkest black at L\* = 0, and the brightest white at L\* = 100.

The colour channels a\* and b\*, represent true neutral grey values at a\* = 0 and b\* = 0. The a\* axis represents the green-red component, with green in the negative direction and red in the positive direction. The b\* axis represents the blue-yellow component, with blue in the negative direction and yellow in the positive direction.

**EXAMPLE 1.10 BEVERAGE STABILITY TEST/INSOLUBLE PROTEIN MATTER**

Whey protein beverage compositions were considered stable if less than 15% of total protein in heated samples precipitated upon centrifugation at 3000 g for 5 minutes:

- Approx. 20 g samples were added to centrifuge tubes and centrifuged at 3000 g 5 min.
- Kjeldahl analysis of protein before centrifugation and the supernatant after centrifugation were used to quantify protein recovery See example 1.5

The loss of protein is calculated:

$$Denaturation\% = \left(\frac{P_{total} - P_{3000xg}}{P_{total}}\right) * 100\%$$

This parameter is also sometimes referred to as the level of insoluble protein matter and can be used for analyzing both liquid and powder samples. If the sample is a powder, 10 g of the powder is suspended in 90 g demineralized water and allowed to hydrate at 22 degrees C under gentle stirring for 1 hour. Approx. 20 g of sample (e.g. liquid sample or the suspended powder sample) to centrifuge tubes and centrifuged at 3000 g 5 min. Kjeldahl analysis of protein before centrifugation ($P_{total}$) and the supernatant after centrifugation ($P_{3000xg}$) were used to quantify protein recovery according to Example 1.5.

The amount of insoluble protein matter is calculated:

$$percentage\ of\ insoluble\ protein\ matter = \left(\frac{P_{total} - P_{3000xg}}{P_{total}}\right) * 100\%$$

**EXAMPLE 1.11: SENSORY EVALUATION**

The heat-treated beverage preparations underwent a descriptive sensory evaluation. The beverage preparations had been subjected to heat using plate heat exchangers.

1 volume sample was mixed with 1 volume water and compared to non-heated whey protein isolate, lactic acid and citric acid are also used to form an attribute list prior to the final tasting session:

| Category | Attributes: |
|----------|-------------|
| Aroma | Whey, acidic (sour milk product) |
| Basic taste | Acid, bitter |
| Flavour | Whey, citric acid, lactic acid |
| Mouth feeling | Drying, astringency |

Crackers, white tea, melon and water were used to cleanse the mouth of participants between each sample.

15mL test sample at ambient temperature (20-25°C) was served in small cups.

Test samples were each served to 10 individuals three times in three different blocks in randomised order.

The attributes (see table above) were rated on a 15cm scale with 0 = low intensity and 15 = high intensity.

The statistical analysis was conducted in 'Panelcheck' software using a 3-way ANOVA test for multiple replicates. Samples were fixed and panel was set to random.

Bonferroni correction implying least significance difference values (pairwise comparisons of groups associated to a letter) was used to evaluate significant differences between samples.

## EXAMPLE 1.12: DETERMINATION OF TRANSPARENCY BY IMAGING

Photographs of beverage preparations were conducted by placing samples in turbidity NTU measuring vials touching a piece of paper with 'lorem ipsum' text. Vials were photographed using a smartphone and the inventors evaluated whether the text could be clearly observed through the vial.

## EXAMPLE 1.13: DETERMINATION OF ASH CONTENT

The ash content of a food product is determined according to NMKL 173:2005 "Ash, gravimetric determination in foods".

## EXAMPLE 1.14: DETERMINATION OF CONDUCTIVITY

The "conductivity" (sometimes referred to as the "specific conductance") of an aqueous solution is a measure of the ability of the solution to conduct electricity. The conductivity may e.g. be determined by measuring the AC resistance of the solution between two electrodes and the result is typically given in the unit milliSiemens per cm (mS/cm). The conductivity may for example be measured according to the EPA (the US Environmental Protection Agency) Method No. 120.1.

Conductivity values mentioned herein have been normalised to 25 degrees C unless it is specified otherwise.

The conductivity is measured on a Conductivity meter (WTW Cond 3210 with a tetracon 325 electrode).

The system is calibrated as described in the manual before use. The electrode is rinsed thoroughly in the same type of medium as the measurement is conducted on, in order to avoid local dilutions. The electrode is lowered into the medium so that the area where the measurement occurs is completely submerged. The electrode is then agitated so that any air trapped on the electrode is removed. The electrode is then kept still until a stable value can be obtained and recorded from the display.

## EXAMPLE 1.15: DETERMINATION OF THE TOTAL SOLIDS OF A SOLUTION

The total solids of a solution may be determined according NMKL 110 2nd Edition, 2005 (Total solids (Water) - Gravimetric determination in milk and milk products). NMKL is an abbreviation for "Nordisk Metodikkomité for Næringsmidler".

The water content of the solution can be calculated as 100% minus the relative amount of total solids (% w/w).

## EXAMPLE 1.16: DETERMINATION OF PH

All pH values are measured using a pH glass electrode and are normalised to 25 degrees C. The pH glass electrode (having temperature compensation) is rinsed carefully before and calibrated before use.

When the sample is in liquid form, then pH is measured directly in the liquid solution at 25 degrees C.

When the sample is a powder, 10 gram of a powder is dissolved in 90 ml of demineralised water at room temperature while stirring vigorously. The pH of the solution is then measured at 25 degrees C.

**EXAMPLE 1.17: DETERMINATION OF LOOSE DENSITY AND BULK DENSITY**

The density of a dry powder is defined as the relation between weight and volume of the powder which is analysed using a special Stampf volumeter (i.e. a measuring cylinder) under specified conditions. The density is typically expressed in g/ml or kg/L.

In this method, a sample of dried powder is tamped in a measuring cylinder. After a specified number of tappings, the volume of the product is read and the density is calculated.

Three types of densities can be defined by this method:

- Poured density, which is the mass divided with the volume of powder after it has been transferred to the specified measuring cylinder.

- Loose density, which is the mass divided with the volume of powder after 100 tappings according to the specified conditions in this standard.

- Bulk density, which is the mass divided with the volume of powder after 625 tappings according to the specified conditions in this standard.

The method uses a special measuring cylinder, 250 ml, graduated 0-250 ml, weight $190 \pm 15$ g (J. Engelsmann A. G. 67059 Ludwigshafen/Rh) and a Stampf volumeter, e.g. J. Engelsmann A. G.

The loose density and the bulk density of the dried product are determined by the following procedure.

Pre-treatment:

The sample to be measured is stored at room temperature.

The sample is then thoroughly mixed by repeatedly rotating and turning the container (avoid crushing particles). The container is not filled more than 2/3.

Procedure:

Weigh $100.0 \pm 0.1$ gram of powder and transfer it to the measuring cylinder. The volume $V_0$ is read in ml.

If 100 g powder does not fit into the cylinder, the amount should be reduced to 50 or 25 gram.

Fix the measuring cylinder to the Stampf volumeter and let it tap 100 taps. Level the surface with the spatula and read the volume $V_{100}$ in ml.

Change the number of tabs to 625 (incl. the 100 taps). After tapping, level the surface and read the volume $V_{625}$ in ml.

Calculation of densities:

Calculate the loose and the bulk densities expressed in g/ml according to the following formula:

$$\text{Bulk density} = M/V$$

where M designates weighed sample in grams and V designates volume after 625 tappings in ml.

**EXAMPLE 1.18: DETERMINATION OF THE WATER CONTENT OF A POWDER**

The water content of a food product is determined according to ISO 5537:2004 (Dried milk - Determination of moisture content (Reference method)). NMKL is an abbreviation for "Nordisk Metodikkomité for Næringsmidler".

**EXAMPLE 1.19: DETERMINATION OF THE AMOUNTS OF CALCIUM, MAGNESIUM, SODIUM, POTASSIUM, PHOSPHORUS (ICP-MS METHOD)**

The total amounts of calcium, magnesium, sodium, potassium, and phosphorus are determined using a procedure in which the samples are first decomposed using microwave digestion, and then the total amount of mineral(s) is determined using an ICP apparatus.

Apparatus:

The microwave is from Anton Paar and the ICP is an Optima 2000DV from PerkinElmer Inc.

Materials:

1 M $HNO_3$
Yttrium in 2% $HNO_3$
Suitable standards for calcium, magnesium, sodium, potassium, and phosphorus in 5% $HNO_3$

Pre-treatment:

Weigh out a certain amount of powder and transfer the powder to a microwave digestion tube. Add 5 mL 1M $HNO_3$. Digest the samples in the microwave in accordance with microwave instructions. Place the digested tubes in a fume cupboard, remove the lid and let volatile fumes evaporate.

Measurement procedure:

Transfer pre-treated sample to DigiTUBE using a known amount of Milli-Q water. Add a solution of yttrium in 2% $HNO_3$ to the digestion tube (about 0.25 mL per 50 mL diluted sample) and dilute to known volume using Milli-Q water. Analyse the samples on the ICP using the procedure described by the manufacturer.

A blind sample is prepared by diluting a mixture of 10 mL 1M $HNO_3$ and 0.5 mL solution of yttrium in 2% $HNO_3$ to a final volume of 100 mL using Milli-Q water.

At least 3 standard samples are prepared having concentrations which bracket the expected sample concentrations.

**EXAMPLE 1.20: DETERMINATION OF THE FUROSINE-VALUE:**

The furosine value is determined as described in "Maillard Reaction Evaluation by Furosine Determination During Infant Cereal Processing", Guerra-Hernandez et al, Journal of Cereal Science 29 (1999) 171-176, and the total amount of protein is determined according to Example 1.5. The furosine value is reported in the unit mg furosine per 100 g protein.

**EXAMPLE 1.21: DETERMINATION OF THE CRYSTALLINITY OF BLG IN A LIQUID**

The following method is used to determine the crystallinity of BLG in a liquid having a pH in the range of 5-6.

a) Transfer a 10 mL sample of the liquid in question to a Maxi-Spin filter with a 0.45 micron pore size CA membrane.
b) Immediately spin the filter at 1500 g for 5 min. keeping the centrifuge at 2 degrees C
c) Add 2 mL cold Milli-Q water (2 degrees C) to the retentate side of the spin filter and immediately, spin the filter at 1500 g for 5 min while keeping the centrifuge cooled at 2 degrees C, collect the permeate (permeate A), measure the volume and determine BLG concentration via HPLC using the method outlined in Example 1.6.
d) Add 4 mL 2M NaCl to the retentate side of the filter, agitate quickly and allow the mixture to stand for 15 minutes at 25 degrees C.
e) Immediately spin the filter at 1500 g for 5 min and collect the permeate (permeate B)
f) Determine the total weight of BLG in permeate A and permeate B using the method outlined in Example 1.6 and convert the results to total weight of BLG instead of weight percent. The weight of BLG in permeate A is referred to as $m_{Permeate\ A}$ and the weight of BLG in permeate B is referred to as $M_{Permeate\ B}$.
g) The crystallinity of the liquid with respect to BLG is determined as:

$$crystallinity = m_{Permeate\ B}/(\ m_{Permeate\ A} + m_{Permeate\ B})*100\%$$

**EXAMPLE 1.22: DETERMINATION OF THE CRYSTALLINITY OF BLG IN A DRY POWDER**

This method is used to determine the crystallinity of BLG in a dry powder.

a) 5.0 gram of the powder sample is mixed with 20.0 gram of cold Milli-Q water (2 degrees C) and allowed to stand for 5 minute at 2 degrees C.

b) Transfer the sample of the liquid in question to a Maxi-Spin filter with a 0.45 micron CA membrane.

c) Immediately spin the filter at 1500 g for 5 min. keeping the centrifuge at 2 degrees C

d) Add 2 mL cold Milli-Q water (2 degrees C) to the retentate side of the spin filter and immediately, spin the filter at 1500 g for 5 min, collect the permeate (permeate A), measure the volume and determine BLG concentration via HPLC using the method outlined in Example 1.6 and convert the results to total weight of BLG instead of weight percent. The weight of BLG in permeate A is referred to as $m_{permeate\ A}$

f) The crystallinity of BLG in the powder is then calculated using the following formula:

$$crystallinity = \frac{m_{BLG\ total} - m_{permeate\ A}}{m_{BLG\ total}} * 100\%$$

where $m_{BLG\ total}$ is the total amount of BLG in the powder sample of step a).

If the total amount of BLG of powder sample is unknown, this may be determined by suspending another 5 g powder sample (from the same powder source) in 20.0 gram of Milli-Q water, adjusting the pH to 7.0 by addition of aqueous NaOH, allowing the mixture to stand for 1 hour at 25 degrees C under stirring, and finally determining the total amount of BLG of the powder sample using Example 1.6.

## EXAMPLE 1.23: DETERMINATION OF UF PERMEATE CONDUCTIVITY

15 mL of sample is transferred to an Amicon Ultra-15 Centrifugal Filter Units with a 3 kDa cut off (3000 NMWL) and centrifugated at 4000 g for 20-30 minutes or until a sufficient volume of UF permeate for measuring conductivity is accumulated in the bottom part of the filter units. The conductivity is measured immediately after centrifugation. The sample handling and centrifugation are performed at the temperature of the source of the sample.

## EXAMPLE 1.24: DETECTION OF DRIED BLG CRYSTALS IN A POWDER

The presence of dried BLG crystals in a powder can be identified the following way:

A sample of the powder to be analysed is re-suspended and gently mixed in demineralised water having a temperature of 4 degrees C in a weight ratio of 2 parts water to 1 part powder, and allowed to rehydrate for 1 hour at 4 degrees C.

The rehydrated sample is inspected by microscopy to identify presence of crystals, preferably using plan polarised light to detect birefringence.

Crystal-like matter is separated and subjected to x-ray crystallography in order verify the existence of crystal structure, and preferably also verifying that the crystal lattice (space group and unit cell dimensions) corresponds to those of a BLG crystal.

The chemical composition of the separated crystal-like matter is analysed to verify that its solids primarily consists of BLG.

## EXAMPLE 1.25: DETERMINATION OF THE TOTAL AMOUNT OF LACTOSE

The total amount of lactose is determined according to ISO 5765-2:2002 (IDF 79-2: 2002) "Dried milk, dried ice-mixes and processed cheese - Determination of lactose content - Part 2: Enzymatic method utilizing the galactose moiety of the lactose".

## EXAMPLE 1.26: DETERMINATION OF THE TOTAL AMOUNT OF CARBOHYDRATE:

The amount of carbohydrate is determined by use of Sigma Aldrich Total Carbohydrate Assay Kit (Cat MAK104-1KT) in which carbohydrates are hydrolysed and converted to furfural and hy-droxyfurfurals which are converted to a chromagen that is monitored spectrophotometrically at 490nm.

## EXAMPLE 1.27: DETERMINATION OF THE TOTAL AMOUNT OF LIPIDS

The amount of lipid is determined according to ISO 1211:2010 (Determination of Fat Content - Röse-Gottlieb Gravimetric Method).

## EXAMPLE 1.28: DETERMINATION OF BRIX

Brix measurements were conducted using a PAL-$\alpha$ digital hand-held refractometer (Atago) calibrated against polished water (water filtered by reverse osmosis to obtain a conductivity of at most 0.05 mS/cm).

Approx. 500$\mu$l of sample was transferred to the prism surface of the instrument and the measurement was started. The measured value was read and recorded.

The Brix of a whey protein solution is proportional to the content of total solids (TS) and TS (%w/w) is approx. Brix * 0.85.

Brix is also sometimes referred to as degrees Brix or °Brix.

## EXAMPLE 1.29 DETERMINATION OF LACTOFERRIN AND LACTOPEROXIDASE

The concentration of lactoferrin is determined by an ELISA immunoassay as outlined by Soyeurt 2012 (Soyeurt et al; Mid-infrared prediction of lactoferrin content in bovine milk: potential indicator of mastitis; Animal (2012), 6:11, pp 1830-1838)
The concentration of lactoperoxidase is determined using a commercially available bovine lactoperoxidase kit.

## EXAMPLE 1.30: DETERMINATION THE NUMBER OF COLONY-FORMING UNITS

The determination of the number of colony-forming units per gram sample is performed according to ISO 4833-1:2013(E): Microbiology of food and animal feeding stuffs - horizontal method for the enumeration of microorganisms - Colony-count technique at 30°C.

## EXAMPLE 1.31: DETERMINATION OF THE TOTAL AMOUNT OF BLG, ALA, AND CMP

This procedure is a liquid chromatographic (HPLC) method for the quantitative analysis of proteins such as ALA, BLG and CMP and optionally also other protein species in a composition. Contrary to the method of Example 1.6 the present method also measures proteins that are present in aggregates and therefore provides a measure of the total amount of the protein species in the composition in question.

The mode of separation is Size Exclusion Chromatography (SEC) and the method uses 6M Guanidine HCl buffer as both sample solvent and HPLC mobile phase. Mercaptoethanol is used as a reducing agent to reduce the disulphide (S-S) in the proteins or protein aggregates to create unfolded monomeric structures.

The sample preparation is easily achieved by dissolving 10mg protein equivalent in the mobile phase.

Two TSK-GEL G3000SWXL (7.7mm x 30.0cm) columns (GPC columns) and a guard column are placed in series to achieve adequate separation of the major proteins in raw materials.

The eluted analytes are dectected and quantified by UV detection (280nm).

### Equipment /Materials :

1. HPLC Pump 515 with manual seal wash ( Waters )
2. HPLC Pump Controller Module II (Waters)
3. Autosampler 717 (Waters)
4. Dual Absorbance Detector 2487 (Waters)
5. Computer software capable of generating quantitative reports ( Empower 3, Waters )
6. Analytical column: Two TSK-GEL G3000SWXL (7.8 x 300mm, P/N: 08541). Guard Column: TSK- Guard Column SWxL (6.0 x 40mm, P/N: 08543) .
7. Ultrasonic Bath ( Branson 5200 )
8. 25mm Syringe filter with 0.2 $\mu$m Cellulose Acetate membrane. ( 514-0060, VWR )

### Procedure :

Mobile Phase :

A. Stock buffer solution.

1. Weigh 56.6g of $Na_2HPO_4$, 3.5g of $NaH_2PO_4$, and 2.9g of EDTA in to a 1000mL beaker. Dissolve in 800mL of water.
2. Measure pH and adjust to 7.5 $\pm$ 0.1, if necessary, with HCl (decrease pH) or NaOH (increase pH).
3. Transfer to a 1000mL volumetric flask and dilute to volume with water.

B. 6M Guanidine HCl Mobile Phase.

1. Weigh 1146 g of Guanidine HCl in to a 2000mL beaker, and add 200mL of the stock buffer solution(A)
2. Dilute this solution to about 1600mL with water while mixing with a magnetic stir bar (50°C)
3. Adjust the pH to 7.5 $\pm$ 0.1 with NaOH.
4. Transfer into a 2000mL volumetric flask and dilute to volume with water.
5. Filter using the solvent filtration apparatus with the 0.22$\mu$m membranefilter.

Calibration Standards.

Calibration standards of each protein to be quantified are prepared the following way:

1. Weigh accurately (to 0.01mg) about 25mg of the protein reference standard into a 10mL volumetric flask and dissolve in 10mL of water.
This is the protein stock standard solution (S1) of the protein
2. Pipette 200 $\mu$l of S1 into a 20ml volumetric flask and dilute to volume with mobile phase. This is the low working standard solution WS1.
3. Pipette 500 $\mu$L of S1 into a 10mL volumetric flask and dilute to volume with mobile phase. This is standard solution WS2.
4. Pipette 500 $\mu$L of S1 into a 5mL volumetric flask and dilute to volume with mobile phase. This is standard solution WS3.
5. Pipette 750 $\mu$L of S1 into a 5mL volumetric flask and dilute to volume with mobile phase. This is standard solution WS4.
6. Pipette 1.0 mL of S1 into a 5mL volumetric flask and dilute to volume with mobile phase. This is the high working standard solution WS5.
7. Using graduated disposable pipettes transfer 1.5mL of WS1-5 into separate vials. Add 10 $\mu$L of 2-mercaptoethanol to each vial and cap. Vortex the solutions for 10 sec. Let the standards stay at ambient temperature for about 1 hr.
8. Filter the standards using 0.22 $\mu$m Cellulose Acetate syringe filters.

The purity of protein is measured using Kjeldahl ( N x 6.38 ) and the area % from standard solution WS5 using the HPLC.

$$protein\ (mg) = \text{``protein standard weight''}\ (mg)\ x\ P1\ x\ P2$$

P1 = P% (Kjeldahl)
P2 = protein area% (HPLC)

Sample preparation

1. Weigh the equivalent of 25mg of protein of the original sample into a 25mL volumetric flask.
2. Add approximately 20mL of mobile phase and let the sample dissolve for about 30min.
3. Add mobile phase to volume and add 167$\mu$L of 2-mercaptoethanol to the 25ml sample solution.
4. Sonicate for about 30min and afterwards let the sample stay at ambient temperature for about 1½ hours.
5. Mix the solution and filter using 0.22$\mu$l Cellulose Acetate syringe filters.

**HPLC system/columns**

Column Equilibration

1. Connect the GPC guard column and the two GPC analytical columns in series. New columns are generally shipped in a phosphate-salt buffer.

2. Run water through a new column gradually from 0.1 to 0.5mL/min in 30 to 60mins. Continue flushing for about 1 hour.

3. Gradually decrease flow rate from 0.5mL/min to 0.1mL/min and replace with mobile phase in the reservoir.

4. Increase pump flow rate gradually from 0.1 to 0.5mL/min in 30 to 60mins to avoid pressure shock and leave at 0.5mL/min.

5. Inject ten samples to allow the column to be saturated and wait for the peaks to elute. This will aid in the conditioning of the column.

This step is done without the need of waiting for each injection to be complete before injecting the next.

6. Equilibrate with the mobile phase at least 1 hour.

Calculation of the results

Quantitative determination of the contents of the proteins to be quantified, e.g. alpha-lactalbumin, beta-lactoglobulin, and caseinomacropeptide, is performed by comparing the peak areas obtained for the corresponding standard proteins with those of the samples. The results are reported as g specific protein/100 g of the original sample or weight percentage of the specific protein relative to the weight of the original sample.

**EXAMPLE 2: PRODUCTION OF A SPRAY-DRIED, ACIDIC BLG ISOLATE POWDER**

Whey protein feed

Lactose-depleted UF retentate derived from sweet whey from a standard cheese production process was filtered through a 1.2 micron filter and had been fat-reduced via a Synder FR membrane prior to being used as feed for the BLG crystallisation process. The chemical composition of the feed can be seen in Table 1. We note that all weight percentages of specific proteins, such as BLG, ALA, mentioned in this Example pertain to the weight percentage of the non-aggregated proteins relative to total protein.

Conditioning

The sweet whey feed was conditioned on an ultrafiltration setup at 20 degrees C, using a Koch HFK-328 type membrane (70 $m^2$ membrane) with a 46 mill spacer feed pressure 1.5-3.0 bar, to a feed concentration of 21% total solids (TS) $\pm 5$, and using as diafiltration medium polished water (water filtered by reverse osmosis to obtain a conductivity of at most 0.05 mS/cm). The pH was then adjusted by adding HCl so that the pH was approx. 5.5. Diafiltration continued until the drop in conductivity of the retentate was below 0.1 mS/cm over a 20 min period. The retentate was then concentrated until the permeate flow was below 1.43 L/h/$m^2$. A first sample of concentrated retentate was taken and subjected to centrifugation at 3000 g for 5 minutes. The supernatant of the first sample was used for the determination of BLG yield.

Crystallisation

The concentrated retentate was transferred to a 300L crystallisation tank where it was seeded with pure BLG crystal material made from rehydrated, spray-dried BLG crystals. Subsequently, the seeded whey protein solution was cooled from 20 degrees C to approx. 6 degrees C over approx. 10 hours to allow the BLG crystals to form and grow.

After cooling, a sample of the crystal-containing whey protein solution (the second sample) was taken and the BLG crystals were separated by centrifugation at 3000 g for 5 minutes. The supernatant and crystal pellets from the second sample were subjected to HPLC analysis as described below. The yield of crystallisation was calculated as outlined below and determined to 57%.

Table 1 Chemical composition of the feed

| Feed standardized to 95% total solids | | |
|---|---|---|
| Protein composition % w/w of total protein | | |
| ALA | | 10.2 |
| BLG | | 59.6 |
| Other proteins | | 30.2 |

(continued)

| Selected other components % w/w | | |
|---|---|---|
| Ca | | 0.438 |
| K | | 0.537 |
| Mg | | 0.077 |
| Na | | 0.131 |
| P | | 0.200 |
| Fat | | 0.220 |
| protein concentration | | 87 |

BLG yield determination using HPLC:

The supernatants of the first and second samples were subjected to the same degree of dilution by adding polished water and the diluted supernatants were filtered through a 0.22$\mu$m filter.

For each filtered and diluted supernatant the same volume was loaded on an HPLC system with a Phenomenex Jupiter® 5 $\mu$m C4 300 Å, LC Column 250 x 4.6 mm, Ea. and detected at 214nm.

The samples were run using the following conditions:

Buffer A: MilliQ water, 0.1%w/w TFA

Buffer B: HPLC grade acetonitrile, 0.085%w/w TFA

Flow: 1mL/min

Column temperature: 40 degrees C

Gradient: 0-30 minutes 82-55%A and 18-45%B; 30-32 minutes 55-10%A and 45-90%B; 32.5-37.5 minutes 10%A and 90%B; 38-48 minutes 10-82%A and 90-18%B.

Data treatment:

As both supernatants were treated in the same way, one can directly compare the area of the BLG peaks to calculate a relative yield. As the crystals only contain BLG and the samples all have been treated in the same way, the concentration of alpha-lactalbumin (ALA) and hence the area of ALA should be the same in all of the samples. Therefore, the area of ALA before and after crystallisation is used as a correction factor (cf) when calculating the relative yield.

$$cf_\alpha = \frac{\text{area of ALA}_{before\ crystallization}}{\text{area of ALA}_{after\ crystallization}}$$

The relative yield is calculated by the following equation:

$$Yield_{BLG} = \left(1 - \frac{cf_\alpha \times area\ of\ BLG_{after\ crystallization}}{area\ of\ BLG_{before\ crystallization}}\right) \times 100$$

Acid dissolution of BLC crystals

The remainder of the material from the crystallisation tank was separated using a decanter at 350 g, 2750 RPM, 150 RPM Diff. with a 64 spacer and a feed flow of 75 L/h before separation the feed was mixed 1:2 with polished water. The BLG crystal/solid phase from the decanter was then mixed with polished water in order to make it into a thinner slurry before a phosphoric acid was added to lower the pH to approx. 3.0 in order to quickly dissolve the crystals.

After dissolving the BLG crystals, the pure BLG protein liquid was concentrated to 15 Brix on the same UF setup as used to prepare the feed for crystallisation and the pH was adjusted to final pH of approx. 3.8. The liquid BLG isolate was then heated to 75 degrees for 5 minutes and subsequently cooled to 10 degrees C. The heat-treatment was found to reduce the microbial load from 137.000 CFU/g prior to the heat-treatment to <1000 CFU/g after the heat-treatment. The heat-treatment did not cause any protein denaturation and the intrinsic tryptophan fluorescence emission ratio (330nm/350nm) was determined to 1.20 indicating native confirmation of the BLG molecules.

The BLG was dried on a pilot plant spray drier with an inlet temperature of 180 degrees C and an exit temperature of 75 degrees C. The resulting powder sampled at the exit had a water content of approx. 4 % w/w; the chemical composition of the powder is shown in Table 2. A sample of the dried powder was dissolved and the degree of protein denaturation was determined to 1.5% and the intrinsic tryptophan fluorescence emission ratio (I330/I350) was measured to 1.20.

*Table 2 The composition of the BLG isolate powder (BDL=below the detection limit)*

| BLG isolate powder standardized to 95% total solids | | |
|---|---|---|
| | | |
| Protein composition % w/w of total protein | | |
| ALA | | 0.4 |
| BLG | | 98.2 |
| Other protein | | 1.4 |
| Other selected components (% w/w) | | |
| Ca | | BDL |
| K | | BDL |
| Mg | | BDL |
| Na | | BDL |
| P | | 0.781 |
| fat | | 0.09 |
| protein concentration | | 90 |

The bulk density (625 taps) of the spray-dried powder was estimated at 0.2-0.3 g/cm$^3$.

Conclusion:

By using the above described process, we were able to produce a high-purity BLG product that can be heat-treated with substantially no protein denaturation or protein unfolding during processing. The heat-treatment greatly lowered the bacteria levels without damaging the protein product.

The inventors have seen indications that even higher bulk density can be obtained by increasing the protein content prior to spray-drying. Also, the inventors have observed that even lower degrees of denaturation are obtained if the entry and/or exit temperature used for spray-drying are reduced.

### EXAMPLE 3: PRODUCTION OF A SPRAY-DRIED, PH-NEUTRAL BLG ISOLATE POWDER

When using the same protocol and experimental setup as in Example 2, the lactose-reduced whey protein isolate shown in Table 3 was conditioned and used for feed for crystallisation. The yield of crystallisation was calculated to be 68%.

We note that all weight percentages of specific proteins, such as BLG and ALA, mentioned in this Example pertain to the weight percentage of the non-aggregated proteins relative to total protein.

Table 3 Composition of the feed

| Feed standardized to 95% total solids | | |
|---|---|---|
| Protein composition % w/w of total protein | | |
| ALA | | 9.1 |
| BLG | | 59.1 |

(continued)

| Feed standardized to 95% total solids | | |
|---|---|---|
| Protein composition % w/w of total protein | | |
| Other protein incl. CMP | | 31.6 |
| Other selected components (% w/w) | | |
| Ca | | 0.445 |
| K | | 0.574 |
| Mg | | 0.074 |
| Na | | 0.128 |
| P | | 0.211 |
| fat | | 0.513 |
| protein concentration | | 84 |

The remainder of the material from the crystallisation tank was separated on a decanter at 350 g, 2750 RPM, 150 RPM Diff. with a 64 spacer and a feed flow of 75 L/h. before separation the feed was mixed 1:2 with polished water. The BLG crystal/solid phase from the decanter was then mixed with polished water in order to make it into a thinner slurry before 0.1 M potassium hydroxide was added to adjust the pH to approx. 7 in order to quickly dissolve the crystals.

After dissolving the crystals, the pure BLG protein liquid was concentrated to brix 15 on a the same UF setup as used to prepare the whey protein solution for crystallisation and the pH was adjusted to the final pH of 7.0. The BLG was dried on a pilot plant spray drier with an inlet temperature of 180 degrees C and an exit temperature of 75 degrees C. The resulting powder sampled at the exit had a water content of approx. 4 % w/w. The composition of the powder is shown in Table 4. After drying, some of the powder was dissolved in demineralized water and the degree of protein denaturation was determined to 9.0% and the intrinsic tryptophan fluorescence emission ratio (330nm/350nm) was 1.16.

Table 4 Chemical composition of the BLG isolate powder

| BLG isolate powder standardized to 95% total solids | | |
|---|---|---|
| | | |
| Protein composition % w/w of total protein | | |
| ALA | | 0.2 |
| BLG | | 98.9 |
| Other protein | | 0.9 |
| Other selected components (% w/w) | | |
| Ca | | 0.003 |
| K | | 2.343 |
| Mg | | BDL |
| Na | | BDL |
| P | | 0.629 |
| fat | | 0.329 |
| protein concentration | | 88 |

The bulk density (625 taps) of the spray-dried powder was estimated at 0.2-0.3 g/cm$^3$.

Conclusion:

By using the above described process, we are able to produce a pH-neutral, high-purity BLG product with minimum to no protein denaturation during processing. The inventors have seen indications that even higher bulk density can be obtained by increasing the protein content prior to spray-drying. Also, the inventors have observed that even lower degrees of denaturation are obtained if the entry and/or exit temperature used for spray-drying are reduced. The level of denaturation may furthermore be reduced by reducing the mineral content prior to spray-drying.

**EXAMPLE 4: WETTABILITY OF SPRAY-DRIED BLG ISOLATE POWDERS**

The wettability of the acidic or pH-neutral, spray-dried BLG isolate powders prepared according to Example 2 and Example 3 were compared with the wettability of a regular spray-dried whey protein isolate (WPI). The wettability was measured as the time it takes before the entire powder sample is wet. 0.5 grams of the powder was measured out and placed on the surface of 100 g demineralized water (10 degrees C) in a cylindrical container with a diameter of 5 cm. The time from placing the powders on the surface of the water to the powder was dissolved or had passed through the water surface was measured. The results are presented below.

| Sample # | Powder | Time to dissolve (minutes) | comments |
|---|---|---|---|
| 1 | WPI | +55 | 5-10% of the powder was still left on the surface after 55 minutes |
| 2 | acidic BLG isolate (pH3.7) | 27 | Completely wetted and dissolved |
| 3 | neutral BLG isolate(pH7) | 15 | Completely wetted and dissolved |

Conclusion: It was surprising that uncoated BLG isolate powder (samples 2, 3) wetted much better than a standard WPI (sample 1). This indicates that BLG isolate powders of the present invention are useful ingredients for instant beverage powders, in which rapid wettability and dissolution are important. The BLG isolate powders furthermore offers an improved usefulness relative to regular WPI in the production of protein beverages as they are wetted and dissolved much faster and therefore are more easily dispersed and dissolved during production. Ultimately this may reduce the time it takes to produce a high protein beverage and improve the hourly production capacity of a beverage production plant.

**EXAMPLE 5: ACIDIC BLG ISOLATES HAVING REDUCED LEVEL OF DRYING MOUTHFEELING**

Two protein beverages , A and B, containing the acidic, spray-dried BLG isolate powder prepared in Example 2 in an amount sufficient to provide 6.0% total protein, were pH-adjusted to pH 3.7 and subject to heat-treatment of A: 75 degrees C for 15 seconds or B: 120 degrees C for 20 seconds. Both beverages were cooled immediately and stored in a refrigerator at 5 degrees C. It was confirm by measuring the intrinsic fluorescence emission ratio (I330/I350) that the protein of Beverage A was still in native conformation whereas significant unfolding and denaturation has taken place in Beverage B.

The turbidity of beverage A and B was measured according to Example 1.7 and both beverage had turbidities less than 40 NTU whereas comparable standard WPIs had turbidities above 200 NTU. The acidic BLG isolates of the present invention are therefore clearly very well-suited for production of transparent, acidic high protein beverages.

Less than a week after production both beverages were subjected to sensory testing with a trained sensory testing panel which found that the drying mouthfeeling, which is characteristic for heat-treated, acidic whey protein beverages, was more than 100% higher for Beverage B (drying mouthfeeling score: approx. 10.5 on a scale from 0-12) than for Beverage A (drying mouthfeeling score: approx. 5.0 on a scale from 0-12).

This finding demonstrates that protein denaturation contributes to the drying mouthfeeling of acidic protein beverage and that the drying mouthfeeling can be reduced significantly by limiting or even avoiding protein denaturation.

**EXAMPLE 6: PRODUCTION OF NEUTRAL BLG ISOLATES WITH ULTRA-LOW MICROBIAL CONTENT**

The inventors have found that the present invention makes it possible to obtain pH neutral BLG powders having a very low bacterial content and a high protein nativeness. This is demonstrated in the present example in which the total process time was extended by inserting 6 days of storage at 10 degrees C during the final steps of the conditioning process to challenge the microbial quality of the product.

Whey protein feed:
Lactose-depleted UF retentate derived from sweet whey from a standard cheese production process was filtered through a 1.2 micron filter and had been fat-reduced via a Synder FR membrane prior to being used as feed for the BLG crystallisation process. The chemical composition of the feed can be seen in Table 5. We note that all weight percentages of specific proteins, such as BLG, ALA, mentioned in this Example pertain to the weight percentage of the non-aggregated proteins relative to total protein.

*Table 5 Composition of the whey protein feed used in Example 6.*

| Feed standardized to 95% TS | |
| --- | --- |
| Protein composition (% w/w relative to total protein) | |
| ALA | 10.6 |
| BLG | 59.3 |
| Other protein | 30.1 |
| Other components (% w/w relative to total weight of the standardized feed) | |
| Ca | 0.407 |
| K | 0.567 |
| Mg | 0.075 |
| Na | 0.231 |
| P | 0.196 |
| fat | 0.220 |
| protein | 87 |

Conditioning:

The sweet whey feed was conditioned on an ultrafiltration setup at around 10 degrees C, using an Alfa Laval GR82PE type membrane with a 30 mill spacer feed pressure 1.5-3.0 bar, to a feed concentration of 21% total solids (TS) ±5, and using polished water (water filtered by reverse osmosis to obtain a conductivity of at most 0.05 mS/cm) as diafiltration medium. The pH was adjusted to around 5.9 using a diluted Hydrochloric acid, and diafiltered in a batch ultrafiltration setup. Diafiltration continued until the drop in conductivity of the retentate was below 0.1 mS/cm over a 20 min period.

The diafiltered whey protein feed was then stored for 6 days at 10 degrees C to challenge the microbial quality of the obtained product.

After the storage the whey protein feed was heated to 20 degrees C and pH was then adjusted by adding HCl so that the pH was approx. 5.5 using a diluted hydrochloric acid.

The whey protein feed was conditioned on an ultrafiltration setup at 20 degrees C, using an Alfa Laval GR82PE type membrane with a 30 mill spacer feed pressure 1.5-3.0 bar, to a feed concentration of 21% total solids (TS) ±5, and using polished water as diafiltration medium. The ultrafiltration was run as continuous ultrafiltration setup, and diafiltration was added so that the end retentate had a conductivity of between 1.9-2.2 mS/cm and a TS of 22 ±5. The retentate was collected in a 800L tank with mantel and stirring. Once the tank was full, a first sample of concentrated retentate was taken for HPLC analysis. The HPLC analysis in this example was carried out as described in Example 2.

Crystallisation:

The concentrated retentate was transferred to an 800L crystallisation tank where it was seeded with pure BLG crystal material made from rehydrated, spray-dried BLG crystals. The crystals were added to 1L of conditioned WPI and rapidly cooled on ice to below 5 degrees C. Subsequently, the seeded whey protein solution was cooled from 20 degrees C to approx. 6 degrees C over approx. 4 hours to allow the BLG crystals to form and grow.

After cooling, a sample of the crystal-containing whey protein solution (the second sample) was taken and the BLG crystals were separated by centrifugation at 3000 g for 5 minutes. The supernatant and crystal pellets from the second sample were subjected to HPLC analysis as described below. The yield of crystallisation was calculated as outlined below and determined to %.

Decanter separation:

The crystals produced in the 800L tank were separated from the mother liquor on a decanter (LEMITECH MD80) at 600 g, 2750 RPM Diff. with a 64 mil spacer (mil means 1/1000 inch), the feed flow was 150 L/h and was a mixture of polished water and feed from the tank in a ratio of 1 volume tank feed to 2 volumes polished water.

Dissolving the crystals:

The collected crystals from the decanter were diluted with polished water to a total solids concentration of around 10% before the pH was adjusted to pH 3 using a diluted hydrochloric acid. The acidified BLG solution was held at pH 3 for approx. 16-48 hours at approx. 6 degrees C.

pH-adjustment of the acidified BLG solution:

The acidified BLG solution was then pH adjusted to pH 7 using a diluted mixture of potassium hydroxide and sodium hydroxide and further conditioned on an ultrafiltration setup at around 10 degrees C, using an Alfa Laval GR82PE type membrane with a 30 mill spacer feed pressure 1.5-3.0 bar, to a concentration of approximately 16% TS.

Microfiltration:

A portion of the concentrated BLG solution as subjected to microfiltration using a Membralox EP-1940-GL-UTP membrane having a nominal pore size of 0.8 micron. The microfiltration was operated at approximately 10 degrees C with a feed pressure of 3.5. The MF permeate was collected and was now ready for drying.
A sample of MF permeate was analysed with respect to its chemical composition (see the results in Table 6) and microbiology (Example 1.30) and was surprisingly found to have less than 10 CFU/g (basically meaning that no colonies were identified and that the samples tested appeared to be close to sterile). Sterility may e.g. be obtained using an even tighter microfiltration membrane and ensuring aseptic conditions on the permeate side of the membrane.
A comparable process without the acidification and the microfiltration could easily result in a BLG solution containing more than 1.000.000 cfu/g.

*Table 6: Composition of the microfiltration permeate of the concentrated BLG solution.*

| Microfiltration permeate standardized to 95% TS | |
| --- | --- |
| Protein composition (% w/w relative to total protein) | |
| ALA | 0.5 |
| BLG | 97.5 |
| Other protein | 2 |
| Other components (% w/w relative to total weight of the standardized feed) | |
| Ca | 0.015 |
| K | 0.652 |
| Mg | BDL |
| Na | 0.273 |
| P | BDL |
| fat | BDL |
| protein | 93 |

Conclusion:

Even under a heavy microbiological load the present invention makes it possible to provide a sterile or near-sterile, pH-neutral BLG product having a high degree of protein nativeness.

## EXAMPLE 7: COMPARING REVERSED OSMOSIS CONCENTRATION AND POWDER BULK DENSITY OF ACIDIC AND NEUTRAL BLG WITH A STANDARD NEUTRAL WPI.

The present inventors have found that it is possible to make spray-dried preparations of purified native BLG provide powder having a bulk density higher than comparable whey protein isolates that are spray-dried under the same conditions
The inventors have furthermore found that liquid high protein preparations of purified native BLG have a lower viscosity than comparable whey protein isolates. This discovery makes it possible to concentrate BLG isolates to a higher total protein concentration (and lower water content) prior to drying and therefore requires removal of less water and hence less energy comsumption per kg dried protein than a comparable WPI powder. The reduced viscosity also makes it possible to perform membrane filtration, e.g. microfiltration, with reduced energy consumption as the energy required for membrane filtration decreases with decreasing viscosity.

The above-mentioned findings were demonstrated in the following experiment.

Raw material for reverse osmosis and drying:

Neutral BLG:

MF retentate produced in Example 6 was concentrated on an Alfa Laval RO98pHt membrane with a feed pressure of up to 52 bars and temperature was kept under 15 degrees C during the conditioning. Concentration continued until a brix of 32.2. During concentration, samples were taken for viscosity and Brix measurements as described in Examples 1.8B and 1.28. Brix for each sample was converted to protein (w/w%) using the equation below:

$$Protein\ \% = Brix \times 0.85 \times \frac{protein_{measured\ in\ sample}}{total\ solids_{measured\ in\ sample}}$$

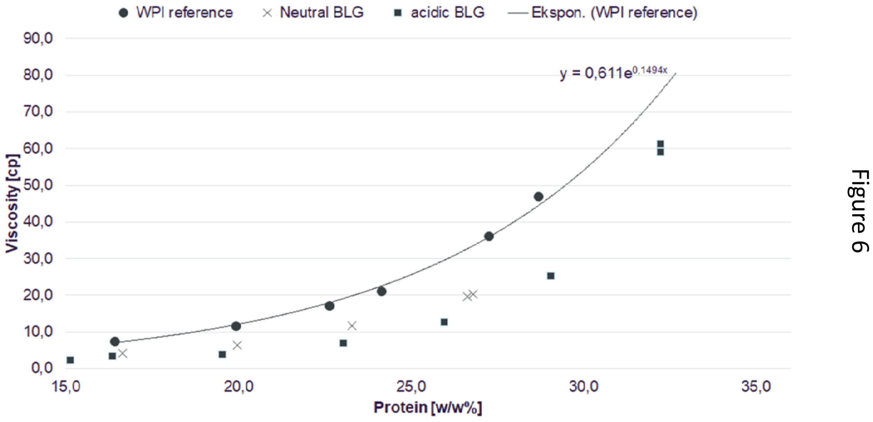

The 0.85 is an empirically obtained conversion giving a good relation between degrees Brix and total solid. The results are shown in Fig. 6.

The total solids content of a whey protein solution is approx. BRIX*0.85.

Acidic BLG:

Acidic BLG solution produced as in Example 6 (with the exception that ultrafiltration was not conducted at pH 5.92 prior to the continuous ultrafiltration at pH 5.5, and that ultrafiltration was conducted at pH 3). The composition of the feed can be seen in Table 7. The acidic BLG permeate subjected to an MF step as described in Example 6 was then concentrated on an Alfa Laval RO98pHt reverse osmosis (RO) membrane with a feed pressure of up to 52 bars and temperature was kept under 15 degrees C during the conditioning. Concentration continued until a brix of 40.0 was obtained. During concentration, samples were taken for measurement of viscosity and brix measurements as described in example 1.8B and 1.28. The Brix values were converted to protein concentration and the results are depicted in Fig. 6. Before drying the BLG was diluted to brix 35.5 with polished water.

*Table 7: Composition of the acidic RO feed*

Feed standardized to 95% TS
Protein composition (% w/w relative to total protein)

| | |
|---|---|
| ALA | BDL |
| BLG | 100 |
| Other protein | BDL |

Other components (% w/w relative to total weight of the standardized feed)

| | |
|---|---|
| Ca | BDL |
| K | BDL |
| Mg | BDL |
| Na | BDL |
| P | BDL |
| fat | BDL |
| protein | 91 |

WPI reference:

To compare the acidic BLG product and pH-neutral BLG product with a traditional pH-neutral WPI product, a standard liquid concentrated WPI based on sweet whey was taken from the production of Arla Foods Danmark Protein and dried on the same pilot plant spray drier and using the same conditions as the acidic and pH-neutral BLG samples. The composition of the liquid concentrated WPI is shown in Table 8.

The viscosity of concentrated WPI was measured initially, and subsequently the concentrated WPI was gradually diluted with polished water to show the correlation between WPI brix and viscosity in order to compare with BLG measurements.

Viscosity curves:

The temperature of the samples taken during RO and of the diluted WPI reference were temperature adjusted to 15 degrees C before making the viscosity measurements (made in triplicate) as described in example 1.8B. Brix was measured as described in example 1.28.

The liquid concentrated WPI was dried with a brix of 36.0.

*Table 8: Composition of the WPI reference.*

| WPI reference standardized to 95% TS | |
|---|---|
| Protein composition (% w/w relative to total protein) | |
| ALA | 9.6 |
| BLG | 59.1 |
| cGMP | 21.6 |
| Other protein | 9.7 |
| Other components (% w/w relative to total weight of the standardized feed) | |
| Ca | 0.072 |
| K | 1.159 |
| Mg | 0.010 |
| Na | 0.489 |
| P | 0.210 |
| fat | 0.100 |
| protein | 89 |

Spray drying:

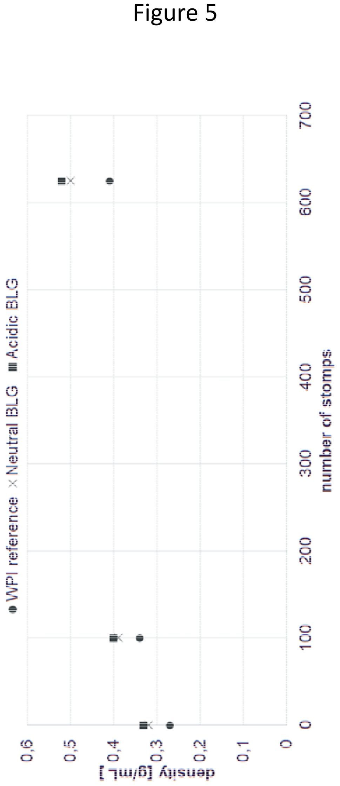

After concentration by reverse osmosis (RO) all samples were dried on a pilot plant spray drier with an inlet temperature of 180 degrees C and an exit temperature of 85 degrees C without any preheating. The temperature of all samples were kept below 12 degrees C until spray drying. The bulk densities of the spray dried powders were then measured as described in example 1.17 , without stomping, with 100 stomps, and with 625 stomps. Surprisingly both the acidic and pH-neutral BLG powder were significantly and consistently higher in bulk density than the WPI reference. On average, the bulk density of the pH-neutral BLG was 18.4 percent higher than the powder of the WPI reference and the acidic was 22.2 percent higher. The results of bulk density measurements are shown in Table 9 and are illustrated in Fig. 5.

*Table 9: Bulk densities of powders obtained from an acidic BLG solution, a pH-neutral BLG solution, and a reference WPI solution which have the same content of total protein and which have been spray-dried under identical conditions.*

| Sample | Stomps | Bulk density (g/mL) | Difference relative to the dried WPI reference (%) |
|---|---|---|---|
| WPI reference | 0 | 0.27 | |
| WPI reference | 100 | 0.34 | |
| WPI reference | 625 | 0.41 | |
| Neutral BLG | 0 | 0.32 | 19 |
| Neutral BLG | 100 | 0.39 | 15 |
| Neutral BLG | 625 | 0.5 | 22 |
| Acidic BLG | 0 | 0.33 | 22 |
| Acidic BLG | 100 | 0.4 | 18 |
| Acidic BLG | 625 | 0.52 | 27 |

Conclusion:

Both the acidic and the pH-neutral BLG isolate types had a lower viscosity compared to the WPI reference and were easier to concentrate on RO. This discovery makes it possible to concentrate BLG isolates to a higher total protein concentration (and lower water content) prior to drying and therefore requires removal of less water and hence less energy consumption per kg dried protein than a comparable WPI powder. The reduced viscosity also makes it possible to perform membrane filtration, e.g. microfiltration, with reduced energy consumption as the energy required for membrane filtration decreases with decreasing viscosity.

The BLG powders of the present invention are furthermore particularly suitable for high protein beverages, or shake powders for preparing high protein beverages as the present BLG powders contribute less to the viscosity than traditional WPIs and hence provide more drinkable beverages.

Surprisingly, the spray-dried BLG powders furthermore had a higher bulk density compared to the WPI reference even when the BLG was dried at a slightly lower amount of total solids, as described by the brix value. A higher powder bulk density means less shipping volume per weight when shipping and denser particles also tend to create less dust when being handled.

## Claims

1. A BLG isolate powder, preferably prepared by spray-drying, having a pH in the range of ii) 6.1-8.5 or iii) 5.0-6.0 and comprising:

   - total protein in an amount of at least 30% w/w,
   - beta-lactoglobulin (BLG) in an amount of at least 85% w/w relative to total protein
   - water in an amount of at most 10% w/w, and
   - at most 15000 colony-forming units/g,

   said BLG isolate powder having one or more of the following:

   - an intrinsic tryptophan fluorescence emission ratio (I330/I350) of at least 1.11, and
   - a degree of protein denaturation of at most 10%.

2. The BLG isolate powder according to claim 1 having one or more of:

   - a heat-stability at pH 3.9 of at most 200 NTU, and
   - a bulk density of at least 0.2 g/cm$^3$.

3. The BLG isolate powder according to claim 1 or 2 having a pH in the range of 6.1-8.5.

4. The BLG isolate powder according to claim 1 or 2 having a pH in the range of 5.0-6.0.

5. The BLG isolate powder according to any of the preceding claims comprising:

   - total protein in an amount of at least 40% w/w, preferably at least 50% w/w, at least 60% w/w, more preferably at least 70% w/w, even more preferably at least 80% w/w, even more preferably at least 90% w/w, and most preferably at least 92% w/w, and/or
   - BLG in an amount of at least 88% w/w relative to total protein, preferably at least 90% w/w relative to total protein.

6. The BLG isolate powder according to any of the preceding claims wherein the powder comprises lipid in an amount of at most 10% w/w, preferably at most 5% w/w, more preferably at most 2% w/w, and even more preferably at most 0.1% w/w.

7. The BLG isolate powder according to any of the preceding claims having a bulk density of at least 0.20 g/cm$^3$, preferably at least 0.30 g/cm$^3$.

8. The BLG isolate powder according to any of the preceding claims having an intrinsic tryptophan fluorescence emission ratio (I330/I350) of at least 1.11.

9. The BLG isolate powder according to any of the preceding claims having a degree of protein denaturation of at most 10%.

10. A liquid BLG isolate having a pH in the range of ii) 6.1-8.5 or iii) 5.0-6.0 and comprising:

    - total protein in an amount of at least 10% w/w,

- beta-lactoglobulin (BLG) in an amount of at least 85% w/w relative to total protein, and
- at most 15000 colony-forming units/g, preferably at most 1000 colony-forming units/g,

said BLG isolate powder having one or more of the following:

- an intrinsic tryptophan fluorescence emission ratio (I330/I350) of at least 1.11, and
- a degree of protein denaturation of at most 10%.

11. The liquid BLG isolate according to claim 10 having a heat-stability at pH 3.9 of at most 200 NTU.

12. A method of producing a dried BLG isolate powder according to claim 1 containing BLG in an amount of at least 85% w/w relative to total protein, the method comprising the steps of:

   a) providing a liquid BLG isolate having

      ii) a pH of in the range of 6.1-8.5, or
      iii) a pH of in the range of 5.0-6.0

   said liquid BLG isolate containing BLG in an amount of at least 85% w/w relative to total protein,
   b) optionally, subjecting the liquid BLG isolate to a physical microbial reduction,
   c) drying the liquid BLG isolate by spray-drying.

13. The method according to claim 12 wherein the protein fraction of the liquid BLG isolate has an intrinsic tryptophan fluorescence emission ratio (I330/I350) of at least 1.11.

14. The method according to any of the claims 12-13 wherein the protein of liquid BLG isolate has a degree of protein denaturation of at most 10% w/w.

15. The method according to any of the claims 12-14, wherein the provision of the liquid BLG isolate involves isolating BLG from whey protein feed, and optionally subjecting the resulting BLG-enriched composition to one or more steps selected from the group of:

   - demineralisation,
   - addition of minerals,
   - dilution,
   - physical microbial reduction,
   - concentration, and
   - pH-adjustment.

16. The method according to any of the claims 12-15, wherein the liquid BLG isolate is subjected to physical microbial reduction, which preferably involves or even consists of heat-treatment.

17. Use of the BLG isolate powder according to any of claims 1-9 or the liquid BLG isolate according to claim 10 or 11 as an ingredient for the production of a food product, e.g. a beverage or an instant beverage powder, having a pH in the range of 2-4.7 and furthermore having one or more of the following:

   - a reduced level of drying mouthfeel,
   - improved transparency, and/or
   - increased content of protein, preferably heat-treated beverages containing a protein content in an amount of at least 3-45% w/w, more preferably 11-40% w/w, even more preferably 15-38% w/w and most preferably 20-36% w/w.

Figure 1

```
        ╭──────────╮
       ╱  BLG isolate  ╲
      │     powder      │
       ╲               ╱
        ╰──────▲─────╯
               │
      ┌────────┴────────┐
      │     Drying       │
      └────────┬────────┘
               │
   ┌───────────┴───────────┐
   │  Optional microbial    │
   │      reduction         │
   └───────────┬───────────┘
               │
        ╭──────┴──────╮
       ╱  Liquid BLG    ╲
      │     isolate      │
       ╲                ╱
        ╰──────────────╯
```

# Figure 2

BLG isolate powder

↑

Drying

Optional microbial reduction

Liquid BLG isolate

↑

Processing

BLG-enriched comp.

↑

BLG isolation

Whey protein feed

## Figure 3

250 micron

## Figure 4

1000 micron

# Figure 5

Figure 6

**EUROPEAN SEARCH REPORT**

Europäisches
Patentamt
European
Patent Office
Office européen
des brevets

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | WO 2018/115520 A1 (ARLA FOODS AMBA) 28 June 2018 (2018-06-28) * page 3, lines 4-13,27-36; claims 1-6,9-18,23-28,31-38,40; examples 1-3,8,11,12,14; table 14 * | 1-17 | INV. A23C1/04 A23J1/00 A23C1/00 A23L33/19 A23C9/142 |
| X | WO 2004/049819 A2 (CAMPINA BV) 17 June 2004 (2004-06-17) * page 3, line 30 - page 7, line 12; claims 1-6,10-16,20-22, 24, 26-33; examples 3-5,9 * | 1-17 | A23L2/10 A23L2/39 A23J1/20 |
| X | EP 0 604 684 A1 (CAMPINA MELKUNIE BV) 6 July 1994 (1994-07-06) | 1-3,5-16 | |
| A | * examples 1-7; claims 1-19 * | 17 | |
| X | WO 2009/113845 A1 (NUTRICIA NV) 17 September 2009 (2009-09-17) * pages 2,4,6,20; claims 1-3,7 * * pages 8-12,21; claims 22,26 * | 1-17 | |
| A | WO 2011/112695 A1 (MILLER KEVIN BURKE) 15 September 2011 (2011-09-15) * examples 1, 4 and 7 * | 1-17 | **TECHNICAL FIELDS SEARCHED (IPC)** A23C A23J A23L |
| A | WO 2010/037736 A1 (UPFRONT CHROMATOGRAPHY AS) 8 April 2010 (2010-04-08) * page 3, lines 4-12,22-23; claims 1,7,10; examples 2, 7 * * page 19, lines 1-7 - page 21, line 23 * * page 29, line 7 - page 31, line 2 * | 1-17 | |

-/--

The present search report has been drawn up for all claims

| Place of search | Date of completion of the search | Examiner |
|---|---|---|
| The Hague | 21 April 2026 | Kanbier, Titia |

EPO FORM 1503 03.82 (P04C01)

Europäisches
Patentamt

European
Patent Office

Office européen
des brevets

## EUROPEAN SEARCH REPORT

**Application Number**

EP 25 22 4794

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2011/051436 A1 (NESTEC SA) 5 May 2011 (2011-05-05) * page 1, lines 19-23; claims 8,13,14; examples 1-2,6-7 * * page 2, lines 18-22 * * page 6, lines 5-18 * * page 8, lines 4-17; figure 8 * | 1-17 | |
| A | S. DOULTANI ET AL: "Whey Protein Isolate and Glyco-macropeptide Recovery from Whey Using Ion Exchange Chromatography", JOURNAL OF FOOD SCIENCE, vol. 68, no. 4, 1 May 2003 (2003-05-01), pages 1389-1395, XP055725997, Hoboken, USA ISSN: 0022-1147, DOI: 10.1111/j.1365-2621.2003.tb09655.x | 1-17 | |
| A | ETZEL M R: "Manufacture and use of dairy protein fractions", THE JOURNAL OF NUTRITION WISTAR INSTITUTE OF ANATOMY AND BIOLOGY., UNITED STATES, vol. 134, 1 January 2004 (2004-01-01), pages 996S-1002S, XP002529524, ISSN: 0022-3166, DOI: 10.1093/JN/134.4.996S | 1-17 | **TECHNICAL FIELDS SEARCHED (IPC)** |
| A | WO 2014/055830 A1 (ABBOTT LAB) 10 April 2014 (2014-04-10) | 1-17 | |
| A | WO 2009/038746 A1 (UNIV NORTH CAROLINA STATE) 26 March 2009 (2009-03-26) | 1-17 | |
| A | WO 01/52665 A1 (HANNAH RES INST) 26 July 2001 (2001-07-26) | 1-17 | |

The present search report has been drawn up for all claims

| Place of search | Date of completion of the search | Examiner |
|---|---|---|
| The Hague | 21 April 2026 | Kanbier, Titia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 22 4794

This annex lists the patent family members relating to the patent documents cited in the above-mentioned European search report.
The members are as contained in the European Patent Office EDP file on
The European Patent Office is in no way liable for these particulars which are merely given for the purpose of information.

21-04-2026

| Patent document cited in search report | | Publication date | Patent family member(s) | | Publication date |
|---|---|---|---|---|---|
| WO 2018115520 | A1 | 28-06-2018 | AU | 2017380258 A1 | 11-07-2019 |
| | | | AU | 2023201182 A1 | 06-04-2023 |
| | | | BR | 112019012944 A2 | 10-12-2019 |
| | | | CA | 3047668 A1 | 28-06-2018 |
| | | | CN | 110381745 A | 25-10-2019 |
| | | | CN | 115624083 A | 20-01-2023 |
| | | | CN | 115624084 A | 20-01-2023 |
| | | | CN | 121058755 A | 05-12-2025 |
| | | | DK | 3558024 T3 | 25-05-2021 |
| | | | EA | 201991293 A1 | 22-01-2020 |
| | | | EP | 3558024 A1 | 30-10-2019 |
| | | | EP | 3858145 A1 | 04-08-2021 |
| | | | EP | 4696149 A2 | 18-02-2026 |
| | | | ES | 2876941 T3 | 15-11-2021 |
| | | | JP | 7222891 B2 | 15-02-2023 |
| | | | JP | 7551707 B2 | 17-09-2024 |
| | | | JP | 2020501580 A | 23-01-2020 |
| | | | JP | 2022188055 A | 20-12-2022 |
| | | | JP | 2025003954 A | 14-01-2025 |
| | | | KR | 20190117492 A | 16-10-2019 |
| | | | KR | 20230161522 A | 27-11-2023 |
| | | | KR | 20250035606 A | 12-03-2025 |
| | | | MY | 191508 A | 28-06-2022 |
| | | | PL | 3558024 T3 | 20-09-2021 |
| | | | PT | 3558024 T | 25-05-2021 |
| | | | US | 2019320682 A1 | 24-10-2019 |
| | | | WO | 2018115520 A1 | 28-06-2018 |
| | | | ZA | 201904760 B | 23-12-2020 |
| WO 2004049819 | A2 | 17-06-2004 | AU | 2003293761 A1 | 23-06-2004 |
| | | | EP | 1565067 A2 | 24-08-2005 |
| | | | JP | 2006508160 A | 09-03-2006 |
| | | | NZ | 540406 A | 30-04-2008 |
| | | | US | 2006204454 A1 | 14-09-2006 |
| | | | WO | 2004049819 A2 | 17-06-2004 |
| EP 0604684 | A1 | 06-07-1994 | AT | E154200 T1 | 15-06-1997 |
| | | | AU | 664934 B2 | 07-12-1995 |
| | | | CA | 2111668 A1 | 24-06-1994 |
| | | | DE | 69220374 T2 | 15-01-1998 |
| | | | DK | 0604684 T3 | 26-01-1998 |
| | | | EP | 0604684 A1 | 06-07-1994 |
| | | | JP | H07123927 A | 16-05-1995 |
| | | | NZ | 250400 A | 27-04-1995 |
| | | | US | 5420249 A | 30-05-1995 |

EPO FORM P0459

For more details about this annex : see Official Journal of the European Patent Office, No. 12/82

page 1 of 3

## ANNEX TO THE EUROPEAN SEARCH REPORT
## ON EUROPEAN PATENT APPLICATION NO.

EP 25 22 4794

This annex lists the patent family members relating to the patent documents cited in the above-mentioned European search report.
The members are as contained in the European Patent Office EDP file on
The European Patent Office is in no way liable for these particulars which are merely given for the purpose of information.

21-04-2026

| Patent document cited in search report | Publication date | Patent family member(s) | Publication date |
|---|---|---|---|
| WO 2009113845 A1 | 17-09-2009 | BR PI0909206 A2 | 04-08-2015 |
| | | CN 102083329 A | 01-06-2011 |
| | | DK 2252167 T3 | 17-06-2013 |
| | | DK 2612561 T3 | 01-12-2014 |
| | | DK 2835059 T3 | 02-01-2024 |
| | | EP 2252167 A1 | 24-11-2010 |
| | | EP 2612561 A1 | 10-07-2013 |
| | | EP 2835059 A1 | 11-02-2015 |
| | | ES 2414534 T3 | 19-07-2013 |
| | | PL 2252167 T3 | 30-09-2013 |
| | | PL 2612561 T3 | 30-04-2015 |
| | | PL 2835059 T3 | 13-05-2024 |
| | | RU 2010141720 A | 20-04-2012 |
| | | US 2011046048 A1 | 24-02-2011 |
| | | US 2014364361 A1 | 11-12-2014 |
| | | WO 2009113845 A1 | 17-09-2009 |
| | | WO 2009113858 A1 | 17-09-2009 |
| WO 2011112695 A1 | 15-09-2011 | AU 2011224427 A1 | 04-10-2012 |
| | | BR 112012023025 A2 | 08-09-2020 |
| | | CA 2792396 A1 | 15-09-2011 |
| | | CN 102917604 A | 06-02-2013 |
| | | EP 2544555 A1 | 16-01-2013 |
| | | ES 2681849 T3 | 17-09-2018 |
| | | JP 6240653 B2 | 29-11-2017 |
| | | JP 2013521779 A | 13-06-2013 |
| | | JP 2016104016 A | 09-06-2016 |
| | | PH 12012501759 A1 | 12-11-2012 |
| | | RU 2012143622 A | 20-04-2014 |
| | | SG 183902 A1 | 29-11-2012 |
| | | SG 2014011142 A | 29-05-2014 |
| | | TR 201811254 T4 | 27-08-2018 |
| | | US 2013065822 A1 | 14-03-2013 |
| | | US 2014342040 A1 | 20-11-2014 |
| | | WO 2011112695 A1 | 15-09-2011 |
| | | ZA 201207652 B | 26-03-2014 |
| WO 2010037736 A1 | 08-04-2010 | DK 2355664 T3 | 06-10-2014 |
| | | EP 2355664 A1 | 17-08-2011 |
| | | EP 2801260 A1 | 12-11-2014 |
| | | US 2011218327 A1 | 08-09-2011 |
| | | WO 2010037736 A1 | 08-04-2010 |
| WO 2011051436 A1 | 05-05-2011 | CN 102711528 A | 03-10-2012 |
| | | EP 2316283 A1 | 04-05-2011 |
| | | EP 2493338 A1 | 05-09-2012 |

EPO FORM P0459

For more details about this annex : see Official Journal of the European Patent Office, No. 12/82

page 2 of 3

## ANNEX TO THE EUROPEAN SEARCH REPORT
## ON EUROPEAN PATENT APPLICATION NO.

EP 25 22 4794

This annex lists the patent family members relating to the patent documents cited in the above-mentioned European search report.
The members are as contained in the European Patent Office EDP file on
The European Patent Office is in no way liable for these particulars which are merely given for the purpose of information.

21-04-2026

| Patent document cited in search report | Publication date | Patent family member(s) | | Publication date |
|---|---|---|---|---|
| | | JP | 2013509335 A | 14-03-2013 |
| | | US | 2012219691 A1 | 30-08-2012 |
| | | WO | 2011051436 A1 | 05-05-2011 |
| WO 2014055830 A1 | 10-04-2014 | BR | 112015007666 A2 | 04-07-2017 |
| | | CA | 2886108 A1 | 10-04-2014 |
| | | CN | 104822278 A | 05-08-2015 |
| | | CN | 107495042 A | 22-12-2017 |
| | | DK | 2903459 T3 | 23-10-2017 |
| | | EP | 2903459 A1 | 12-08-2015 |
| | | EP | 3235387 A1 | 25-10-2017 |
| | | ES | 2641374 T3 | 08-11-2017 |
| | | HK | 1212561 A1 | 17-06-2016 |
| | | JP | 2015530123 A | 15-10-2015 |
| | | MX | 356892 B | 19-06-2018 |
| | | PH | 12015500738 A1 | 01-06-2015 |
| | | SG | 11201502647P A | 28-05-2015 |
| | | US | 2015250221 A1 | 10-09-2015 |
| | | US | 2017238583 A1 | 24-08-2017 |
| | | WO | 2014055830 A1 | 10-04-2014 |
| WO 2009038746 A1 | 26-03-2009 | US | 2009074918 A1 | 19-03-2009 |
| | | WO | 2009038746 A1 | 26-03-2009 |
| WO 0152665 A1 | 26-07-2001 | AU | 2693801 A | 31-07-2001 |
| | | EP | 1251746 A1 | 30-10-2002 |
| | | JP | 2003520037 A | 02-07-2003 |
| | | US | 2003078392 A1 | 24-04-2003 |
| | | WO | 0152665 A1 | 26-07-2001 |

EPO FORM P0459

For more details about this annex : see Official Journal of the European Patent Office, No. 12/82

page 3 of 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2790790 A1
- EP 0604684 A1
- WO 2010037736 A1
- WO 2011112695 A
- EP 2017084553 W
- WO 2009113858 A1
- WO 2010085957 A3

**Non-patent literature cited in the description**

- **JONGH et al.** Mild Isolation Procedure Discloses New Protein Structural Properties of β-Lactoglobulin. *J Dairy Sci.*, 2001, vol. 84 (3), 562-571
- **VYAS et al.** Scale-Up of Native β-Lactoglobulin Affinity Separation Process. *J. Dairy Sci.*, 2002, vol. 85, 1639-1645
- **PALMER**. Crystalline Globulin from Cow's Milk. *J. Biol. Chem.*, 1934, vol. 104, 359-372
- **GÉR-ARD COQUERELA**. Crystallization of molecular systems from solution: phase diagrams, supersaturation and other basic concepts. *Chemical Society Reviews*, 2014 (7), 2286-2300
- **JONGH**. Mild Isolation Procedure Discloses New Protein Structural Properties of β-Lactoglobulin. *J Dairy Sci*, 2001, vol. 84 (3), 562-571
- **VYAS**. Scale-Up of Native β-Lactoglobulin Affinity Separation Process. *J. Dairy Sci.*, 2002, vol. 85, 1639-1645
- **GUERRA-HERNANDEZ et al.** Maillard Reaction Evaluation by Furosine Determination During Infant Cereal Processing. *Journal of Cereal Science*, 1999, vol. 29, 171-176
- **SOYEURT et al.** Mid-infrared prediction of lactoferrin content in bovine milk: potential indicator of mastitis. *Animal*, 2012, vol. 6 (11), 1830-1838